# EUROPEAN PATENT SPECIFICATION

(11) **EP 2 999 788 B1**
(45) Date of publication and mention of the grant of the patent: **08.07.2020**
(21) Application number: 14800698.4
(22) Date of filing: 22.05.2014
(51) Int. Cl.: C12N 15/66, C12N 9/22, C12N 15/55, C12N 5/10

(54) **RNA-DIRECTED DNA CLEAVAGE AND GENE EDITING BY CAS9 ENZYME FROM NEISSERIA MENINGITIDIS**
RNA-GERICHTETE DNA-SPALTUNG UND GENMANIPULATION DURCH DAS ENZYM CAS9 AUS NEISSERIA MENINGITIDIS
CLIVAGE DE L'ADN ORIENTÉ VERS ARN ET ÉDITION GÉNÉTIQUE PAR L'ENZYME CAS9 PROVENANT DE NEISSERIA MENINGITIDIS

(30) Priority: 22.05.2013 US 201361826338 P
(43) Date of publication of application: 30.03.2016
(73) Proprietor: Northwestern University, Evanston, IL 60201 (US); Wisconsin Alumni Research Foundation, Madison, WI 53726 (US)
(72) Inventor: SONTHEIMER, Erik, J., Auburndale, MA 02466 (US); ZHANG, Yan, Shrewsbury, MA 01545 (US); MONDRAGON, Alfonso, Chicago, IL 60640 (US); RAJAN, Rakhi, Norman, OK 73072 (US); THOMSON, James, Madison, WI 53726 (US); HOU, Zhonggang, Shrewsbury, MA 01545 (US)
(74) Representative: Boult Wade Tennant LLP
(86) International application number: PCT/US2014/039195
(87) International publication number: WO 2014/190181

(56) References cited:
- WO-A2-2010/075424
- L. CONG ET AL: "Multiplex Genome Engineering Using CRISPR/Cas Systems", SCIENCE, vol. 339, no. 6121, 14 February 2013 (2013-02-14), pages 819-823, XP055236048, ISSN: 0036-8075, DOI: 10.1126/science.1231143
- M. JINEK ET AL: "Supplementary Materials for A Programmable Dual-RNA-Guided DNA Endonuclease in Adaptive Bacterial Immunity", SCIENCE, vol. 337, no. 6096, 28 June 2012 (2012-06-28), pages 1-37, XP55067747, ISSN: 0036-8075, DOI: 10.1126/science.1225829
- Z. HOU ET AL: "Efficient genome engineering in human pluripotent stem cells using Cas9 from Neisseria meningitidis", PROCEEDINGS OF THE NATIONAL ACADEMY OF SCIENCES, vol. 110, no. 39, 12 August 2013 (2013-08-12), pages 15644-15649, XP55118866, ISSN: 0027-8424, DOI: 10.1073/pnas.1313587110
- MALI P. ET AL.: 'RNA-guided human genome engineering via Cas9.' SCIENCE vol. 339, no. 6121, 15 February 2013, pages 823 - 826, XP055153369
- SAMPSON T.R. ET AL.: 'A CRISPR/Cas system mediates bacterial innate immune evasion and virulence.' NATURE vol. 497, no. 7448, 09 May 2013, pages 254 - 258, XP055205805
- GASIUNAS G. ET AL.: 'Cas9 ribonucleoprotein complex mediates specific DNA cleavage for adaptive immunity in bacteria.' PROCEEDINGS OF THE NATIONAL ACADEMY OF SCIENCES vol. 109, no. 39, 2012, pages E2579 - E2586, XP055068588
- PAIGE J.S. ET AL.: 'RNA mimics of green fluorescent protein.' SCIENCE vol. 333, 2011, pages 642 - 646, XP055044003
- BELETSKII A. ET AL.: 'PNA interference mapping demonstrates functional domains in the noncoding RNA Xist.' PROCEEDINGS OF THE NATIONAL ACADEMY OF SCIENCES vol. 98, no. 16, 2001, pages 9215 - 9220, XP002735636
- Krzysztof Chylinski ET AL: "The tracrRNA and Cas9 families of type II CRISPR-Cas immunity systems", RNA Biology, vol. 10, no. 5, 1 May 2013 (2013-05-01), pages 726-737, XP055116068, ISSN: 1547-6286, DOI: 10.4161/rna.24321

## Description

### BACKGROUND

The field of the invention relates to methods and kits for modifying DNA. In particular, the field of the invention relates to kits and methods for RNA-directed DNA cleavage and gene editing.

Genome editing has proven to be quite difficult in cells, particularly in mammalian cells. One way to improve genome-editing efficiency is to introduce a double-strand break (DSB) in the desired DNA region. DSBs stimulate the DNA repair machinery and, in the presence of a homologous repair template, greatly enhance genome editing efficiency. Currently, there are two widely used systems to introduce targeted DSBs in genomes of mammalian cells - Zinc Finger Nucleases (ZFNs) and Transcription activator-like effector nucleases (TALENs), both of which are engineered by fusing site-specific DNA recognition domains to FokI endonucleases. One major obstacle to the wide application of these two systems is the difficulty of engineering peptides that recognize specific target DNA sites. Also, for each unique target sequence, a different pair of ZFNs or TALENs has to be engineered. For ZFNs, the optimal designing algorithm is proprietary and only available through commercial sources. For TALENs, the design rules are quite straightforward, but it still takes weeks to make one pair of targeting constructs, and each must be adequately expressed and validated.

Clustered, regularly interspaced short palindromic repeats (CRISPRs) are known in the art (see Marraffini and Sontheimer, Nature Reviews Vol. 11, Mach 2010, 181-190, U.S. Published Patent Application No. 2010/0076057, and U.S. Patent No. 8,697,359), and have been utilized for genome editing (see Cain, SciBX, Vol. 6, No. 4, January 2010, 1-7). Recently, CRISPR RNAs (crRNAs) have been developed that direct DNA cleavage by a bacterial protein called Cas9. (See Cong et al., Science, Vol. 339, February 15, 2013, 819-822; and Mali et al., Science, Vol. 339, February 15, 2013, 823-826). This system requires only three components: a Cas9 endonuclease, a trans-activating CRISPR RNA (tracrRNA), and the target-specifying crRNA which hybridizes to a target DNA sequence and targets the DNA sequence for cleavage by the Cas9 endonuclease. Accordingly, nearly any genomic locus can be targeted by the same Cas9 protein, as long as a crRNA complementary to the targeted sequence is provided. Two Cas9 proteins (SpCas9 from *Streptococcus pyogenes* and StCas9 from *Streptococcus thermophilus*) have been reported as effective in genome editing, and each has its own targeting sequence requirements. However, there is a need for the identification of new systems in order to maximize the potential of CRISPR as a gene editing tool.

Here, we report a new form of Cas9 (NmCas9 from *Neisseria meningitidis*) that has distinct targeting requirements which are less likely to result in off-target effects. Furthermore, unlike SpCas9 and StCas9, NmCas9 can function with crRNAs that are embedded within longer unprocessed precursors, indicating that NmCas9 can accommodate a greater range of targeting crRNA structures and functionalities. In addition, mutant forms of NmCas9 can be used that bind DNA in an RNA-directed fashion, but that do not cleave the DNA.

### SUMMARY

The present invention is defined in the claims. Disclosed are methods and kits for RNA-directed DNA cleavage and gene editing. The methods utilize components including a Cas9 protein from Neisseria and one or more RNA molecules in order to direct the Cas9 protein to bind to and optionally cleave or nick a target sequence.

The methods modify a target DNA sequence in a cell and include: (a) expressing a Cas9 protein from a *Neisseria* species or a variant protein thereof having at least 80% amino acid sequence identity relative to SEQ ID NO: 1; wherein the Cas9 protein or variant has nuclease activity and the ability to bind to a target polynucleotide sequence in the cell (*e.g.,* by transfecting the cell with a DNA molecule or an RNA molecule that expresses the Cas9 protein (*i.e.,* Cas9 mRNA)); and (b) expressing or transfecting RNA comprising a CRISPR RNA (crRNA) and a trans-activating CRISPR RNA (tracrRNA) in the cell, wherein the RNA molecule binds to the Cas9 protein or variant, and the RNA molecule hybridizes to the target DNA sequence with the proviso that the method is not a method for modifying the germ line genetic identity of human beings; and with the proviso that the method is: (i) performed *in vitro* or *ex vivo;* or (ii) not a method for treatment of the human or animal body by therapy. The Cas9 protein or variant protein has nuclease activity (*e.g*., DNase activity and/or RNase activity) and, optionally, cleaves one (*i.e.,* nicks) or both strands of the target DNA sequence. Optionally, the methods further comprise contacting the target DNA sequence with a homologous DNA fragment, wherein homologous recombination is induced between the homologous DNA fragment and the target DNA sequence (*e.g*., homologous recombination to effect gene repair or to effect gene disruption).

In some embodiments, the Cas9 protein or variant protein is encoded and expressed by a nucleic acid having a codon sequence that is optimized for expression in the cell. For example, the nucleic acid may have a codon sequence that is optimized for expression in an animal cell (*e.g.,* a human or non-human mammalian cell). The Cas9 protein may be expressed from an expression vector comprising a prokaryotic or eukaryotic promoter for expressing the Cas9 protein which is transfected into the cell.

The Cas9 protein may be from *Neisseria meningitidis.* Variants of Cas9 proteins may include proteins having an amino acid sequence that has at least about 85%, 90%, 95%, 96%, 97%, 98%, or 99% sequence identity to an amino acid sequence of a Cas9 protein.

The methods are utilized to target a DNA sequence in a cell. Suitable cells may include prokaryotic cells and eukaryotic cells. In some embodiments, the methods are performed to target a DNA sequence in a stem cell (*e.g.,* an embryonic stem cell or an induced pluripotent stem cell).

The methods utilize an RNA molecule that comprises a sequence that hybridizes with a target DNA sequence in a cell. The RNA molecule also binds with the Cas9 protein or a variant thereof. In some embodiments, the RNA molecule comprises two molecules of duplexed RNA (*e.g.,* crRNA duplexed with tracRNA). In other embodiments, the RNA molecule is a single RNA molecule forming a hairpin structure (e.g., where crRNA is linked to tracRNA via a linker such as GAAA and the crRNA and tracRNA form the stem of the hairpin). In further embodiments, the RNA may include an RNA mimic of green fluorescent protein (GFP). As such, the RNA may be utilized to map a target DNA sequence via adding 4-hydroxybenzylidene (HPD), 3,5-dimethoxy-4-hydroxybenzylidene (DMHPD), or a 3,5-difluoro-4-hydroxybenzylidene to the cell (DFHPD), wherein the RNA binds to HPD, DMHPD, or DFHPD to form a fluorescent complex. In further embodiments, the RNA may comprise Xist RNA or fragments thereof, which may be utilized to modulate the expression of the target DNA sequence. The RNA may be transfected directly into a cell and/or may be expressed from an expression vector comprising a prokaryotic or eukaryotic promoter for expressing the RNA when the expression vector is transfected into the cell.

Also disclosed are kits for performing the disclosed methods.

The contemplated kits comprise: (a) a vector for expressing a Cas9 protein from a *Neisseria* species or a variant protein thereof having at least 80% amino acid sequence identity relative to SEQ ID NO: 1, wherein the Cas9 protein or variant has nuclease activity and the ability to bind to a target polynucleotide sequence in a cell; and (b) a vector for expressing an RNA in the cell, wherein the RNA comprises a CRISPR RNA (crRNA) and a trans-activating CRISPR RNA (tracrRNA) wherein the RNA binds to the Cas9 protein or variant, and the RNA hybridizes to the target DNA sequence.

### BRIEF DESCRIPTION OF THE DRAWINGS

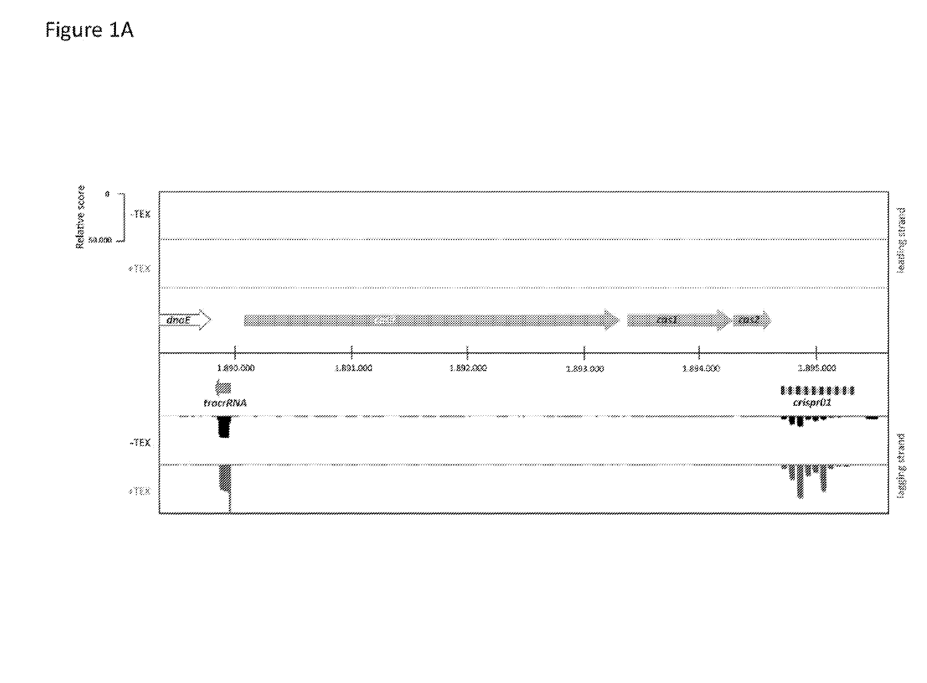

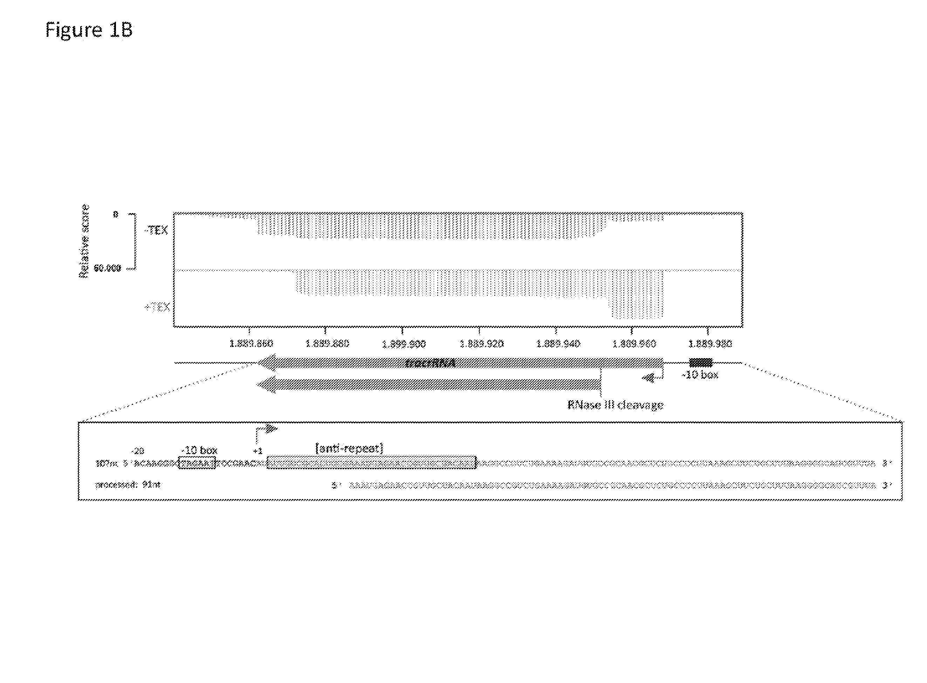

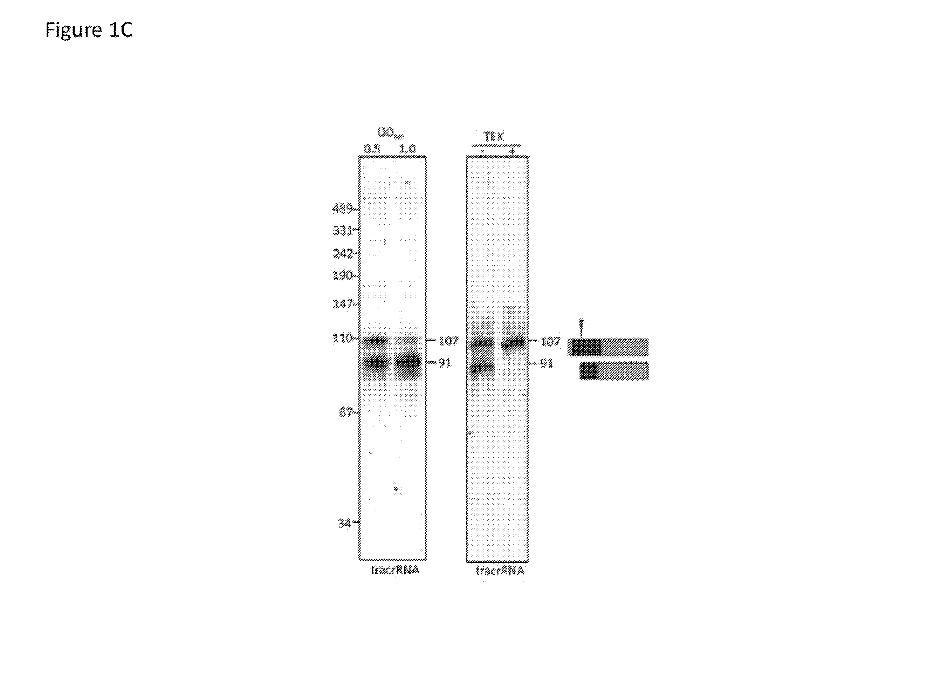

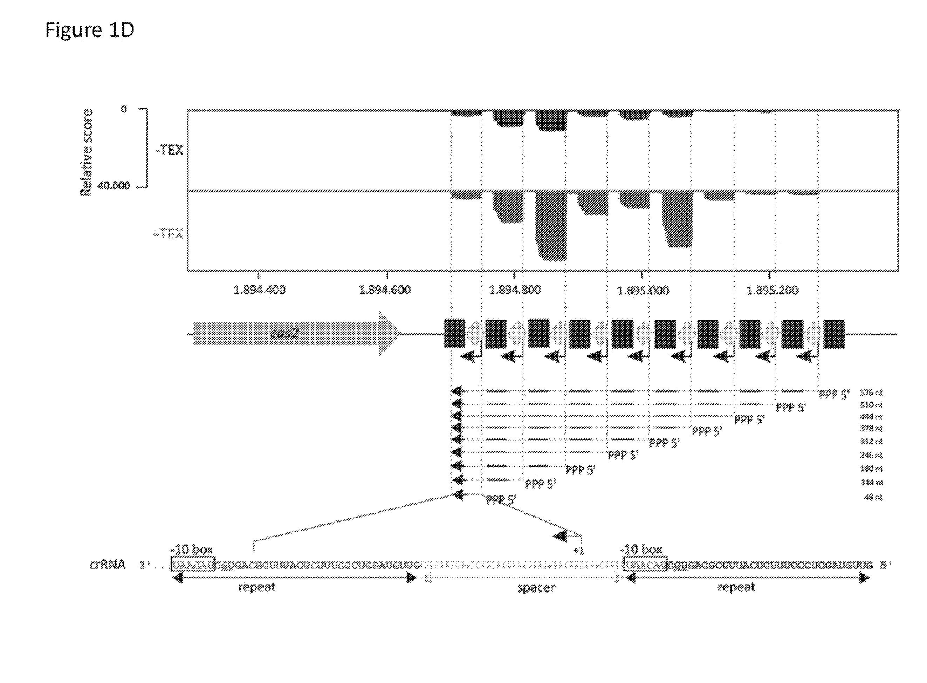

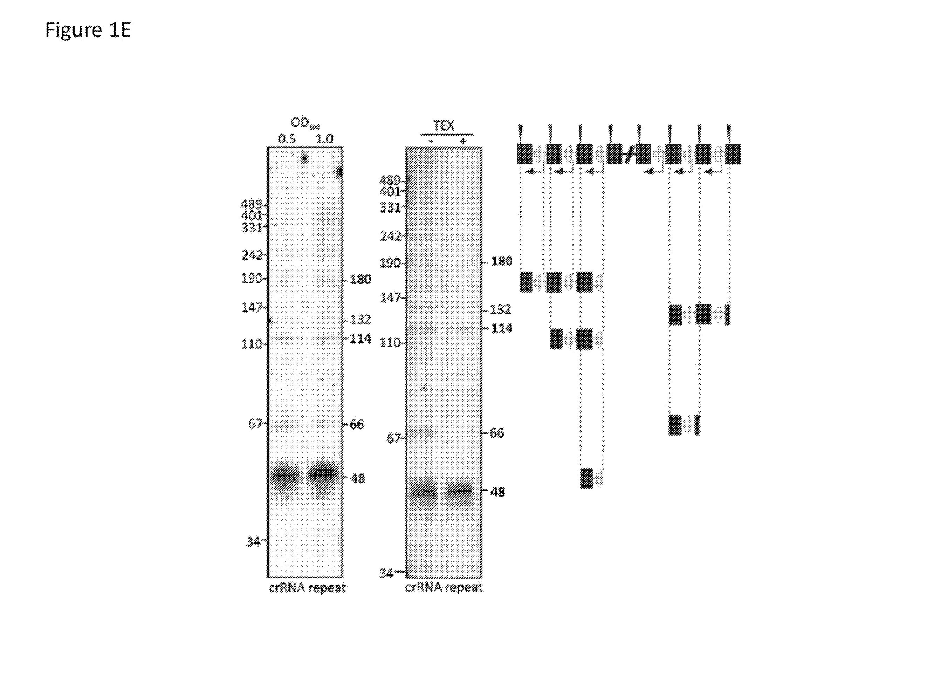

Figure 1. A newly identified mode of crRNA biogenesis in *Neisseria lactamica.* (A) Differential RNA-seq (dRNA-seq)-based analysis of the minimal CRISPR/Cas system in *N. lactamica* 020-06 reveals expression of tracrRNA and crRNAs. Approximately three million cDNAs from untreated and TEX-treated RNA were sequenced and mapped to the genome. Read counts are plotted here for the CRISPR/*cas* locus. Both strands of all libraries were adjusted to the same scale (maximum of 50,000 for leading strand; minimum of -50,000 for lagging strand) that reflects a relative expression score. The number of reads obtained for tracrRNA and crRNAs are in the range of 40,000 to 50,000 each, which is comparable to the range we observe for other high-abundance classes of RNAs. (B) Top: dRNA-seq data were mapped onto the genomic region corresponding to the *tracrRNA* gene. Expression scores at each position were adjusted to the same relative scale. Bottom: sequence of the full-length 107-nt form and processed 91-nt form of tracrRNA. The arrow indicates the primary tracrRNA transcription start site (TSS) based on enrichment in the TEX+ libraries. Boxes denote the extended -10 promoter element and the anti-CRISPR-repeat region of the tracrRNA. (C) Northern analysis of *N. lactamica* 020-06 tracrRNA. OD₆₀₀ (left panel) and TEX treatment (right panel) is denoted at the top of each lane. Processed and unprocessed tracrRNAs are schematized on the right, with the RNase III processing site indicated with an arrowhead and the anti-repeat region indicated as the left-hand box. (D) Top: dRNA-seq data were mapped onto the genomic region corresponding to the CRISPR array. Expression scores at each position were adjusted to the same relative scale. Middle: the primary TSS of each CRISPR spacer-repeat unit (based on enrichment in the TEX+ libraries) is indicated by an arrow. Primary CRISPR transcripts of different lengths with likely 5' triphosphates also are indicated by arrows. Bottom: The sequence of spacer 4 and its flanking CRISPR repeats, with the putative extended -10 box (consensus sequence 5'-tgnTACAAT-3') in each single repeat. (E) Northern analysis of *N. lactamica* 020-06 crRNA, using a probe complementary to the CRISPR repeat. OD₆₀₀ (left panel) and TEX treatment (right panel) is denoted at the top of each lane. Candidate monomeric, dimeric and trimeric crRNAs are schematized on the right, each of a predicted size consistent with bands observed on the blots.

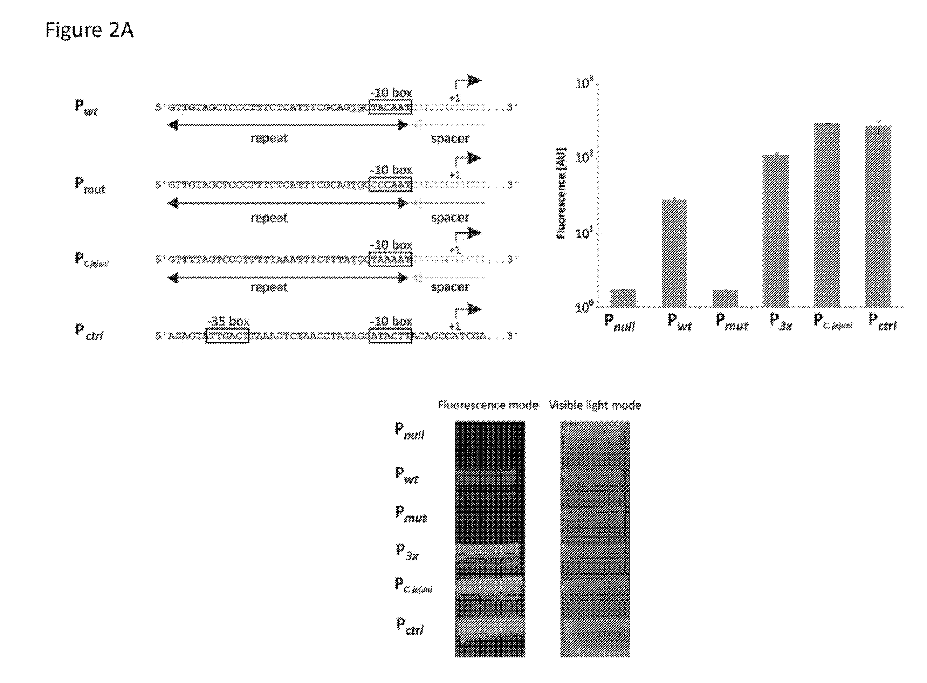

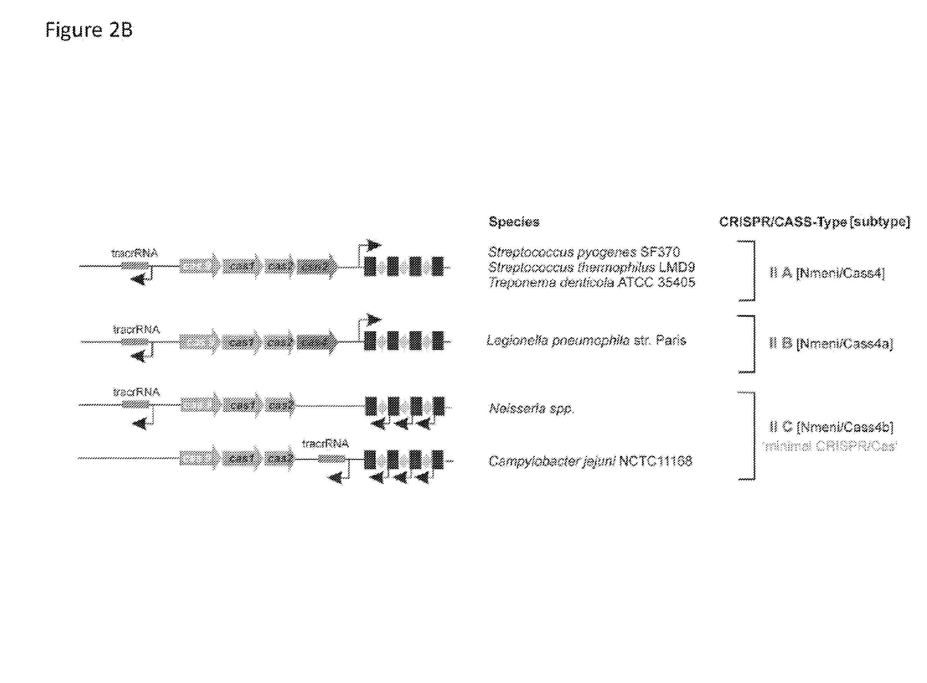

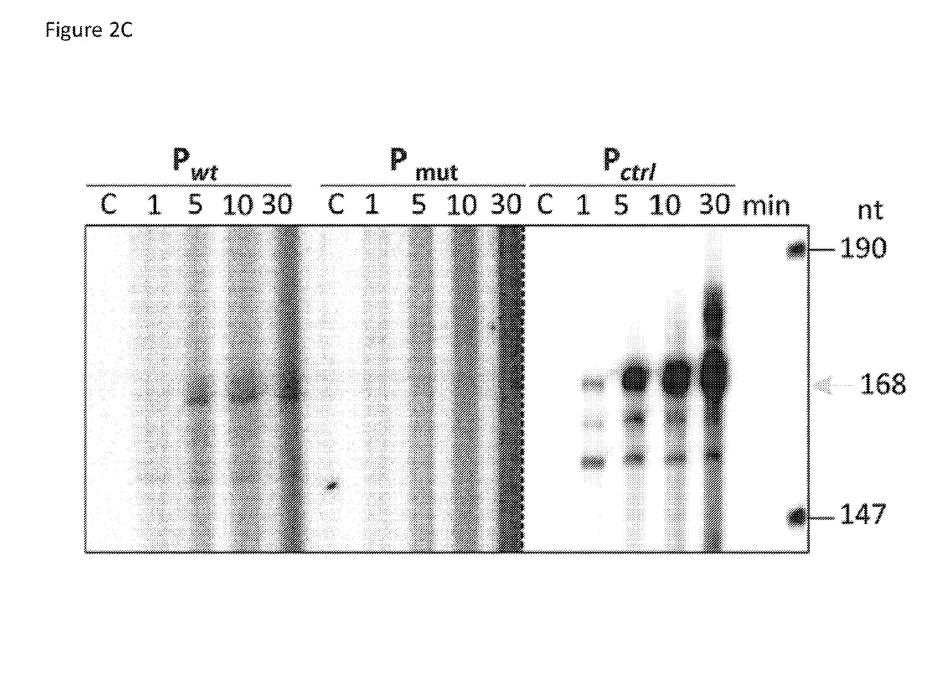

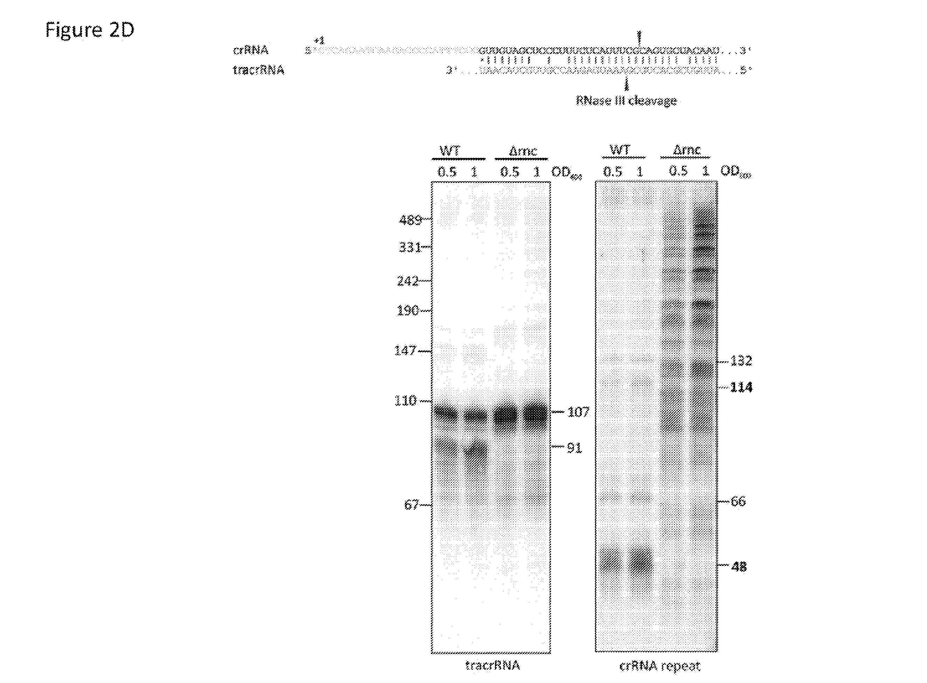

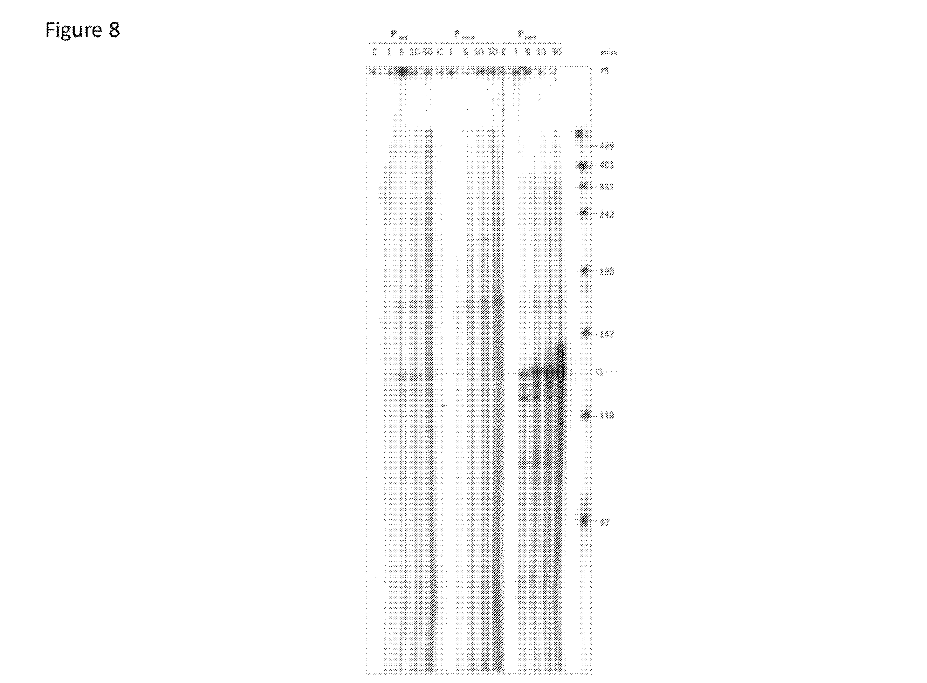

Figure 2. CRISPR repeats contain active promoters that form the 5' ends of mature crRNAs, whereas RNase III processing forms crRNA 3' ends. (A) Top left panel: Promoter-element-containing sequences used for *gfp* fusions. Plasmids included a wild-type (wt) and mutated (mut) *N. lactamica* 020-06 CRISPR repeat (pNH13 and pNH14, respectively), a *C. jejunii* NCTC11168 CRISPR repeat (pNH18), and a positive control promoter from T7 phage A1. The promoterless control *gfp* construct (pAS00046) and a construct with three wildtype *N. lactamica* 020-06 CRISPR repeats (pNH17) are not shown. Promoter elements are indicated by boxes. P*ₘᵤₜ* has a TA→CC mutation in the promoter element as compared to P*_{wt}*. Top right panel: Flow-cytometric fluorescence intensity measurements of cells containing the transcriptional *gfp* fusions described in the left panel. Fluorescence values are expressed in arbitrary units (AU). Error bars indicate standard deviation for three independent biological replicates. Bottom panel: Fluorescence images of transcriptional *gfp* fusion strains grown on agar plates. The right image was captured in the visible light mode; the left image shows the same plate in fluorescence mode (460 nm excitation, 510 nm emission). (B) Classification of Type II CRISPR/*cas* loci. The genomic organization of representative Type II-A or Type II-B loci, as defined previously, is given on top. Below are two CRISPR/*cas* loci (including from neisseriae) from the newly defined Subtype II-C. (C) Gel electrophoresis of radiolabeled RNAs from *in vitro* transcription reactions using linear DNA templates from a subset of the CRISPR repeat-containing sequences given in (A). Full-length run-off transcripts (168 nt) are denoted by the arrow. The area to the right of the dotted line was imaged at lower contrast to avoid overexposure. See also Figure 8. (D) Top: Base pairing between a primary crRNA and tracrRNA. RNase III cleavage sites inferred from dRNA-seq are indicated by arrows. Bottom: Northern analysis of total RNA from *N. meningitidis* WUE2594 and its Δ*rnc* derivative during mid-log and early stationary phase, probed for tracrRNA (left) and crRNAs (right).

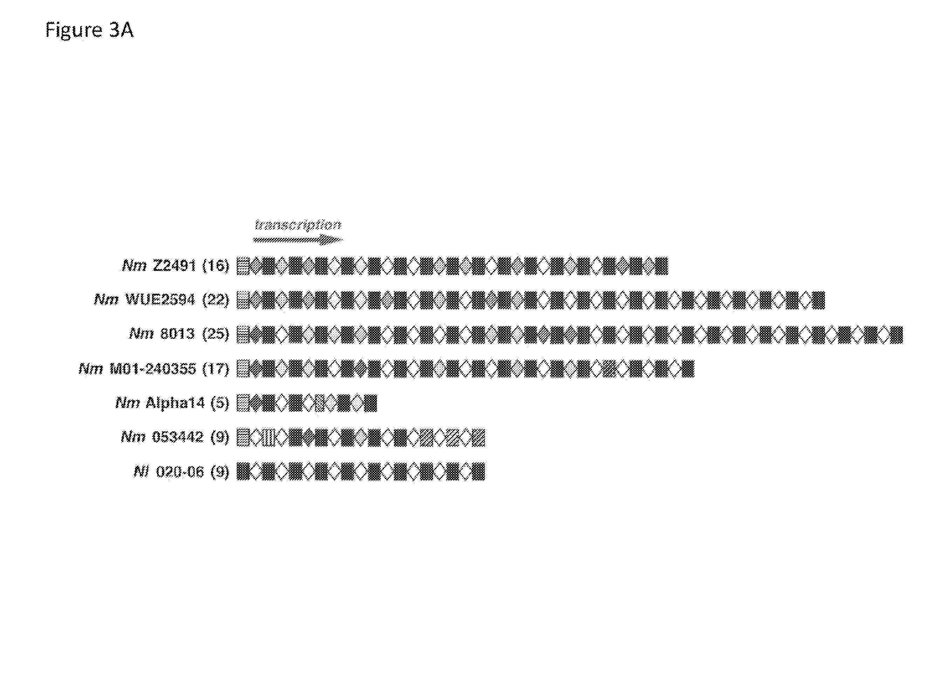

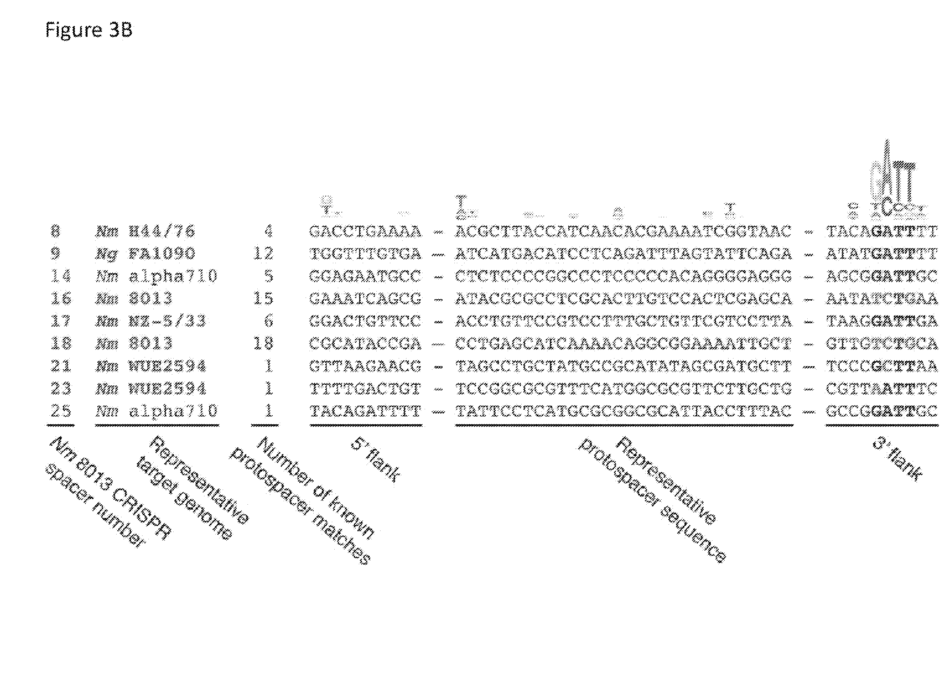

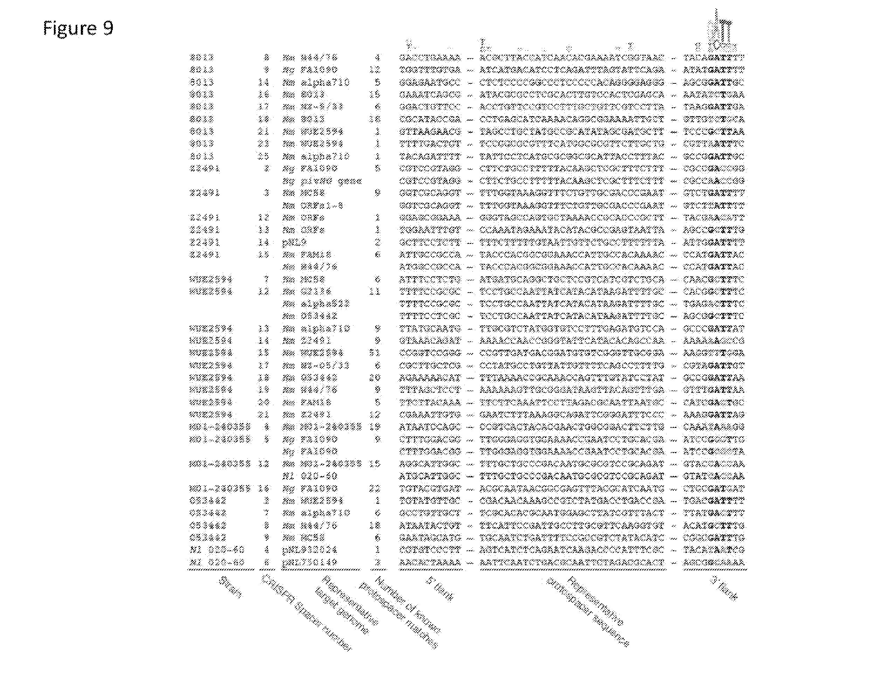

Figure 3. CRISPR organization in *Neisseria.* (A) Schematic representation of CRISPR loci from seven different *Neisseria* strains. Strain names are indicated (*Nm, N. meningitidis; Nl, N. lactamica*) with the total number of spacers in each strain shown in parentheses. The arrow indicates the direction of crRNA transcription. Repeats and spacers are shown as rectangles and diamonds, respectively. Unique spacers, repeats that match the consensus, and variant repeats are illustrated (see Tables 2 and 3). (B) Potential natural targets for *N. meningitidis* 8013 spacers. 9 out of 25 spacers match varying numbers of *Neisseria* genomes. For each spacer, its number in the 8013 array, the quantity of known protospacer matches, and a representative target genome (*Nm, N. meningitidis; Ng, N. gonorrhoea*) are listed. Protospacers and 10 flanking nts (on both sides) from the representative target genomes are aligned. Sequence similarities are indicated at the top, revealing the 5'-GATT-3' PAM consensus 5-8 nts 3' of the protospacer. The WebLogo is derived from the alignment of all *Neisseria*-matched protospacers, not just those shown here that match spacers from the *N. meningitidis* 8013 CRISPR. The PAM regions and non-consensus nucleotides are illustrated. Potential self-targeting spacers and spacers with possible prophage-like targets are illustrated. (See also Figure 9).

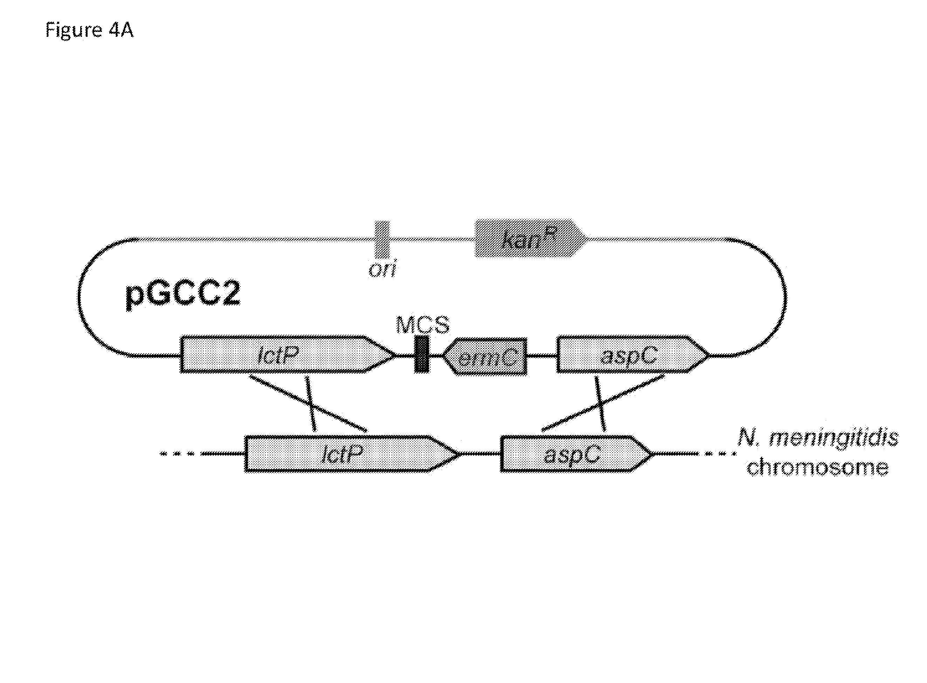

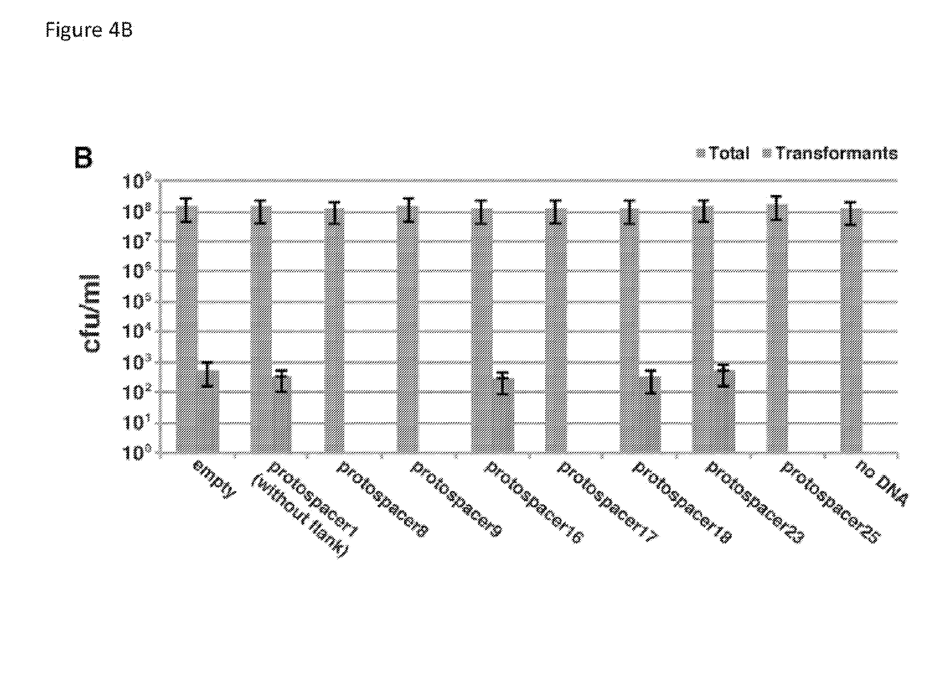

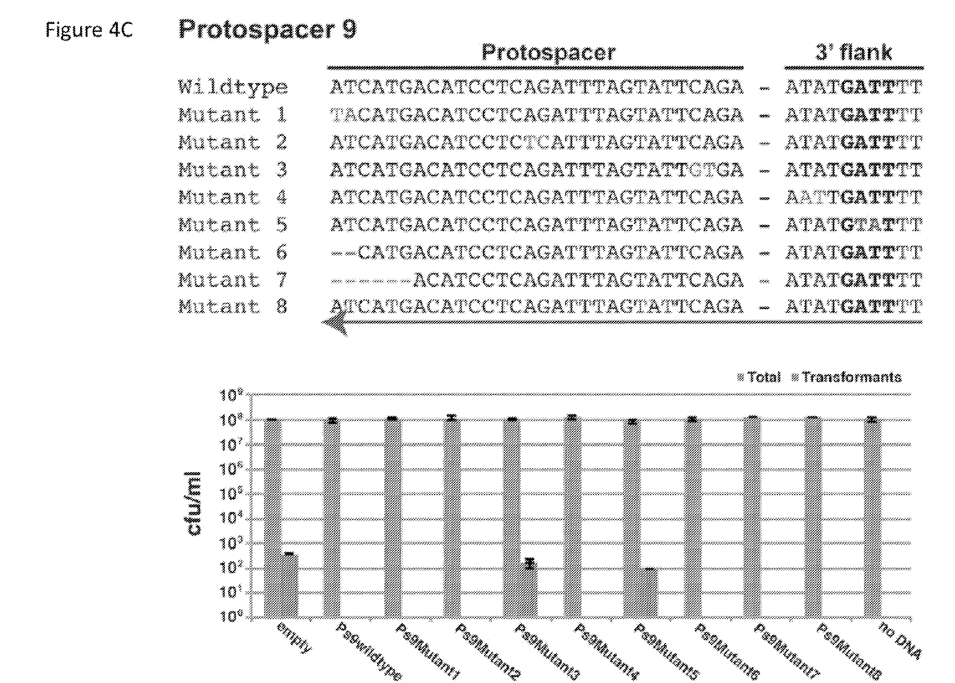

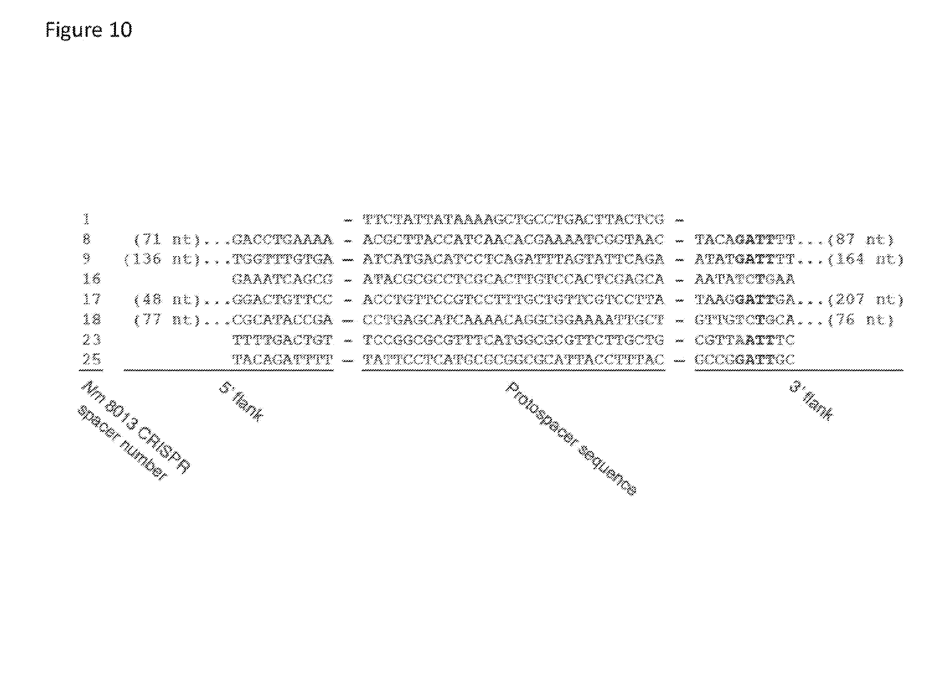

Figure 4. Natural transformation is limited by the native *Neissiera* CRISPR/Cas system. (A) Schematic representation of integrational vector pGCC2, and recombination between pGCC2 and the meningococcal chromosome (Tobiason and Seifert, 2010). Individual elements are not drawn to scale. (B) pGCC2 derivatives with potential targets for different *N. meningitidis* 8013 spacers (1, 8, 9, 16, 17, 18, 23, and 25) were tested by natural transformation assays using wild-type 8013 as the recipient strain. The data show log-scale plots of colony-forming units (cfu) per ml [mean ± s.e.m. (standard error of the mean) for three independent biological replicates] for total cells (left bars) and Erm^{R} transformants (right bars) from three independent experiments. See also Figure 10. (C) Top panel, sequences of a series of mutations in the pGCC2 derivative carrying the 350 nt target for spacer 9. The arrow indicates reversed orientation of the target sequence in the plasmid. Bottom panel, pGCC2 constructs containing the spacer 9 target mutations are tested by natural transformation into wild-type *N. meningitidis* 8013. Data are presented as in (B). Error bars represent s.e.m. for three independent biological replicates.

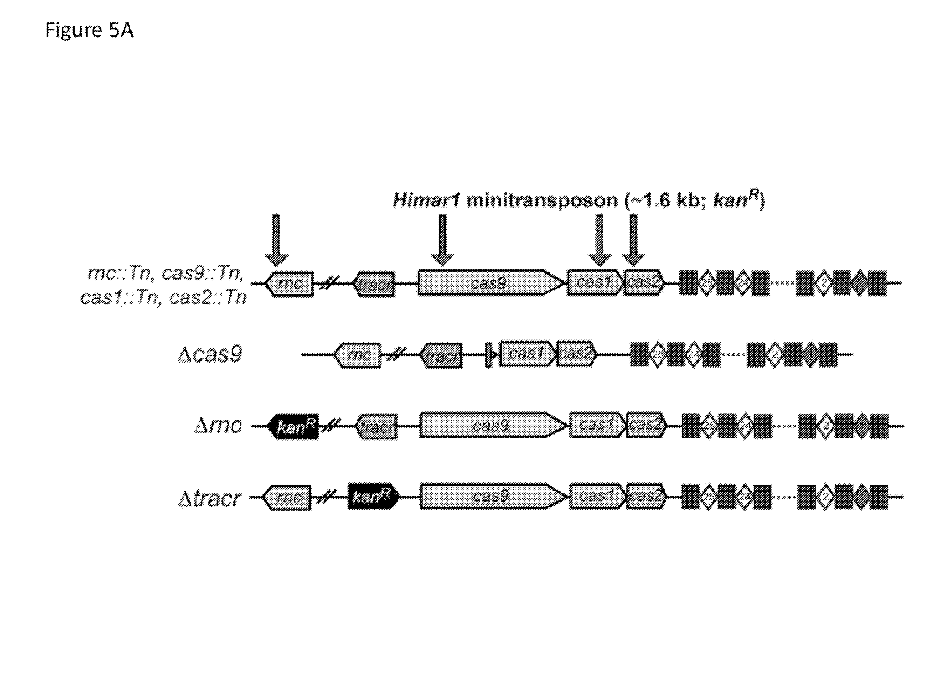

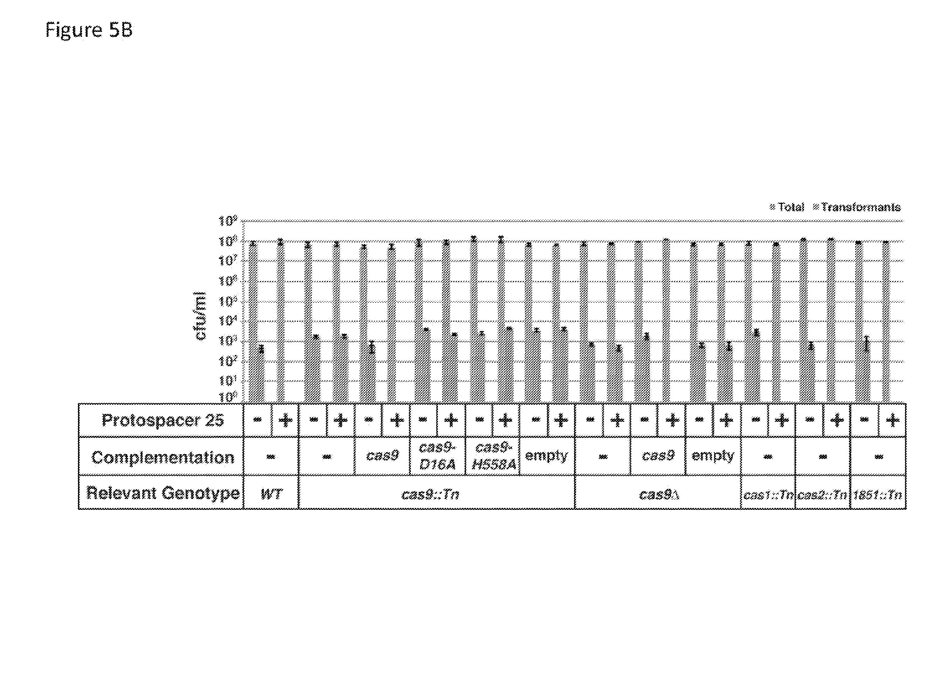

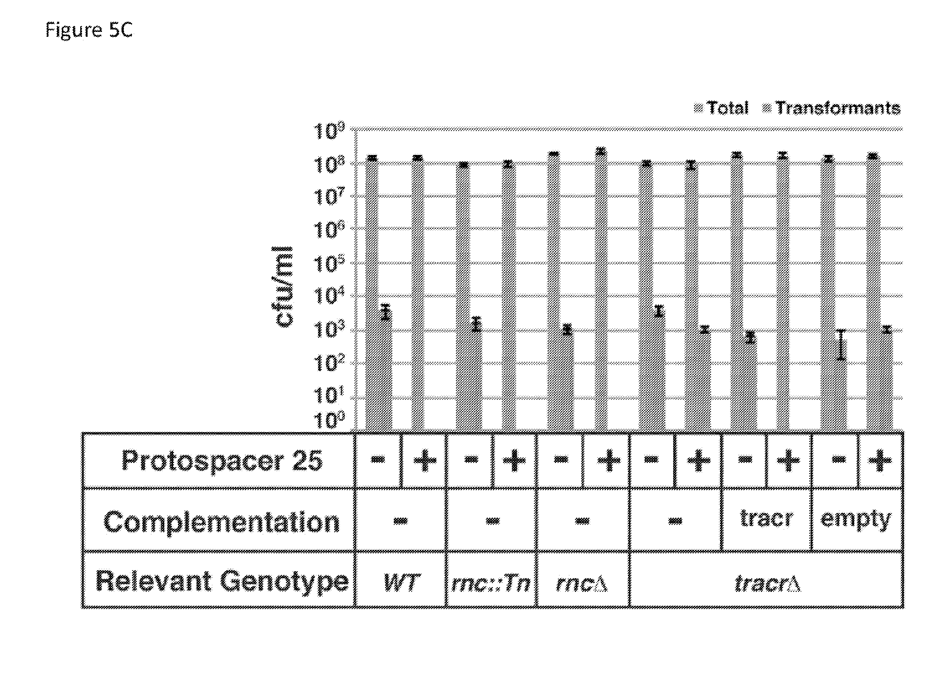

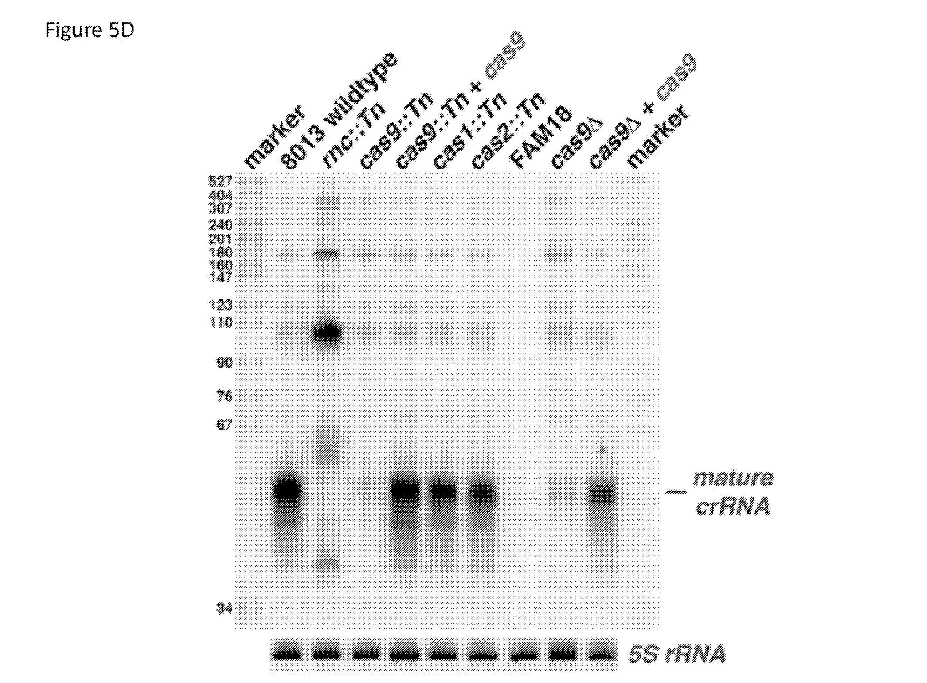

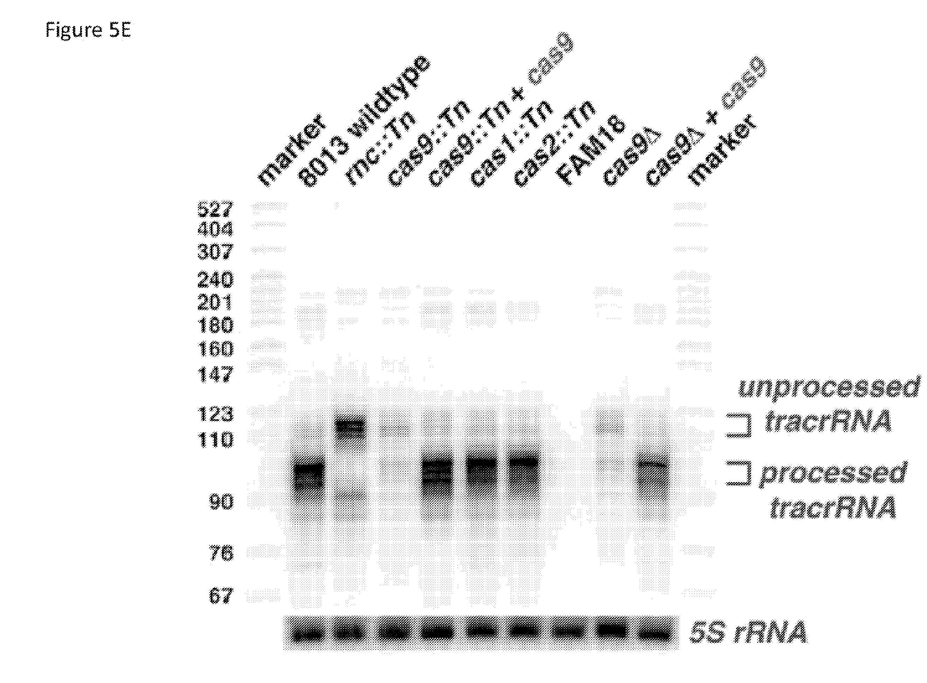

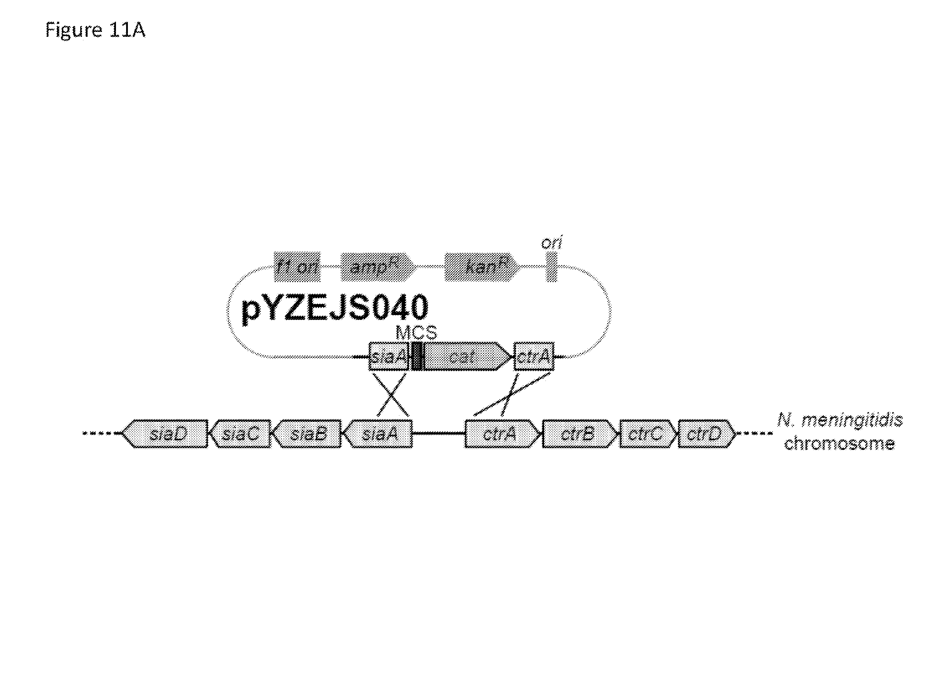

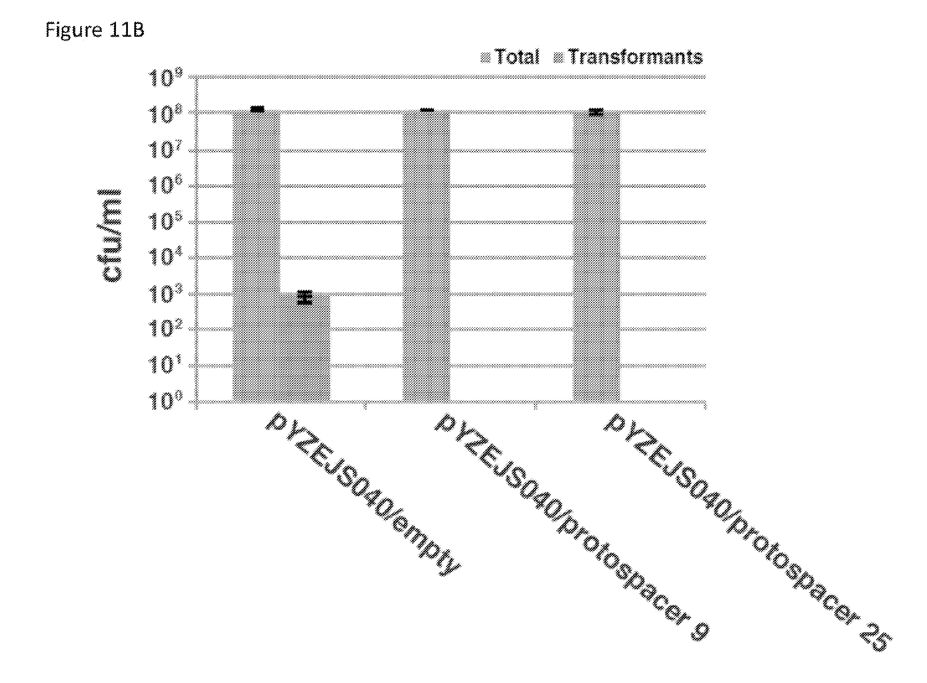

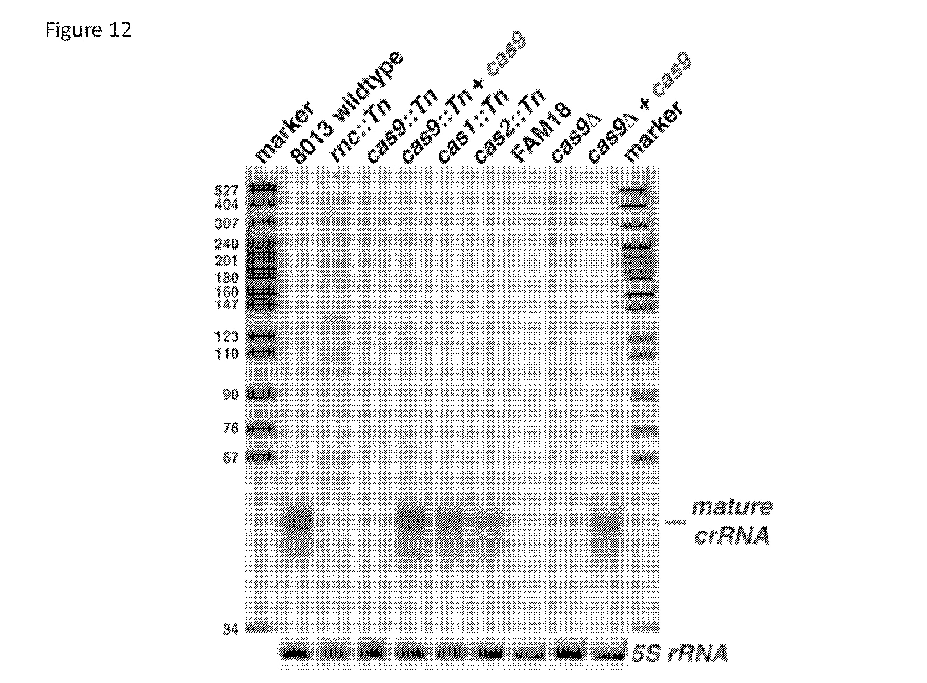

Figure 5. Type II-C CRISPR interference requires Cas9 and tracrRNA but is independent of RNase III-mediated processing. (A) Schematic representations of *N. meningitidis* 8013 mutant strains: including *cas1, cas2, cas9* and *rnc* genes; *tracrRNA* gene; kanamycin resistance gene; and CRISPR repeats and spacers (squares and diamonds, respectively). Arrows indicate transposon insertions in the *rnc::Tn, cas9::Tn, cas1::Tn,* and *cas2::Tn* mutants. (B, C) pYZEJS040 (-) and its protospacer 25-containing derivative (+) were tested by natural transformation assays using *N. meningitidis* 8013 and its mutant derivatives as recipients. Relevant genotypes as well as the presence or absence of pGCC2-mediated *cas9* complementation are given at the bottom. The data show cfu/ml (log scale, mean ± s.e.m. for three independent biological replicates) for total cells (left bars) and chloramphenicol-resistant transformants (right bars). (See also Figure 11). (D) Total RNA from the indicated strains were subjected to northern analysis using a probe complementary to spacer 25 (top). In the lower panel, the same blot was probed for 5S RNA as a loading control. (See also Figure 12). (E) As in (D), except that a probe specific for tracrRNA was used.

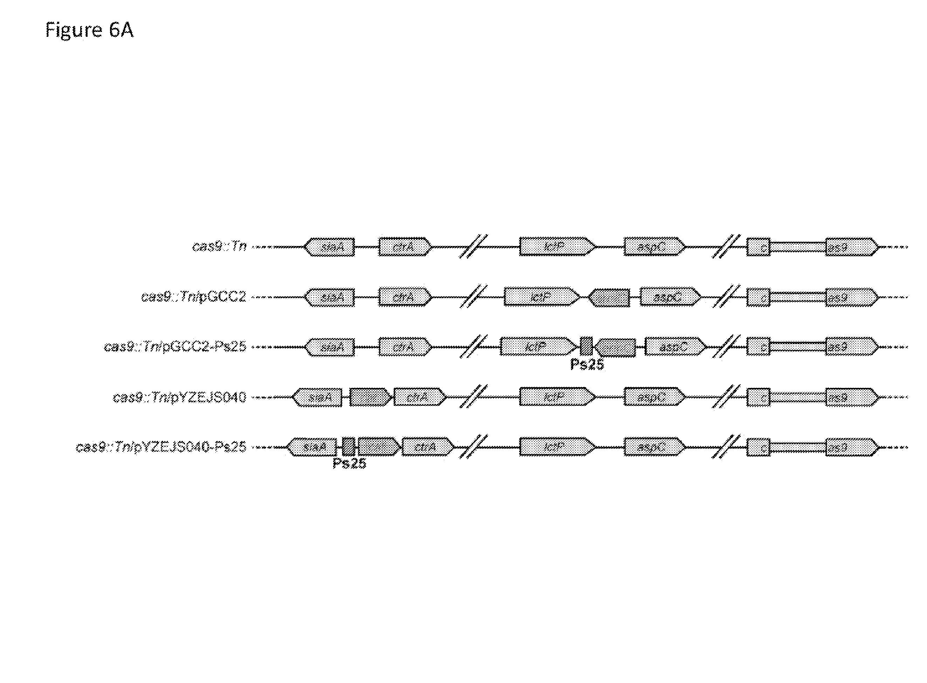

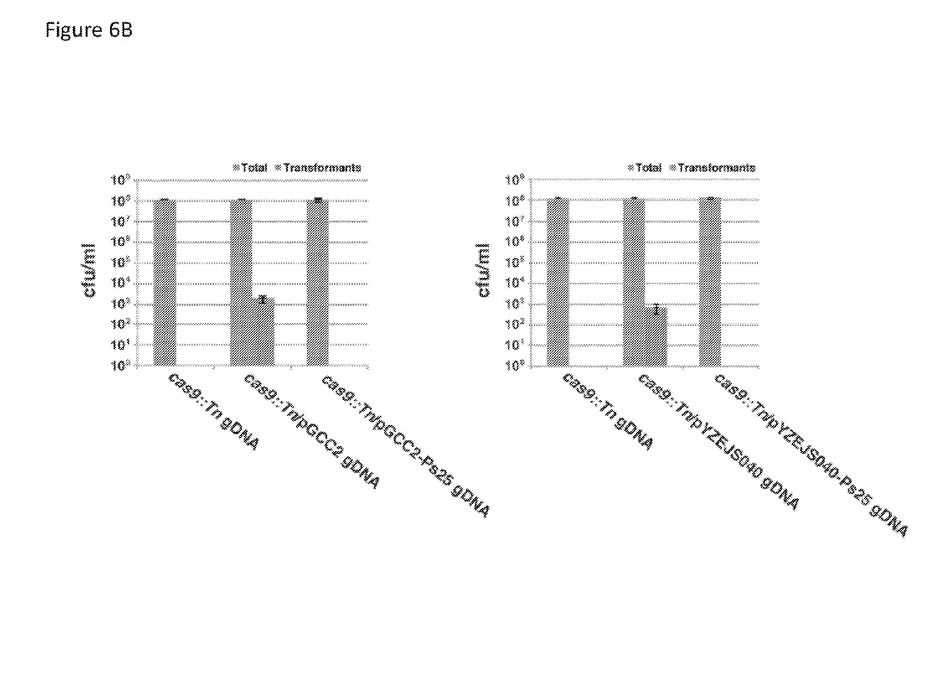

Figure 6. *Neisseria* type II-C CRISPR/Cas limits natural transformation of meningococcal chromosomal DNA. (A) Schematic representation of the *siaA*/*ctrA* and *lctP*/*aspC* chromosomal loci in the *cas9::Tn* strain (top; with the transposon insertion indicated). Below are derivatives of the same *cas9::Tn* strain following transformation with the *ermC*-marked vector pGCC2 with or without a protospacer that matches CRISPR spacer 25, or with the *cat*-marked vector pYZEJS040 with or without a protospacer that matches CRISPR spacer 25. (B) For the left panel, gDNA from the *ermC*-marked strains shown in (A), as well as the unmarked control strain, was used in transformation assays with wild-type *N. meningitidis* 8013. Natural transformations were performed and presented as in Figure 4B. The right panel shows an analogous experiment using gDNA from the *cat*-marked strains shown in (A), as well as the unmarked control strain. Error bars represent s.e.m. for three independent biological replicates.

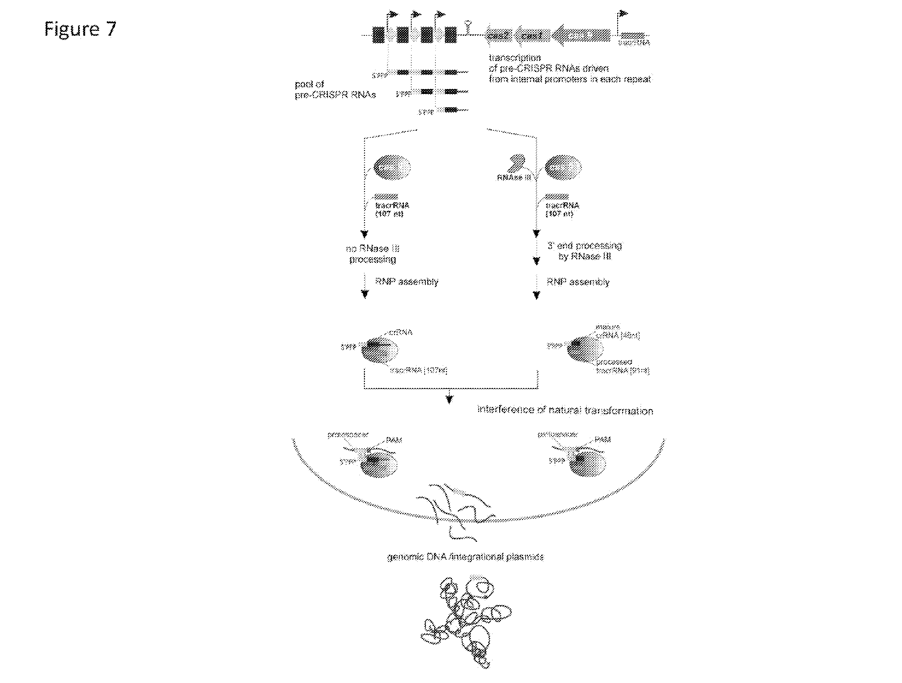

Figure 7. CrRNA biogenesis and CRISPR interference in *Neisseria.* Type II-C CRISPR/*cas* loci in *Neisseria* spp. initiate transcription within each spacer, driven by promoter elements embedded within each repeat. The resulting crRNAs and pre-crRNAs carry 5'-terminal triphosphates. Following tracrRNA annealing, RNase III can cleave both strands of the tracrRNA/pre-crRNA duplex (right pathway). Unexpectedly, pre-crRNA processing is not required: when RNase III is unavailable or fails to act, Cas9 can still form functional complexes with tracrRNA and crRNA (left pathway). The naturally-encoded crRNAs target sequences present in other *Neisseria* spp. chromosomes, consistent with the high frequency of genetic exchange by natural transformation. Because Type II-C have only three protein-coding genes, lack leader sequences upstream of the CRISPR array, and do not require the host factor RNase III, they are the most streamlined natural CRISPR/Cas systems known.
Figure 8. *In vitro* transcription assay with *E. coli* σ⁷⁰ polymerase (RNAP) holoenzyme with linear DNA templates containing either a wild-type or a mutant repeat. The complete gel of the experiment from Figure 2C is shown. The area to the right of the dotted line was imaged at lower contrast to avoid overexposure.
Figure 9. Alignment of potential natural targets for all *Neisseria* spacers revealed a putative 3' PAM. A total of 35 unique *Neisseria* spacer sequences have potential matches to varying numbers of *Neisseria* genomes or plasmids. For each of them, spacer number, the number of known protospacer matches, and one representative target genome are listed. Protospacers and 10 nts of 5' and 3' flanking sequences are aligned and the resulting sequence logos are shown on the top. A putative 3' PAM (5'-NNNNGATT-3') is deduced. PAM equivalent regions in the targets are illustrated as well as nucleotides differing from consensus PAM. The five spacers with "self-targeting" potential, their spacer name and representative target genome also are indicated. Spacers with at least one phage-related potential natural target, their spacer number and representative target genome also are indicated.
Figure 10. Protospacer-containing potential targets cloned into pGCC2. For each protospacer, the number of the matching spacer in the *N. meningitidis* 8013 CRISPR array is listed. Representative protospacers and 10 flanking nts on both sides are aligned. Potential targets for spacers 16, 23, and 25, cloned from synthetic oligonucleotides, include 10 nts on both sides of the protospacers. Potential targets for spacers 8, 9, 17, and 18, cloned from PCR products, include varying numbers (81-217 nt) of 5' and 3' flanking nts, as indicated in parentheses. As a control, Protospacer 1 is cloned without any flanking sequences. The PAM regions in potential targets are illustrated as well as non-consensus nucleotides. Potential self-targeting spacers also are indicated.
Figure 11. *Neisseria* Type II-C CRISPR/Cas limits natural transformation of pYZEJS040 plasmids. (A) Schematic representation of integrational vector pYZEJS040, and recombination between *siaA-MCS-CAT-ctrA* region of pYZEJS040 and the capsule locus of meningococcal chromosomes, including: genes of the capsule biosynthesis operon (*siaA-D*) and the capsule transport operon (*ctrA-D*); *CAT* gene; cloning site (MCS); and regions required for maintenance in *E. coli.* Individual elements are not drawn to scale. (B) pYZEJS040 derivatives with potential natural targets for spacers 9 and 25 are tested by natural transformation using wild-type *N. meningitidis* 8013 as recipient strain. Natural transformations are performed and presented as in Figure 4B, except that chloramphenicol-resistant (Cm^{R}) transformants are scored. Error bars represent s.e.m. for three independent biological replicates. Potential targets for spacers 9 and 25 are identical to those used in Figure 4B.
Figure 12. Northern analysis of the complete crRNA pool in *N. meningitidis.* Total RNAs from the indicated strains were subjected to northern blot analysis using a probe complementary to 1-22nt of repeat (top). Bottom, the same blot probed for 5S RNA as a loading control. Size markers were indicated.

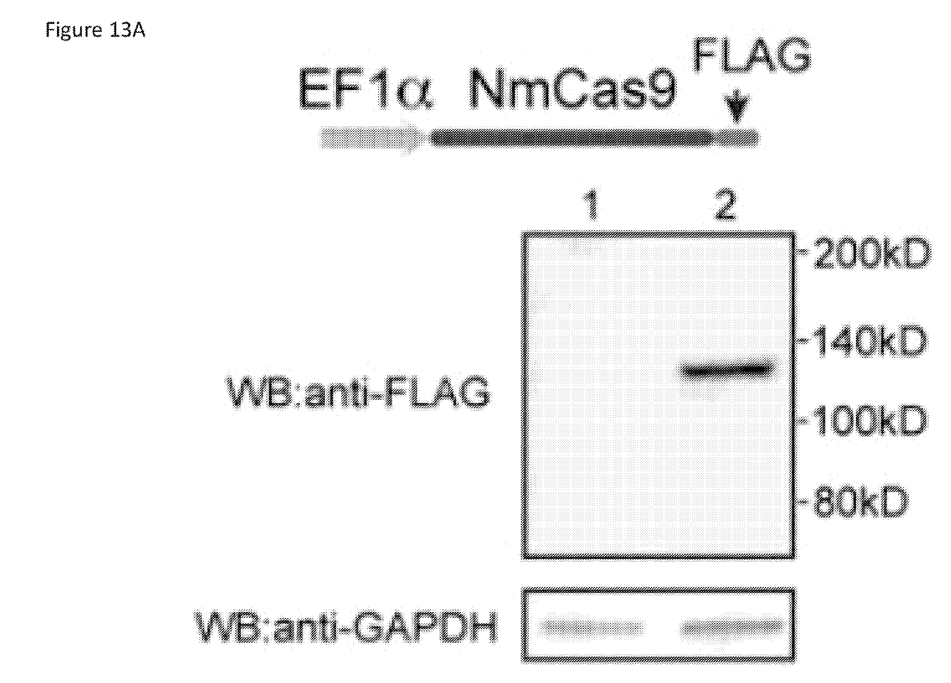

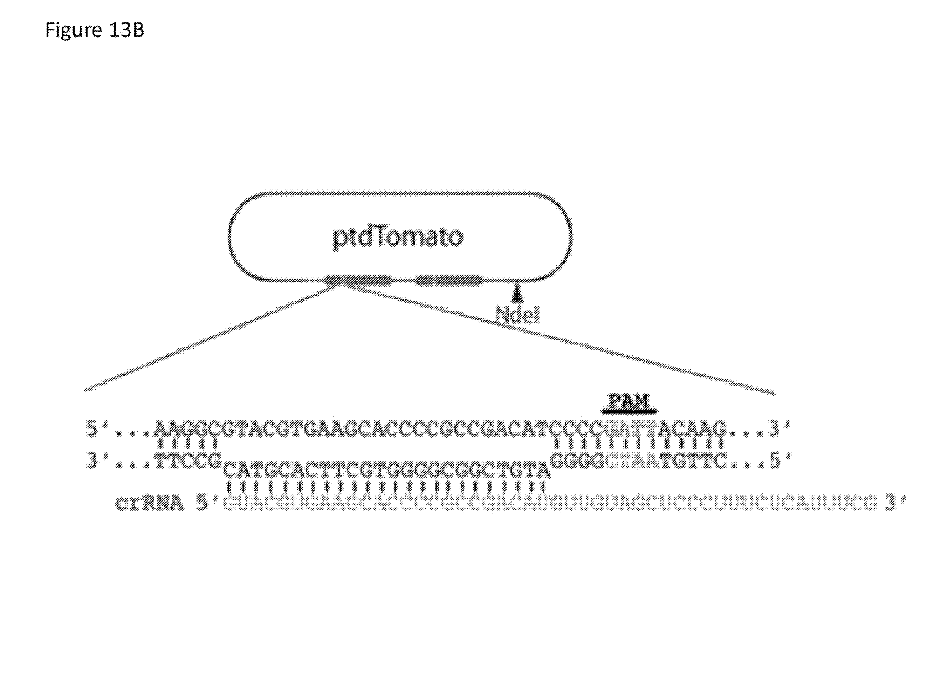

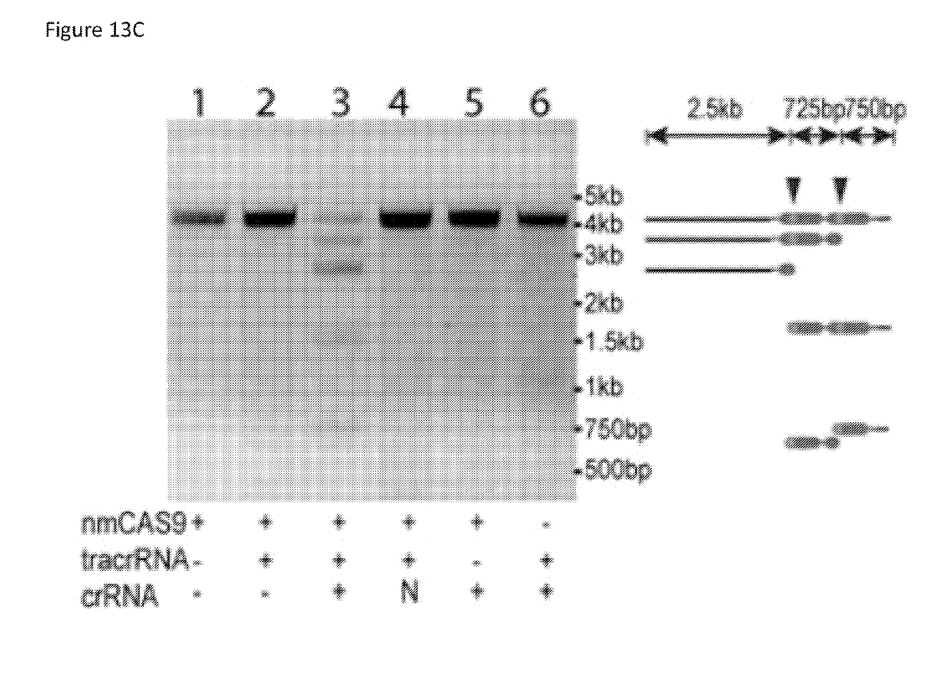

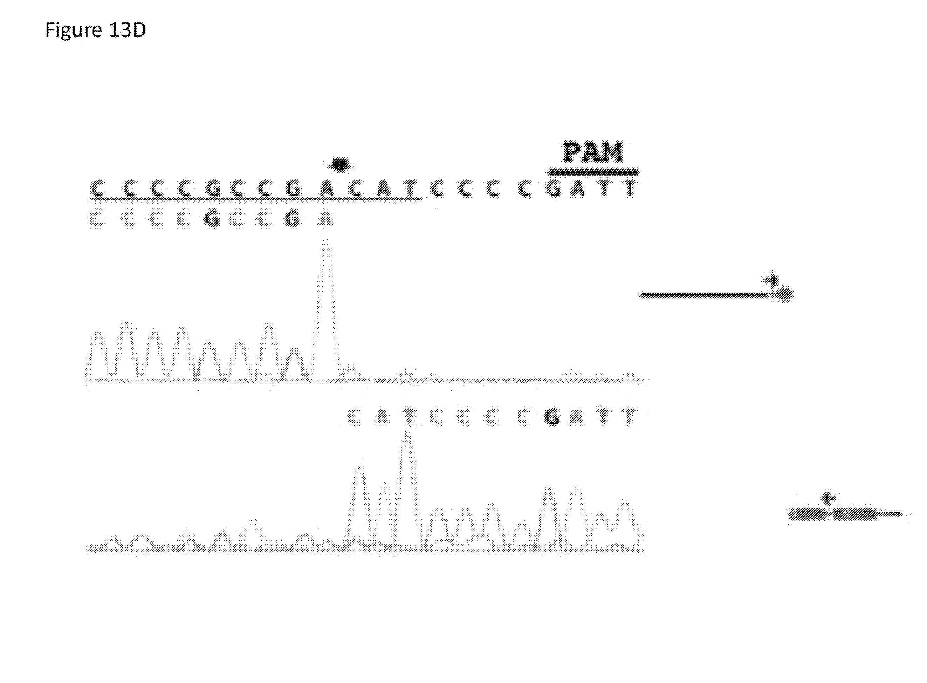

Figure 13. Functional NmCas9 can be expressed in mammalian cells. (*A*) Western blot analysis demonstrates that FLAG-tagged NmCas9 is expressed in 293 cells. Lane 1: Untransfected 293FT cells. Lane 2: 293FT cells transfected with FLAG-tagged NmCas9 expressing plasmid. Upper panel: anti-FLAG tag western. Lower panel: anti-GAPDH western as loading control. (*B*) Design of the crRNA that targets the tdTomato coding sequence; PAM sequence; crRNA spacer; and crRNA repeat. (*C*) The plasmid containing the tdTomato coding sequence (see section *B*) was linearized with *Nde*I and mixed with different combinations of tracrRNA, crRNA and cell lysate prepared from NmCas9-expressing 293FT cells. After incubation at 37°C, DNA was purified and analyzed by agarose gel electrophoresis. The diagram on the right shows the expected cleavage products and their predicted sizes. 'N' indicates inclusion of a nonspecific crRNA that does not target tdTomato. (*D*) Cleavage product (see Section C) was extracted from the gel and analyzed by Sanger sequencing using the primers indicated in the right panel. The cleavage site, indicated by the arrow, was inferred from the sequencing.

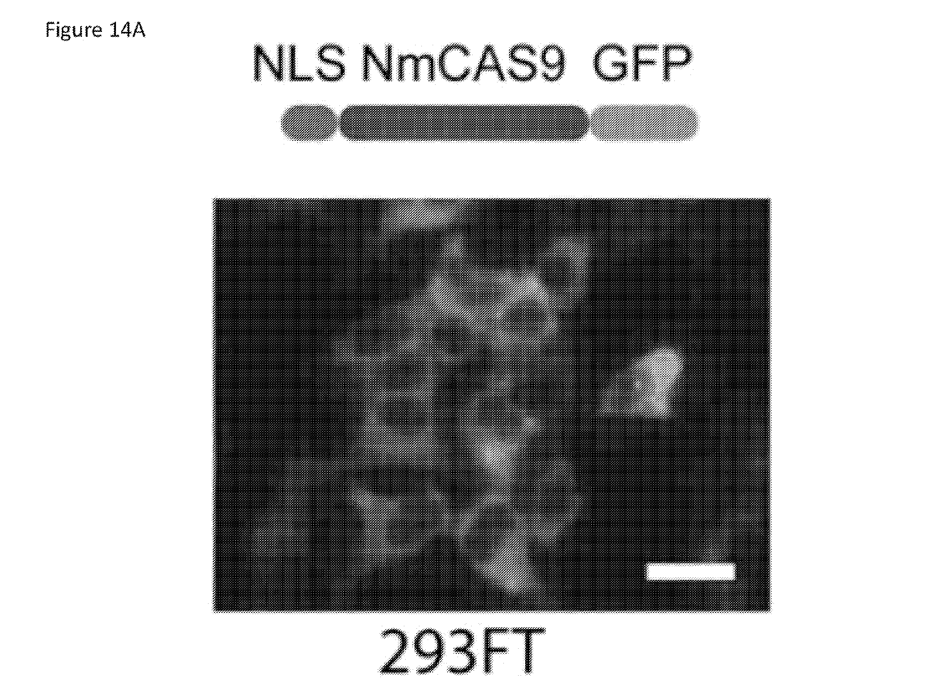

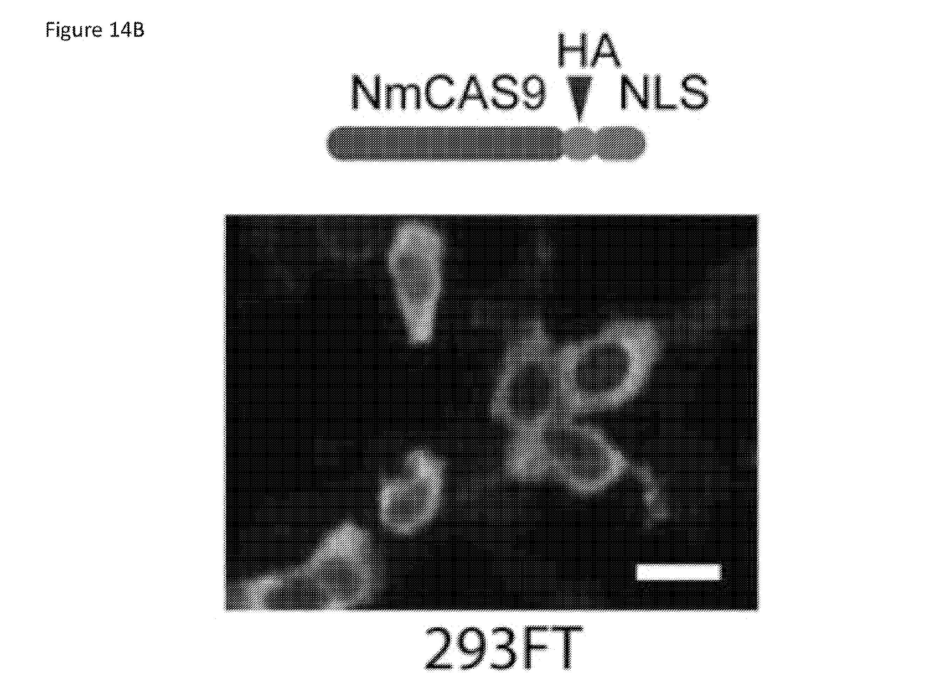

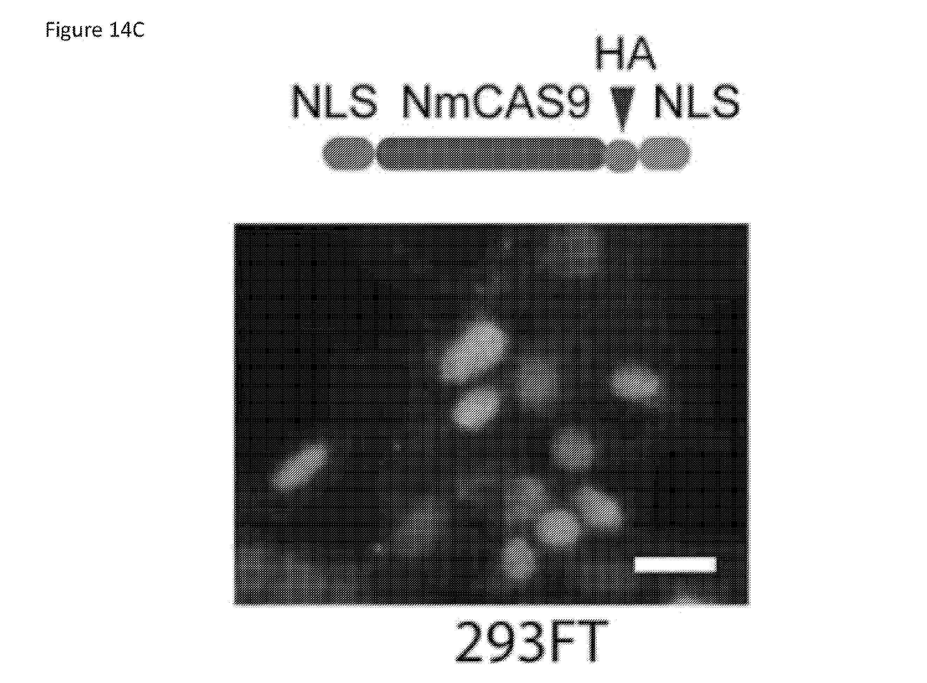

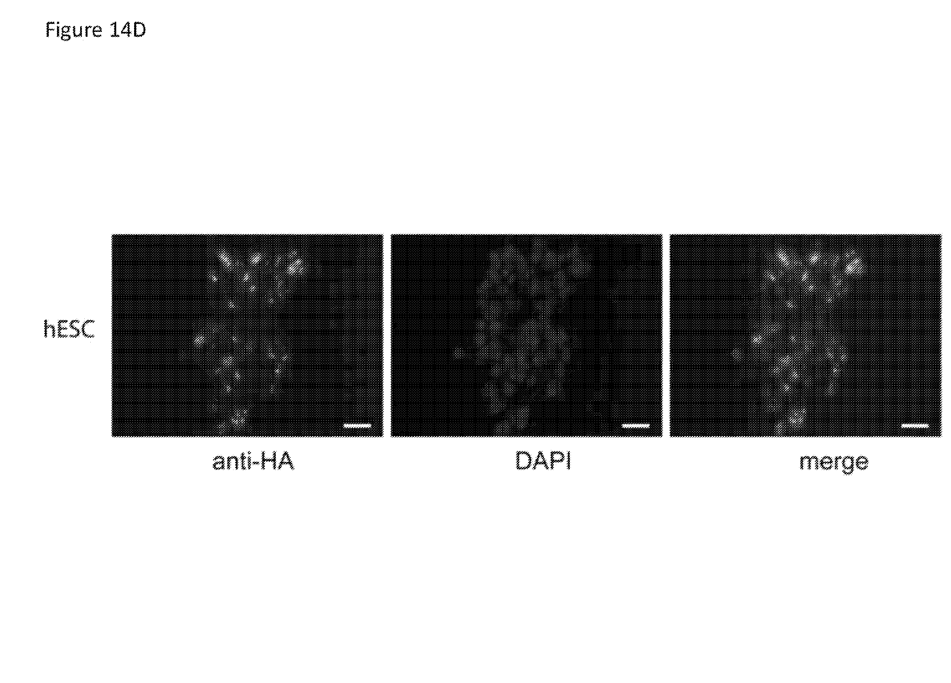

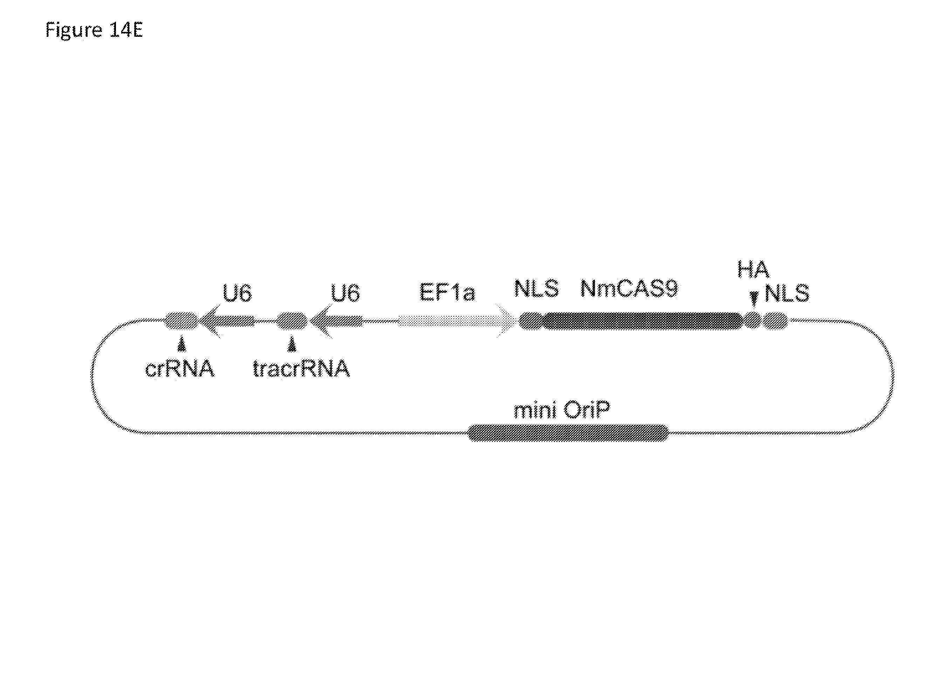

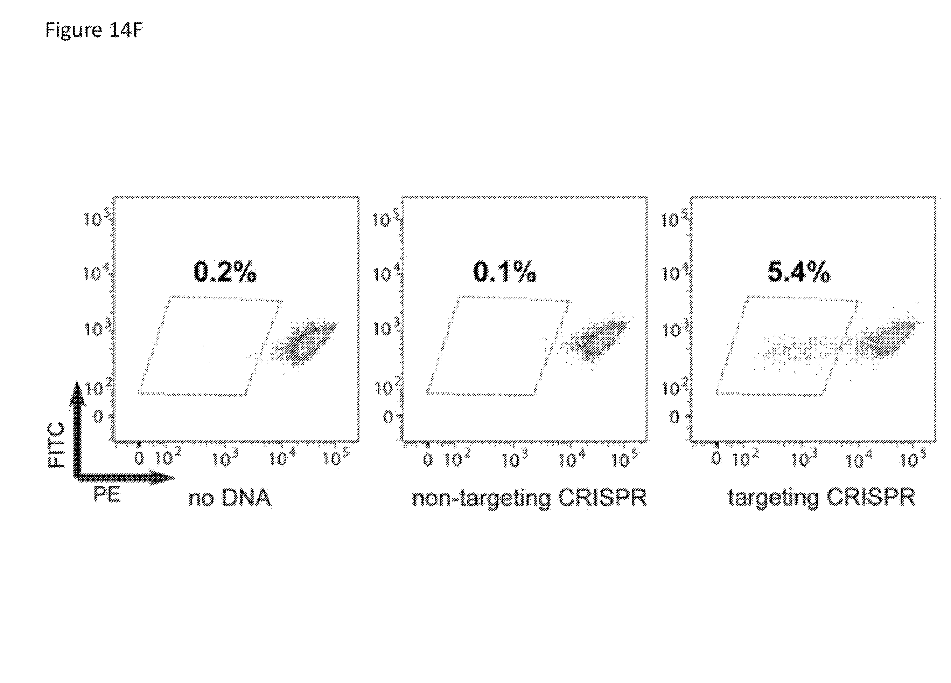

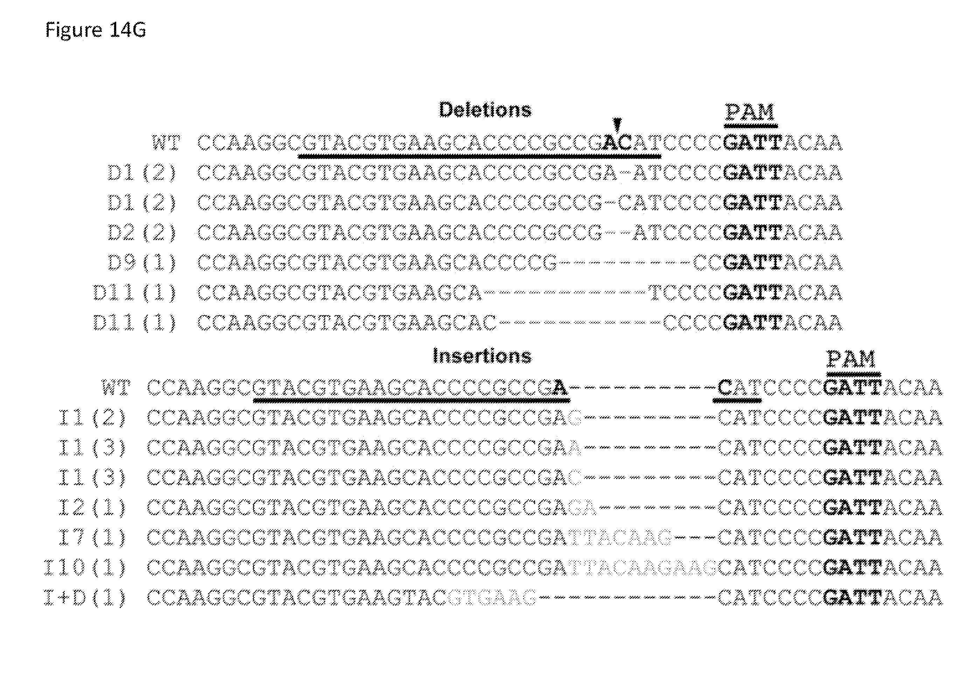

Figure 14. NmCas9 functions in gene disruption in human ES cells. (*A-C*) The localization of NmCas9 with an NLS at the N-terminus (*A*), C-terminus (*B*), or both termini (*C*) was analyzed by either EGFP fluorescence (*A*) or anti-HA immunofluorescence (*B* and *C*) in 293 cells. Scale bar: 20 µm. (*D*) The localization of NmCas9 with the double NLS (see Section C) was analyzed by anti-HA immunofluorescence in human ES cells. Scale bar: 20 µm. (*E*) Design of a single plasmid used for gene editing in hES cells. (*F*) FACS analysis of tdTomato reporter human ES cell lines after electroporation of the indicated crRNA/tracrRNA/NmCas9 constructs. The number in the plot indicates the percentage of tdTomato-fluorescence-negative cells five days after electroporation. (*G*) Indels introduced by the targeting CRISPR in the tdTomato negative population (see Section F) were analyzed by targeted PCR amplification and sequencing. The protospacer sequence is underlined. The numbers in parentheses indicate the number of sequenced clones containing that specific indel.

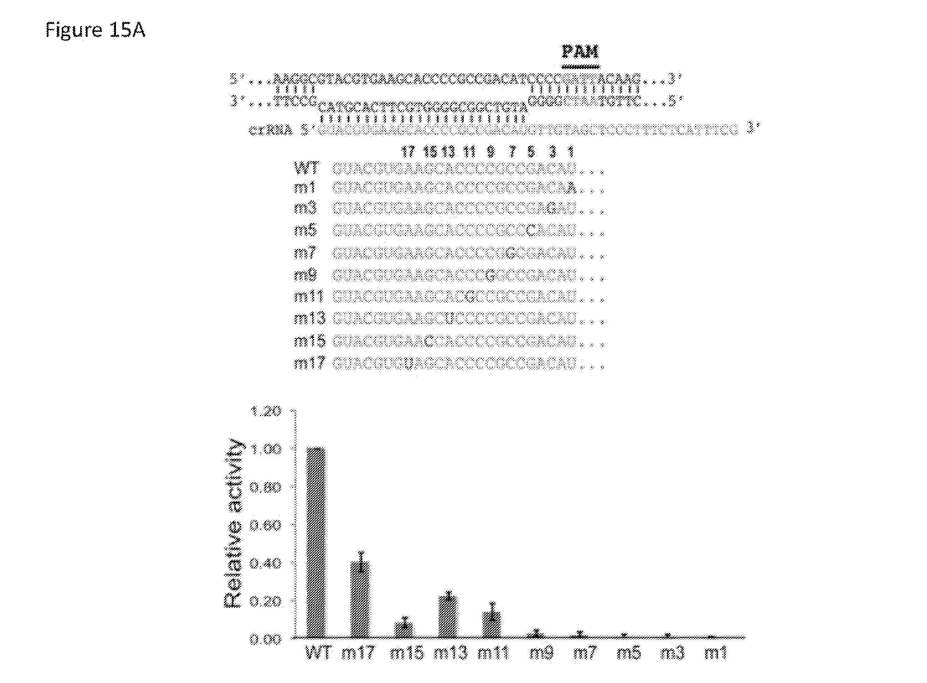

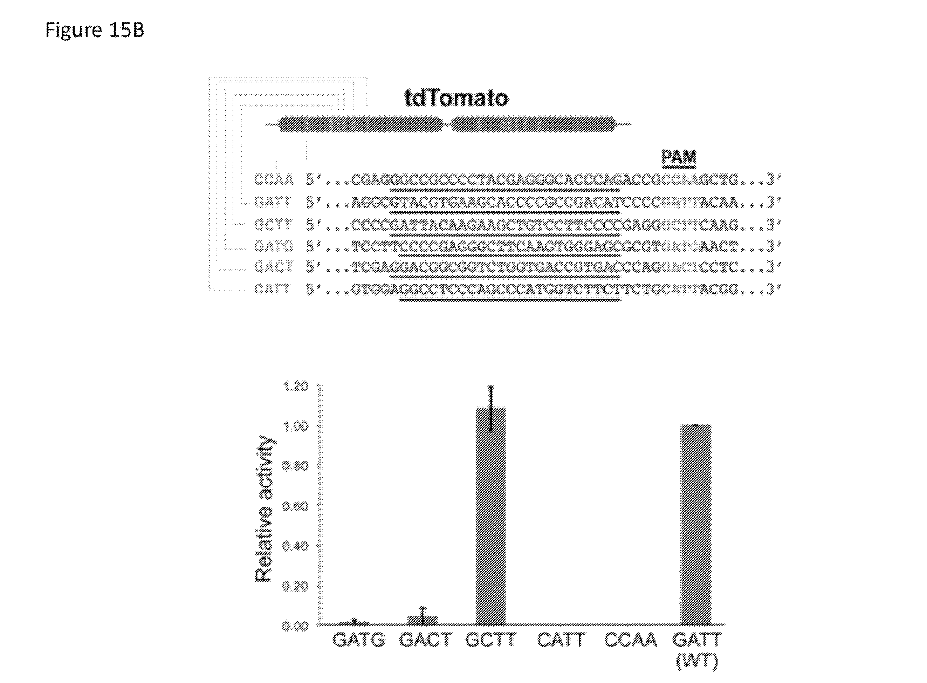

Figure 15. Specificity screen of NmCas9 system. (*A*) The top panel shows the crRNA sequence targeting tdTomato, with locations of single point mutations in the spacer region of the tdTomato targeting crRNA. The bottom panel shows the efficiency of each mutant at disrupting tdTomato expression. All mutants were tested as described in Figure 2, Section F. The efficiency is defined as percent tdTomato-negative cells (mutant spacer) divided by percent tdTomato-negative cells (wildtype spacer). Error bars: S.E.M. (*B*) Top panel depicts the locations of different mutant PAMs in the tdTomato sequence including: wildtype PAM; mutant PAM; and spacer sequence (underlined). For the bottom-most protospacer, the opposite strand was targeted, and the reverse complement sequence is therefore shown. The bottom panel shows the efficiency of targeting at each site associated with the indicated PAM, as revealed by the loss of tdTomato expression. All targeting experiments were performed as described in Figure 2, Section F. The efficiency is defined as percent tdTomato-negative cells (mutant PAM) divided by percent tdTomato-negative cells (wildtype PAM). Error bars: S.E.M.

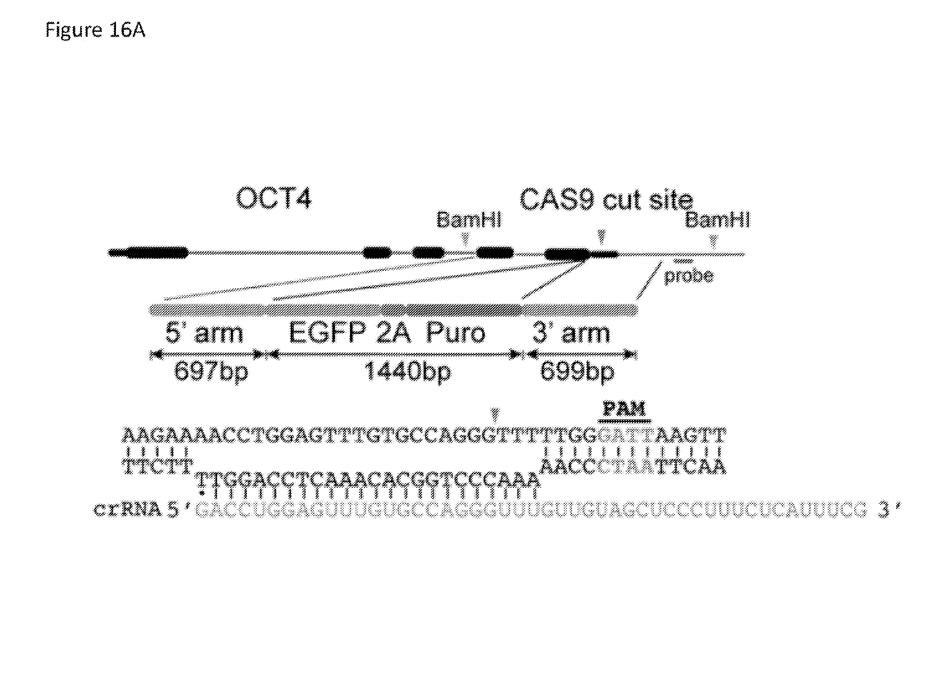

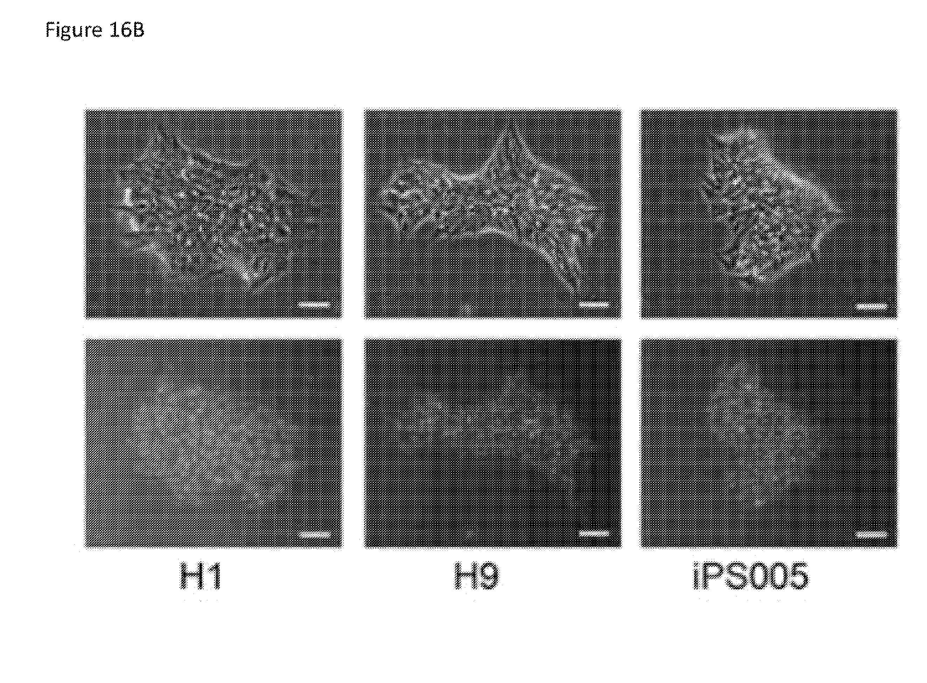

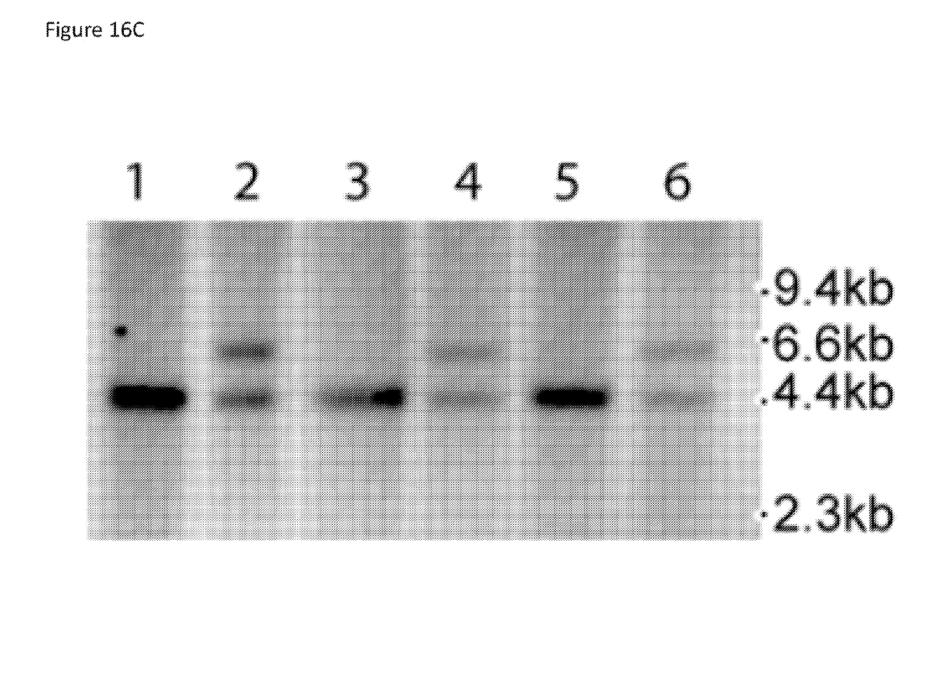

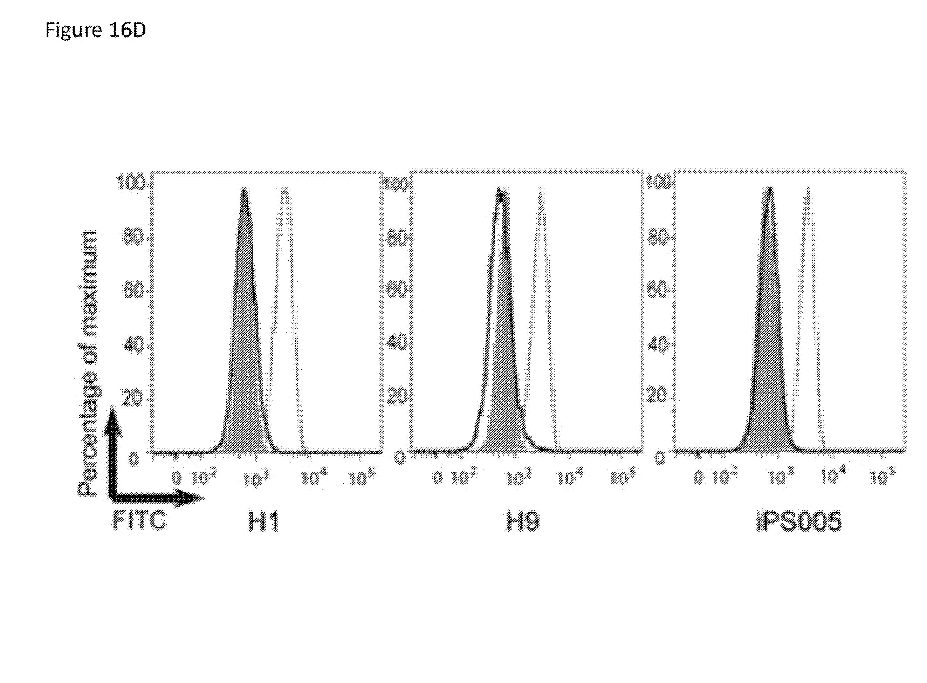

Figure 16. Gene targeting in hESCs using NmCas9. (*A*) Donor DNA and crRNA design. The mismatch in the first nt of crRNA is to satisfy the requirement of the U6 promoter for a G residue at the transcription start site. (*B*) Phase-contrast (top) and fluorescent (bottom) images of targeted clones from HI, H9 and iPS005 line. Scale bar: 50 µM. (*C*) Southern blot analysis of targeted clones of HI, H9 and iPS005 line. Genomic DNA was digested with BamHI. The Southern probe is located outside of donor DNA (see Section A). The wildtype clone should give one band of 4.2 kb and targeted heterozygous clone should give one additional band of 5.6 kb. (*D*) Targeted clones (see Section B) were treated with 10 µM SB431542 and 10 ng/ml BMP4 to initiate differentiation. The EGFP signal was analyzed by FACS 3 days after differentiation to identify undifferentiated parental cells before targeting; targeted cells before differentiation; and targeted cells after differentiation.

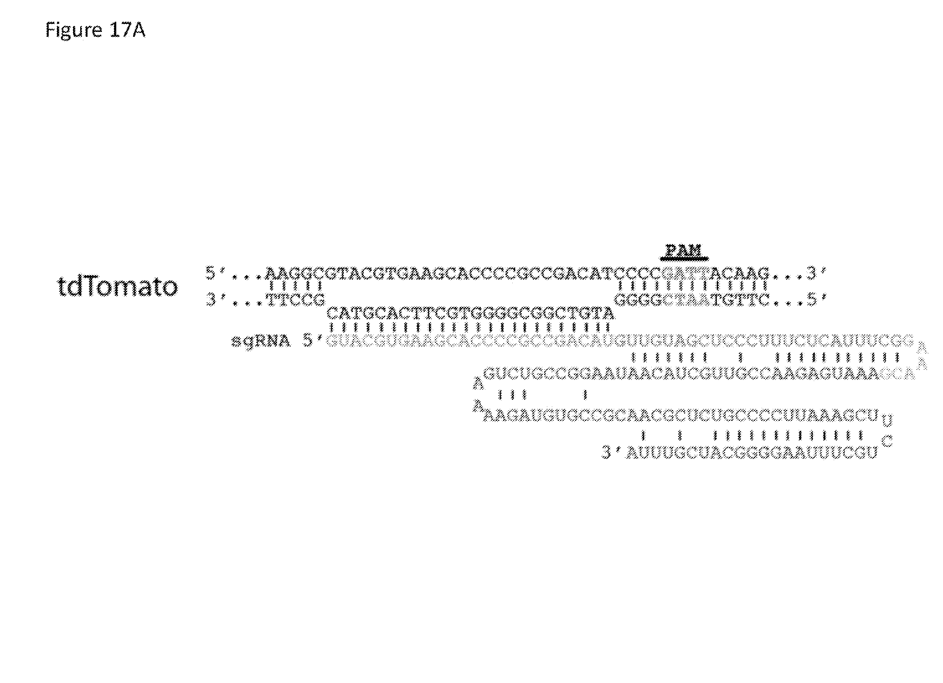

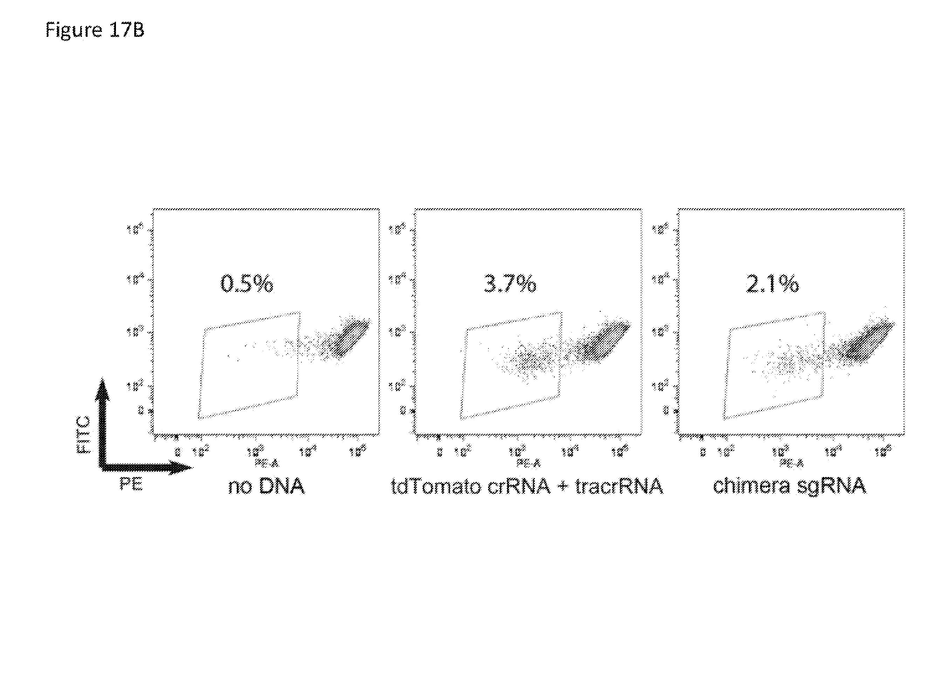

Figure 17. Single-guide RNA (sgRNA) is able to direct NmCas9-catalyzed genome editing. *A*. Design of an sgRNA that targets tdTomato including a spacer, crRNA repeat, linker and tracrRNA. *B*. FACS analysis of tdTomato reporter human ES cell lines after electroporation of the sgRNA construct and the nmCas9 expressing plasmid. The number in the plot indicates the percentage of tdTomato-fluorescence-negative cells five days after electroporation.

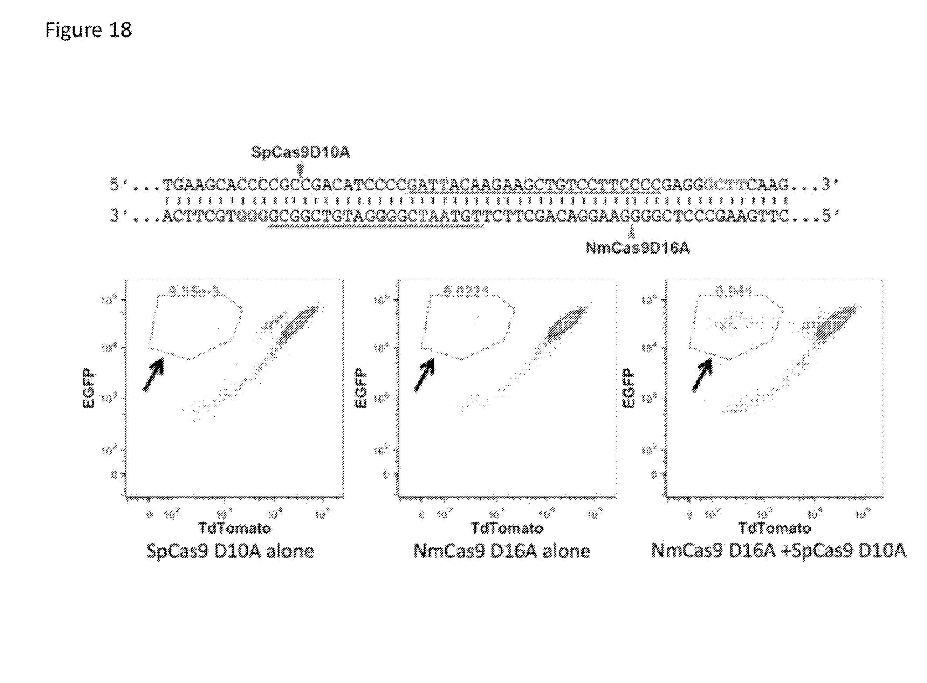

Figure 18. NmCas9 D16A functions as a nickase in human pluripotent stem cells. Sequences targeted by the sgRNAs are as indicated and cellular TdTomato fluorescence was analyzed by flow cytometer 5 days after transfection.

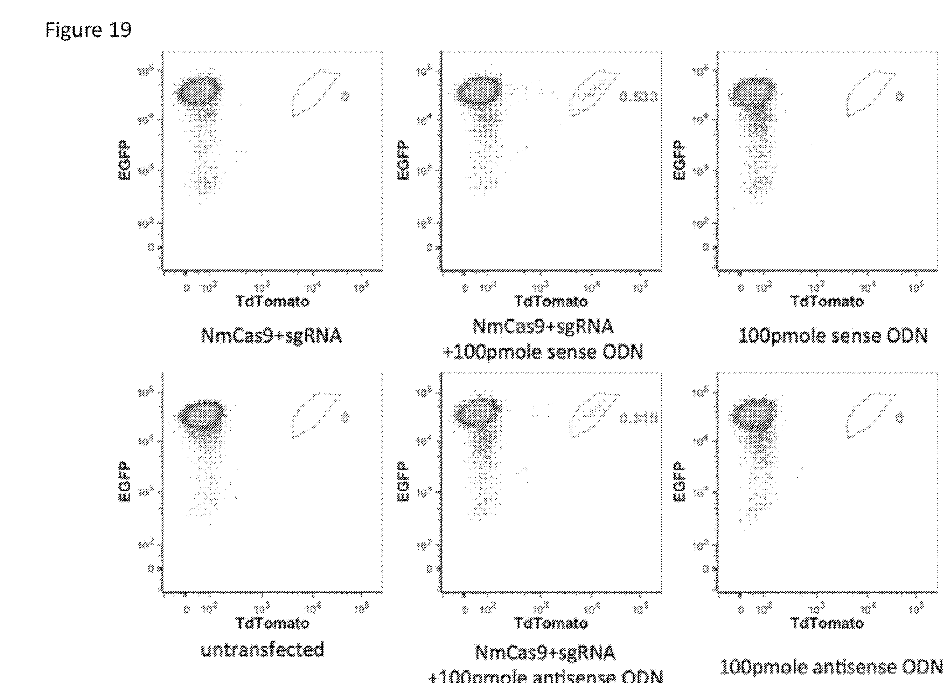

Figure 19. NmCas9 mediates homology-directed repair using either sense or antisense ssODN. The Human ES cell line used here, which expresses both EGFP and a single mutated (deficient, 9nt deleted) copy of Tomato fluorescent protein gene, was transfected with plasmids expressing NmCas9 and gRNA targeting the mutated region of tomato gene with or without 100pmole of 160nt ssODN as repair template. Cellular fluorescence was analyzed by flow cytometry 5 days after transfection..

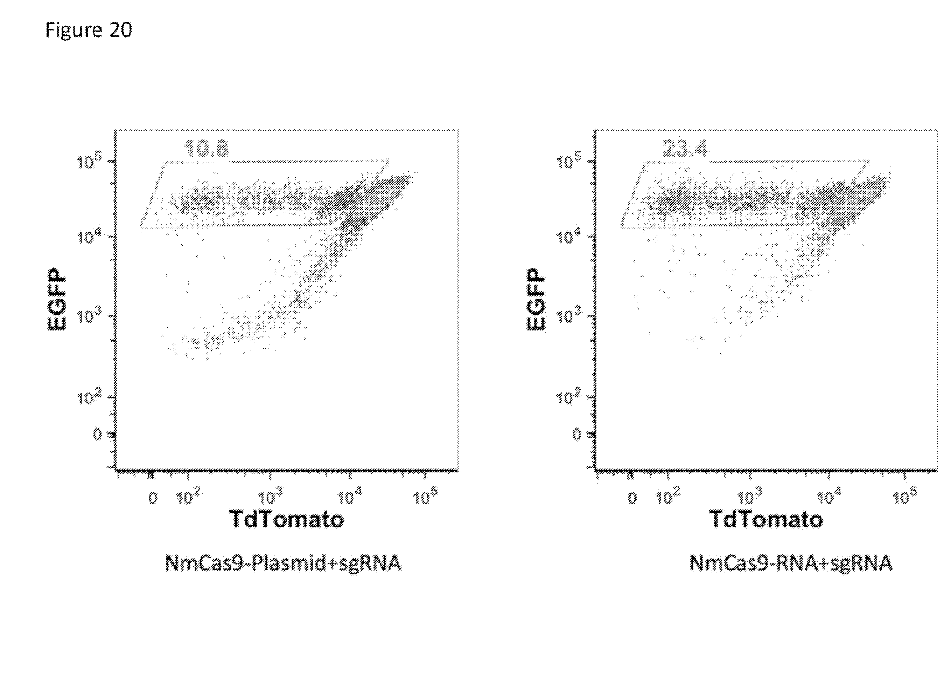

Figure 20. NmCas9 can be delivered in the form of mRNA instead of plasmid DNA. Human ES cells expressing both TdTomato and EGFP were transfected with NmCas9 mRNA or an NmCas9 expressing plasmid, together with a plasmid expressing sgRNA that targets tomato gene. Cellular fluorescence was analyzed by flow cytometry 5 days after transfection.

### DESCRIPTION

The present invention is described herein using several definitions, as set forth below and throughout the application.

Unless otherwise specified or indicated by context, the terms "a", "an", and "the" mean "one or more." For example, "a protein" or "an RNA" should be interpreted to mean "one or more proteins" or "one or more RNAs," respectively.

As used herein, "about," "approximately," "substantially," and "significantly" will be understood by persons of ordinary skill in the art and will vary to some extent on the context in which they are used. If there are uses of these terms which are not clear to persons of ordinary skill in the art given the context in which they are used, "about" and "approximately" will mean plus or minus ≤10% of the particular term and "substantially" and "significantly" will mean plus or minus >10% of the particular term.

As used herein, the terms "include" and "including" have the same meaning as the terms "comprise" and "comprising" in that these latter terms are "open" transitional terms that do not limit claims only to the recited elements succeeding these transitional terms. The term "consisting of," while encompassed by the term "comprising," should be interpreted as a "closed" transitional term that limits claims only to the recited elements succeeding this transitional term. The term "consisting essentially of," while encompassed by the term "comprising," should be interpreted as a "partially closed" transitional term which permits additional elements succeeding this transitional term, but only if those additional elements do not materially affect the basic and novel characteristics of the claim.

Disclosed are methods that utilize and kits and compositions that comprise components for RNA-directed DNA cleavage and gene editing. The methods utilize a Cas9 protein, or a variant protein thereof, and RNA that hybridizes to a target DNA sequence. The Cas9 protein and RNA bind and form a complex with the target DNA sequence. The Cas9 protein has nuclease activity (*e.g*., DNAse activity and/or RNase activity) and may cleave one (*i.e.,* nick) or both strands of the target DNA sequence. The term "nick" will be understood as an interruption in the covalent continuity of one strand of a double-stranded nucleic acid molecule. The term "nick' can also describe an enzymatic activity that results in the production of a nick in a nucleic acid molecule. The disclosed methods may be utilized for RNA-directed DNA cleavage in vitro, RNA-directed genome editing in vivo, and RNA-directed genome binding by Cas9 proteins.

CRISPR/Cas is a recently discovered, adaptive, sequence-based immune system identified in bacteria and archaea. A "Type II" CRISPR/Cas system from *Streptococcus pyogenes* SF370 has been developed into a simple eukaryotic genome editing tool. This system requires only three components: Ca9 endonuclease, a trans-activating CRISPR RNA (tracrRNA), and the target-specifying crRNA. By fusing the crRNA and tracrRNA into a single transcript referred to as an sgRNA, the machinery can be further streamlined into a two-component system. The target DNA sequence that base-pairs with the crRNA is referred to as the "protospacer." The two nuclease domains (RuvC and HNH) of Cas9 each cleave one DNA target strand and thus induce a DSB. Cleavage by Cas9 also depends on the presence of a short motif called a protospacer adjacent motif (PAM) that flanks the target region recognized by crRNA base pairing.

The present inventors have demonstrated that the *Neisseria meningitidis* (Nm) Cas9/crRNA/tracrRNA system can work efficiently for genome editing in human embryonic stem cells (hESCs), leaving behind small insertions and deletions. They have also shown that the NmCas9-induced DSB can serve as a site of transgene insertion. They have mapped the NmCas9 cleavage site to the third and fourth base pairs of the protospacer, at the end closest to the PAM. Importantly, the Cas9/crRNA/tracrRNA system uses the same Cas9 protein and tracrRNA for every targeting event. Only one component - the crRNA - needs to be customized for each individual target, which makes the system very user-friendly.

In addition to these *in vivo* advances, the inventors have shown that recombinant NmCas9 can be expressed in *E. coli* cells and that it can catalyze crRNA-directed DNA cleavage *in vitro.* This could enable enhanced recombinant DNA capabilities.

The inventors have also demonstrated that the NmCas9 system, in its native bacterial context, has a novel feature: It can function with long, unprocessed crRNA precursors. In bacterial cells, Type II CRISPR/Cas systems generate pre-crRNAs that are cleaved by a protein called RNase III. In *N. meningitidis,* deletion of the rnc gene that encodes RNase III has no deleterious effect on the CRISPR pathway, unlike all other Type II systems examined to date. *In vitro* experiments have confirmed that unprocessed crRNAs can direct DNA cleavage by NmCas9. Together these indicate that NmCas9 can tolerate extensions on its cognate crRNAs without loss of function, which SpCas9 and StCas9 cannot, perhaps enabling expanded functionality by fusing the crRNAs to other useful RNAs such as RNA mimics (see Paige et al., Science, 29 July 2011) and Xist RNA or fragments thereof (see Plath et al., Annu Rev Genet. 2002; 36:233-78 Epub 2002 Jun 11).

The inventors have also demonstrated that the NmCas9 system has distinct PAM requirements versus Type II CRISPR/Cas systems from different bacteria. For example, for SpCas9 the PAM is 5'-NGG3', while for NmCas9, the PAM is 5'-NNNNGATT3' (in both cases the dash represents the terminal nucleotide of the crRNA-paired sequence). Thus, the presently disclosed methods will open up potential target sites that are not cleavable with existing systems. Also, the specificity for genome editing may increase with a longer PAM.

The present inventors have identified a novel Cas9 protein. As used herein, the terms "protein" or "polypeptide" or "peptide" may be used interchangeably to refer to a polymer of amino acids. Typically, a "polypeptide" or "protein" is defined as a longer polymer of amino acids, of a length typically of greater than 50, 60, 70, 80, 90, or 100 amino acids. A "peptide" is defined as a short polymer of amino acids, of a length typically of 50, 40, 30, 20 or less amino acids.

A "protein" as contemplated herein typically comprises a polymer of naturally occurring amino acids (*e.g*., alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine). The proteins contemplated herein may be further modified in vitro or in vivo to include non-amino acid moieties. These modifications may include but are not limited to acylation (*e.g*., O-acylation (esters), N-acylation (amides), S-acylation (thioesters)), acetylation (*e.g*., the addition of an acetyl group, either at the N-terminus of the protein or at lysine residues), formylation lipoylation (*e.g*., attachment of a lipoate, a C8 functional group), myristoylation (*e.g.,* attachment of myristate, a C14 saturated acid), palmitoylation (*e.g.,* attachment of palmitate, a C16 saturated acid), alkylation (*e.g*., the addition of an alkyl group, such as an methyl at a lysine or arginine residue), isoprenylation or prenylation (*e.g.,* the addition of an isoprenoid group such as farnesol or geranylgeraniol), amidation at C-terminus, glycosylation (*e.g*., the addition of a glycosyl group to either asparagine, hydroxylysine, serine, or threonine, resulting in a glycoprotein). Distinct from glycation, which is regarded as a nonenzymatic attachment of sugars, polysialylation (*e.g*., the addition of polysialic acid), glypiation (*e.g*., glycosylphosphatidylinositol (GPI) anchor formation, hydroxylation, iodination (*e.g*., of thyroid hormones), and phosphorylation (*e.g.,* the addition of a phosphate group, usually to serine, tyrosine, threonine or histidine).

The Cas9 proteins disclosed herein may include "wild type" Cas9 protein and variants, mutants, and derivatives thereof. As used herein the term "wild type" is a term of the art understood by skilled persons and means the typical form of an organism, strain, gene or characteristic as it occurs in nature as distinguished from mutant or variant forms. As used herein, a "variant, "mutant," or "derivative" refers to a protein molecule having an amino acid sequence that differs from a reference protein or polypeptide molecule. A variant or mutant may have one or more insertions, deletions, or substitutions of an amino acid residue relative to a reference molecule. A variant or mutant may include a fragment of a reference molecule. For example, a Cas9 mutant or variant molecule may one or more insertions, deletions, or substitution of at least one amino acid residue relative to the Cas9 full-length polypeptide. The sequence of the full-length Cas9 protein from *Neisseria meningitidis* is presented as SEQ ID NO:1 and may be used as a reference in this regard.

Regarding proteins, a "deletion" refers to a change in the amino acid sequence that results in the absence of one or more amino acid residues. A deletion may remove at least 1, 2, 3, 4, 5, 10, 20, 50, 100, 200, or more amino acids residues. A deletion may include an internal deletion and/or a terminal deletion (*e.g.,* an N-terminal truncation, a C-terminal truncation or both of a reference polypeptide).

Regarding proteins, "fragment" is a portion of an amino acid sequence which is identical in sequence to but shorter in length than a reference sequence. A fragment may comprise up to the entire length of the reference sequence, minus at least one amino acid residue. For example, a fragment may comprise from 5 to 1000 contiguous amino acid residues of a reference polypeptide, respectively. In some embodiments, a fragment may comprise at least 5, 10, 15, 20, 25, 30, 40, 50, 60, 70, 80, 90, 100, 150, 250, or 500 contiguous amino acid residues of a reference polypeptide. Fragments may be preferentially selected from certain regions of a molecule. The term "at least a fragment" encompasses the full length polypeptide. A fragment of a Cas9 protein may comprise or consist essentially of a contiguous portion of an amino acid sequence of the full-length Cas9 protein (SEQ ID NO:1). A fragment may include an N-terminal truncation, a C-terminal truncation, or both truncations relative to the full-length Cas9 protein.

Regarding proteins, the words "insertion" and "addition" refer to changes in an amino acid sequence resulting in the addition of one or more amino acid residues. An insertion or addition may refer to 1, 2, 3, 4, 5, 10, 20, 30, 40, 50, 60, 70, 80, 90, 100, 150, 200, or more amino acid residues. A variant of a Cas9 protein may have N-terminal insertions, C-terminal insertions, internal insertions, or any combination of N-terminal insertions, C-terminal insertions, and internal insertions.

Regarding proteins, the phrases "percent identity" and "% identity," refer to the percentage of residue matches between at least two amino acid sequences aligned using a standardized algorithm. Methods of amino acid sequence alignment are well-known. Some alignment methods take into account conservative amino acid substitutions. Such conservative substitutions, explained in more detail below, generally preserve the charge and hydrophobicity at the site of substitution, thus preserving the structure (and therefore function) of the polypeptide. Percent identity for amino acid sequences may be determined as understood in the art. (*See, e.g.,* U.S. Patent No. 7,396,664). A suite of commonly used and freely available sequence comparison algorithms is provided by the National Center for Biotechnology Information (NCBI) Basic Local Alignment Search Tool (BLAST), which is available from several sources, including the NCBI, Bethesda, Md., at its website. The BLAST software suite includes various sequence analysis programs including "blastp," that is used to align a known amino acid sequence with other amino acids sequences from a variety of databases. As described herein, variants, mutants, or fragments (*e.g*., a Cas9 protein variant, mutant, or fragment thereof) may have 99%, 98%, 97%, 96%, 95%, 94%, 93%, 92%, 91%, 90%, 80%, 70%, 60%, or 50% amino acid sequence identity relative to a reference molecule (*e.g*., relative to the Cas9 full-length polypeptide (SEQ ID:1)).

Regarding proteins, percent identity may be measured over the length of an entire defined polypeptide sequence, for example, as defined by a particular SEQ ID number, or may be measured over a shorter length, for example, over the length of a fragment taken from a larger, defined polypeptide sequence, for instance, a fragment of at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured.

Regarding proteins, the amino acid sequences of variants, mutants, or derivatives as contemplated herein may include conservative amino acid substitutions relative to a reference amino acid sequence. For example, a variant, mutant, or derivative protein may include conservative amino acid substitutions relative to a reference molecule. "Conservative amino acid substitutions" are those substitutions that are a substitution of an amino acid for a different amino acid where the substitution is predicted to interfere least with the properties of the reference polypeptide. In other words, conservative amino acid substitutions substantially conserve the structure and the function of the reference polypeptide. The following table provides a list of exemplary conservative amino acid substitutions which are contemplated herein:

| Original Residue | Conservative Substitution |
|---|---|
| Ala | Gly, Ser |
| Arg | His, Lys |
| Asn | Asp, Gln, His |
| Asp | Asn, Glu |
| Cys | Ala, Ser |
| Gln | Asn, Glu, His |
| Gln | Asp, Gln, His |
| Gly | Ala |
| His | Asn, Arg, Gln, Glu |
| Ile | Leu, Val |
| Leu | Ile, Val |
| Lys | Arg, Gln, Glu |
| Met | Leu, Ile |
| Phe | His, Met, Leu, Trp, Tyr |
| Ser | Cys, Thr |
| Thr | Ser, Val |
| Trp | Phe, Tyr |
| Tyr | His, Phe, Trp |
| Val | Ile, Leu, Thr |

Conservative amino acid substitutions generally maintain (a) the structure of the polypeptide backbone in the area of the substitution, for example, as a beta sheet or alpha helical conformation, (b) the charge or hydrophobicity of the molecule at the site of the substitution, and/or (c) the bulk of the side chain.

The disclosed proteins, mutants, variants, or described herein may have one or more functional or biological activities exhibited by a reference polypeptide (*e.g*., one or more functional or biological activities exhibited by wild-type Cas9 protein). For example, the disclosed Cas9 proteins, mutants, variants, or derivatives thereof may have one or more biological activities that include: binding to a single-stranded RNA, binding to a double-stranded RNA, binding to a target polynucleotide sequence, nicking a single strand of the target DNA sequence, and/or cleaving both strands of the target DNA sequence.

The disclosed Cas9 proteins may be substantially isolated or purified. The term "substantially isolated or purified" refers to amino acid sequences that are removed from their natural environment, and are at least 60% free, preferably at least 75% free, and more preferably at least 90% free, even more preferably at least 95% free from other components with which they are naturally associated.

Also disclosed herein are polynucleotides, for example polynucleotide sequences that encode Cas9 proteins (*e.g*., DNA that encodes a polypeptide having the amino acid sequence of SEQ ID NO:1 or a polypeptide variant having an amino acid sequence with at least about 50%, 60%, 70%, 80%, 90%, 95%, 96%, 97%, 98%, or 99% sequence identity to SEQ ID NO:1; DNA comprising the polynucleotide sequence of SEQ ID NO:2; DNA comprising the polynucleotide sequence of SEQ ID NO:3; or Cas9 mRNA). Other polynucleotides contemplate herein are RNAs that direct Cas9-mediated binding, nicking, and/or cleaving of a target DNA sequence (*e.g*., tracrRNA, crRNA, sgRNA) and DNA that encodes such RNAs. The terms "polynucleotide," "polynucleotide sequence," "nucleic acid" and "nucleic acid sequence" refer to a nucleotide, oligonucleotide, polynucleotide (which terms may be used interchangeably), or any fragment thereof. These phrases also refer to DNA or RNA of genomic, natural, or synthetic origin (which may be single-stranded or double-stranded and may represent the sense or the antisense strand).

Regarding polynucleotide sequences, the terms "percent identity" and "% identity" refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity for a nucleic acid sequence may be determined as understood in the art. (*See, e.g.,* U.S. Patent No. 7,396,664). A suite of commonly used and freely available sequence comparison algorithms is provided by the National Center for Biotechnology Information (NCBI) Basic Local Alignment Search Tool (BLAST), which is available from several sources, including the NCBI, Bethesda, Md., at its website. The BLAST software suite includes various sequence analysis programs including "blastn," that is used to align a known polynucleotide sequence with other polynucleotide sequences from a variety of databases. Also available is a tool called "BLAST 2 Sequences" that is used for direct pairwise comparison of two nucleotide sequences. "BLAST 2 Sequences" can be accessed and used interactively at the NCBI website. The "BLAST 2 Sequences" tool can be used for both blastn and blastp (discussed above).

Regarding polynucleotide sequences, percent identity may be measured over the length of an entire defined polynucleotide sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length supported by the sequences shown herein, in the tables, figures, or Sequence Listing, may be used to describe a length over which percentage identity may be measured.

Regarding polynucleotide sequences, "variant," "mutant," or "derivative" may be defined as a nucleic acid sequence having at least 50% sequence identity to the particular nucleic acid sequence over a certain length of one of the nucleic acid sequences using blastn with the "BLAST 2 Sequences" tool available at the National Center for Biotechnology Information's website. (*See* Tatiana A. Tatusova, Thomas L. Madden (1999), "Blast 2 sequences - a new tool for comparing protein and nucleotide sequences", FEMS Microbiol Lett. 174:247-250). Such a pair of nucleic acids may show, for example, at least 60%, at least 70%, at least 80%, at least 85%, at least 90%, at least 91%, at least 92%, at least 93%, at least 94%, at least 95%, at least 96%, at least 97%, at least 98%, or at least 99% or greater sequence identity over a certain defined length.

Nucleic acid sequences that do not show a high degree of identity may nevertheless encode similar amino acid sequences due to the degeneracy of the genetic code where multiple codons may encode for a single amino acid. It is understood that changes in a nucleic acid sequence can be made using this degeneracy to produce multiple nucleic acid sequences that all encode substantially the same protein. For example, polynucleotide sequences as contemplated herein may encode a Cas9 protein and may be codon-optimized for expression in a particular host. In the art, codon usage frequency tables have been prepared for a number of host organisms including humans, mouse, rat, pig, *E. coli,* plants, and other host cells.

A "recombinant nucleic acid" is a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, e.g., by genetic engineering techniques known in the art. The term recombinant includes nucleic acids that have been altered solely by addition, substitution, or deletion of a portion of the nucleic acid. Frequently, a recombinant nucleic acid may include a nucleic acid sequence operably linked to a promoter sequence. Such a recombinant nucleic acid may be part of a vector that is used, for example, to transform a cell.

"Substantially isolated or purified" nucleic acid or amino acid sequences are contemplated herein. The term "substantially isolated or purified" refers to nucleic acid or amino acid sequences that are removed from their natural environment, and are at least 60% free, preferably at least 75% free, and more preferably at least 90% free, even more preferably at least 95% free from other components with which they are naturally associated.

"Transformation" or "transfected" describes a process by which exogenous nucleic acid (*e.g.,* DNA or RNA) is introduced into a recipient cell. Transformation or transfection may occur under natural or artificial conditions according to various methods well known in the art, and may rely on any known method for the insertion of foreign nucleic acid sequences into a prokaryotic or eukaryotic host cell. The method for transformation or transfection is selected based on the type of host cell being transformed and may include, but is not limited to, bacteriophage or viral infection, electroporation, heat shock, lipofection, and particle bombardment.

Methods of non-viral delivery of nucleic acids include lipofection, nucleofection, microinjection, electroporation, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam.TM. and Lipofectin.TM.). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration). The term "transformed cells" or "transfected cells" includes stably transformed or transfected cells in which the inserted DNA is capable of replication either as an autonomously replicating plasmid or as part of the host chromosome, as well as transiently transformed or transfected cells which express the inserted DNA or RNA for limited periods of time.

The polynucleotide sequences contemplated herein may be present in expression vectors. For example, the vectors may comprise: (a) a polynucleotide encoding an ORF of a Cas9 protein; (b) a polynucleotide that expresses an RNA that directs Cas9-mediated binding, nicking, and/or cleaving of a target DNA sequence; and both (a) and (b). The polynucleotide present in the vector may be operably linked to a prokaryotic or eukaryotic promoter. "Operably linked" refers to the situation in which a first nucleic acid sequence is placed in a functional relationship with a second nucleic acid sequence. For instance, a promoter is operably linked to a coding sequence if the promoter affects the transcription or expression of the coding sequence. Operably linked DNA sequences may be in close proximity or contiguous and, where necessary to join two protein coding regions, in the same reading frame. Vectors contemplated herein may comprise a heterologous promoter (*e.g*., a eukaryotic or prokaryotic promoter) operably linked to a polynucleotide that encodes a Cas9 protein. A "heterologous promoter" refers to a promoter that is not the native or endogenous promoter for the protein or RNA that is being expressed. For example, a heterologous promoter for a Cas9 protein of *Neisseria menigitidis* may include a eukaryotic promoter or a prokaryotic promoter that is not the native, endogenous promoter for the Cas9 protein of *Neisseria menigitidis.*

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The term "vector" refers to some means by which nucleic acid (e.g., DNA) can be introduced into a host organism or host tissue. There are various types of vectors including plasmid vector, bacteriophage vectors, cosmid vectors, bacterial vectors, and viral vectors. As used herein, a "vector" may refers to a recombinant nucleic acid that has been engineered to express a heterologous polypeptide (*e.g.,* a Cas9 protein). The recombinant nucleic acid typically includes *cis*-acting elements for expression of the heterologous polypeptide.

Any of the conventional vectors used for expression in eukaryotic cells may be used for directly introducing DNA into a subject. Expression vectors containing regulatory elements from eukaryotic viruses may be used in eukaryotic expression vectors (*e.g.,* vectors containing SV40, CMV, or retroviral promoters or enhancers). Exemplary vectors include those that express proteins under the direction of such promoters as the SV40 early promoter, SV40 later promoter, metallothionein promoter, human cytomegalovirus promoter, murine mammary tumor virus promoter, and Rous sarcoma virus promoter. Expression vectors as contemplated herein may include prokaryotic control sequences that modulate expression of a heterologous protein (*e.g*. Cas9 protein. Prokaryotic expression control sequences may include constitutive or inducible promoters (*e.g.,* T3, T7, Lac, trp, or phoA), ribosome binding sites, or transcription terminators.

The vectors contemplated herein may be introduced and propagated in a prokaryote, which may be used to amplify copies of a vector to be introduced into a eukaryotic cell or as an intermediate vector in the production of a vector to be introduced into a eukaryotic cell (e.g. amplifying a plasmid as part of a viral vector packaging system). A prokaryote may be used to amplify copies of a vector and express one or more nucleic acids, such as to provide a source of one or more proteins for delivery to a host cell or host organism. Expression of proteins in prokaryotes may be performed using Escherichia coli with vectors containing constitutive or inducible promoters directing the expression of either a Cas9 protein or a fusion protein comprising a Cas9 protein or a fragment thereof. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; (iii) to aid in the purification of the recombinant protein by acting as a ligand in affinity purification (*e.g*., a His tag); (iv) to tag the recombinant protein for identification (e.g., such as Green fluorescence protein (GFP) or an antigen (*e.g*., an HA tag such as SEQ ID NOs:6 and 7) that can be recognized by a labelled antibody); (v) to promote localization of the recombinant protein to a specific area of the cell (*e.g.,* where the Cas9 protein is fused (*e.g.,* at its N-terminus or C-terminus) to a nuclear localization signal (NLS) which may include the NLS of SV40 (*e.g.,* SEQ ID NOs:4 and 5, which is a monopartite NLS), nucleoplasmin (which comprises a bipartite signal of two clusters of basic amino acids separated by a spacer of about 10 amino acids), C-myc, M9 domain of hnRNP A1, or a synthetic NLS (*e.g.,* SEQ ID NOs:8 and 9)). The importance of neutral and acidic amino acids in NLS have been studied. (*See* Makkerh et al. (1996) Curr Biol 6(8):1025-1027). Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

The presently disclosed methods may include delivering one or more polynucleotides, such as or one or more vectors as described herein, one or more transcripts thereof, and/or one or proteins transcribed therefrom, to a host cell. Further contemplated are host cells produced by such methods, and organisms (such as animals, plants, or fungi) comprising or produced from such cells. In some embodiments, a CRISPR enzyme (*e.g*., Cas9 protein) in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (*e.g.* a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell.

In the methods contemplated herein, a host cell may be transiently or non-transiently transfected (*i.e.,* stably transfected) with one or more vectors described herein. In some embodiments, a cell is transfected as it naturally occurs in a subject (*i.e.,* in situ). In some embodiments, a cell that is transfected is taken from a subject (*i.e.,* explanted). In some embodiments, the cell is derived from cells taken from a subject, such as a cell line. Suitable cells may include stem cells (*e.g*., embryonic stem cells and pluripotent stem cells). A cell transfected with one or more vectors described herein may be used to establish a new cell line comprising one or more vector-derived sequences. In the methods contemplated herein, a cell may be transiently transfected with the components of a CRISPR system as described herein (such as by transient transfection of one or more vectors, or transfection with RNA), and modified through the activity of a CRISPR complex, in order to establish a new cell line comprising cells containing the modification but lacking any other exogenous sequence.

The methods, vectors, and systems described herein may be used to produce a non-human transgenic animal or a transgenic plant or algae. Transgenic animals may include a mammal, such as a mouse, rat, or rabbit. Methods for producing transgenic plants and animals are known in the art, and generally begin with a method of cell transfection with one or more vectors as contemplated herein.

### EXAMPLES

The following Examples are illustrative and are not intended to limit the scope of the claimed subject matter.

### Example 1

Reference is made to Zhang et al., "Processing-Independent CRISPR RNAs Limit Natural Transformation in Neisseria meningitidis" Molecular Cell 50, 488-503, May 23, 2013, the contents of which are incorporated herein by reference.

### Abstract

CRISPR interference confers adaptive, sequence-based immunity against viruses and plasmids and is specified by CRISPR RNAs (crRNAs) that are transcribed and processed from spacer-repeat units. Pre-crRNA processing is essential for CRISPR interference in all systems studied thus far. Here, our studies of crRNA biogenesis and CRISPR interference in naturally competent *Neisseria* spp. reveal a unique crRNA maturation pathway in which crRNAs are transcribed from promoters that are embedded within each repeat, yielding crRNA 5' ends formed by transcription and not by processing. Although crRNA 3' end formation involves RNase III and *trans-*encoded tracrRNA, as in other Type II CRISPR systems, this processing is dispensable for interference. The meningococcal pathway is the most streamlined CRISPR/*cas* system characterized to date. Endogenous CRISPR spacers limit natural transformation, which is the primary source of genetic variation that contributes to immune evasion, antibiotic resistance, and virulence in the human pathogen *N. meningitidis.* Highlights of these new CRISPRS include the following: unlike previously described CRISPRs, each *Neisseria* repeat carries its own promoter; pre-crRNA processing is dispensable for CRISPR interference in *Neisseria* spp; CRISPR interference blocks natural transformation in the pathogen *N. meningitides*; and *Neisseria* CRISPR/Cas systems are the most streamlined observed to date

### Introduction

Clustered, regularly interspaced, short palindromic repeat (CRISPR) loci confer sequence-based, adaptive immunity against virus infection and plasmid conjugation in bacteria and archaea (Haft et al., 2005; Makarova et al., 2006; Barrangou et al., 2007; Terns and Terns, 2011; Wiedenheft et al., 2012). CRISPRs consist of short repeats separated by similarly sized, non-repetitive sequences called spacers, which are derived from previously encountered invasive sequences such as viral genomes or plasmids (Bolotin et al., 2005; Mojica et al., 2005; Pourcel et al., 2005). CRISPR loci are transcribed as long CRISPR RNA (crRNA) precursors that are processed into small crRNAs (Brouns et al., 2008; Hale et al., 2008). Pre-crRNA transcription is generally driven by promoters within "leader" sequences outside of the CRISPR array. The crRNAs assemble with CRISPR-associated (Cas) proteins into complexes that cleave complementary "protospacer" sequences within invasive nucleic acids, a phenomenon known as CRISPR interference (Karginov and Hannon, 2010; Marraffini and Sontheimer, 2010; Terns and Terns, 2011; Wiedenheft et al., 2012). The sequence information in crRNAs is used to guide Cas complexes to their targets on viruses and plasmids, leading to their destruction (Barrangou et al., 2007; Brouns et al., 2008; Marraffini and Sontheimer, 2008; Hale et al., 2009; Garneau et al., 2010; Westra et al., 2012). Most CRISPR/Cas systems cleave incoming DNAs (Marraffini and Sontheimer, 2008; Garneau et al., 2010; Westra et al., 2012), though RNA-cleaving systems have also been identified (Hale et al., 2009, 2012; Zhang et al., 2012).

CRISPR/Cas systems have been classified into types I, II and III based primarily on their *cas* gene complement (Makarova et al., 2011a). Common to all of these three types is that the CRISPR array is transcribed as a multimeric pre-crRNA that is processed into crRNAs that each contain an individual spacer flanked on one or both sides by partial repeat sequences (Bhaya et al., 2011). However, the molecular events underlying processing dramatically differ. Whereas in Type I and III systems the processing enzymes are encoded within the CRISPR/*cas* locus, Type II systems use the host enzyme RNase III (encoded by the *rnc* gene) and a noncoding RNA called tracrRNA (Deltcheva et al., 2011). In *Streptococcus pyogenes* SF370, an *rnc* mutant abolishes the function of a Type II CRISPR/*cas* locus, indicating that pre-crRNA processing is essential (Deltcheva et al., 2011).

The importance of the Type II CRISPR/Cas pathway has been dramatically enhanced by its development into a system for RNA-guided DNA cleavage *in vitro* (Jinek et al., 2012) and genome editing *in vivo* (Jinek et al., 2013; Cho et al., 2013; Cong et al., 2013; DiCarlo et al., 2013; Hwang et al., 2013; Jiang et al., 2013; Mali et al., 2013). Our ability to exploit this new technology further will depend on a deeper understanding of the underlying molecular mechanisms, and will be increased by the characterization of systems that are as simplified and streamlined as possible. Type II CRISPR/Cas loci, which are found in bacteria but not archaea, usually contain four *cas* genes: *cas1, cas2, cas9,* and either *csn2* (subtype II-A) or *cas4* (subtype II-B) (Makarova et al., 2011b). Cas9 is the effector protein for the interference function of existing spacer sequences in the CRISPR array (Sapranauskas et al., 2011; Gasiunas et al., 2012; Jinek et al., 2012), whereas the other proteins are thought to be involved in the acquisition of new CRISPR spacers. The tracrRNA is essential for crRNA-directed DNA recognition and Cas9-catalyzed DNA cleavage *in vitro,* even with crRNAs that bypass processing (Jinek et al., 2012). DNA targeting in both Type I and Type II systems requires not only crRNA/target complementarity but also a protospacer adjacent motif (PAM), which is a short (2-5 nt), conserved sequence just outside of the protospacer (Deveau et al., 2008; Horvath et al., 2008; Mojica et al., 2009; Semenova et al., 2011; Sashital et al., 2012).

Although CRISPR interference was originally defined as a phage defense pathway, CRISPR/Cas systems are now understood to play a broader role in limiting horizontal gene transfer (HGT) (Marraffini and Sontheimer, 2008). The three primary routes of HGT are transformation, conjugation, and phage transduction, and the latter two are well established as being subject to interference by naturally occurring spacers. Artificial means of transformation (e.g. electroporation) can also be blocked by CRISPR interference (Marraffini and Sontheimer, 2008; Deltcheva et al., 2011; Sapranauskas et al., 2011; Semenova et al., 2011), though natural transformation uses a very different DNA uptake process (Chen et al., 2005). An engineered spacer can prevent natural transformation specified by an *S. pyogenes* CRISPR/*cas* locus transplanted into *Streptococcus pneumonia* (Bikard et al., 2012). However, although this artificial system suggests that natural CRISPR/Cas contexts may do likewise, the fundamental question of whether native CRISPR/Cas systems play a role in natural transformation remains to be addressed.

Strains from the genus *Neisseria* serve as paradigms for natural transformation, as they are competent during all phases of their life cycle and use this process for frequent genetic exchange (Hamilton and Dillard, 2006). Although functional CRISPR/*cas* systems have not been identified in *Neisseria gonorrhoeae,* some strains of *Neisseria lactamica* and *Neisseria meningitidis* carry apparent Type II CRISPR/*cas* loci (Grissa et al., 2007). Meningococci are obligate human commensals that can invade the bloodstream and cerebrospinal fluid (Bratcher et al., 2012), and meningococcal disease is responsible for tens of thousands of deaths per year (Stephens et al., 2007).

Here we characterize the CRISPR pathway in neisseriae and find that it exhibits several unique features, most notably a streamlined functional architecture that includes a previously unknown, processing-independent mode of crRNA biogenesis. Furthermore, naturally occurring spacers match sequences from other *Neisseria* genomes, including a prophage-like meningococcal disease-associated (MDA) island that correlates with invasiveness and pathogenicity (Bille et al., 2005, 2008). We find that a native meningococcal CRISPR/*cas* locus prevents natural transformation of spacer-matched sequences, suggesting that it can limit the horizontal spread of virulence genes.

### Results

dRNA-seq reveals that each repeat in the Neisseria CRISPR carries its own promoter. We analysed all 19 sequenced *Neisseria* genomes available in the NCBI database (fifteen from *N. meningitidis,* three from *N. gonorrhoeae,* and one from *N. lactamica*) using CRISPRFinder (Grissa et al., 2007) or CRISPRi (Rousseau et al., 2009). We identified seven putative Type II CRISPR/cas loci: six in *N. meningitidis* strains, and one in *N. lactamica* 020-06. All were highly similar, and unlike other Type II loci characterized previously (Barrangou et al., 2007; Deltcheva et al., 2011; Magadán et al., 2012), included a set of only three predicted protein-coding genes (*cas9, cas1* and *cas2*) but neither *csn2* nor *cas4.* To examine the expression status of a representative locus, we performed our recently developed dRNA-seq approach (Sharma et al., 2010) on *N. lactamica* 020-06. We prepared two cDNA libraries from total RNA using a strategy that allows us to distinguish between transcripts with either primary or processed 5' ends: one library is generated from untreated RNA, whereas the other is treated with terminator exonuclease (TEX), which specifically degrades RNAs with 5'-monophosphate ends (including the abundant rRNAs and tRNAs) that are formed by processing. Primary transcripts with 5'-triphosphate ends survive TEX treatment, resulting in their relative enrichment in the TEX+ library.

Our global mapping of cDNA reads identified a tracrRNA and small crRNAs as highly abundant classes of transcripts (Figure 1A), which suggests a highly active CRISPR/Cas system. Reads obtained from the tracrRNA locus reveal the existence of two tracrRNA forms [107 nucleotides (nt) and 91 nt] (Figure 1B). The treatment with TEX eliminated the shorter tracrRNA reads, which indicates that they are products of processing as observed in a Type II-A CRISPR system (Deltcheva et al., 2011). These sequencing results were confirmed by Northern analyses (Figure 1C). In striking contrast, crRNAs were enriched rather than depleted in the TEX-treated libraries (Figure ID), suggesting that formation of many if not most crRNA 5' ends is processing-independent. TEX treatment did affect the relative amounts of individual crRNAs; for example, crRNAs from spacers 4, 6 and 9 exhibited very similar read counts in the untreated sample but varied over a 5-fold range in the TEX-treated sample. These results suggest that the crRNA pool contains some 5'-monophosphorylated crRNAs, the fraction of which varies from spacer to spacer. Northern blots confirmed the resistance of a population of mature, ∼48-nt crRNAs, as well as a subset of longer crRNA precursors, to TEX digestion (Figure IE). This crRNA profile indicates a different mode of crRNA biogenesis from that reported for other Type II systems (Deltcheva et al., 2011). Intriguingly, we noted that the terminal nine nucleotides of each CRISPR repeat exhibit sequence hallmarks of an extended -10 box promoter element (Hook-Barnard and Hinton, 2007) and that the TEX-resistant crRNA 5' ends map 9-10 nt downstream from the center of each such element (Figure 1D).

crRNA biogenesis in *Neisseria lactamica* depends on single promoter elements in each CRISPR repeat. The dRNA-seq results and -10 box similarity suggest that in *N. lactamica* 020-06, each CRISPR repeat carries its own minimal promoter, and that pre-crRNA transcription initiates independently within each spacer. As an initial test of this hypothesis, we designed a series of transcriptional green fluorescent protein (*gfp*) fusion constructs containing either single or multiple CRISPR repeats, introduced these constructs into *E. coli,* and analysed cellular GFP fluorescence. As shown in Figure 2A, the *gfp* fusion construct with a wild-type CRISPR repeat led to robust cellular fluorescence, whereas a two-nt substitution in the extended -10 promoter TG motif (Hook-Barnard and Hinton, 2007) reduced *gfp* expression to background levels. Constructs with *gfp* fused to three CRISPR repeat-spacer units increased the fluorescence signal almost two-fold, consistent with the possibility of increased transcription from the multiple repeats. We obtained similar results using repeat sequences from a Type II CRISPR/*cas* locus in *Campylobacter jejunii* NCTC11168 (Figure 2A); this locus, like that of *N. lactamica* 020-06, also contains only *cas9, cas1* and *cas2* and has CRISPR repeats that include an extended -10 box consensus. Thus, our *gfp* reporter assays prove that the putative promoter elements in each *N. lactamica* repeat are indeed active in bacterial cells, and are likely also present in some other Type II CRISPR/*cas* loci with similar, minimized *cas* gene content (Figure 2B and Table 1). The *Neisseria* and *Campylobacter* systems are also unusual in that the crRNAs and *cas* genes are transcribed in the opposite direction, and the CRISPR arrays lack recognizable "leader" sequences with external promoters. Based on these considerations as well as independent phylogenetic analyses (Koonin and Makarova, 2013; Chylinski et al., 2013), we now consider these variant Type II CRISPR/Cas loci as members of a distinct and newly defined subtype, Type II-C (Figure 2B).

To obtain additional proof that each *N. lactamica* CRISPR repeat carries its own promoter element, we used purified *E. coli* σ⁷⁰ RNA polymerase (RNAP) holoenzyme in *in vitro* transcription assays with linear DNA templates containing either a wild-type or a mutant repeat. A transcript of the expected length (168 nt) was obtained with the wild-type CRISPR repeat template (and with a control -10/-35 promoter construct), but not with the mutated repeat (Figures 2C and 8). This result demonstrated that the extended -10 motif in the *N. lactamica* CRISPR repeat was recognized even by the heterologous *E. coli* σ⁷⁰ RNAP holoenzyme. These data show that crRNAs in *N. lactamica* exhibit a unique mode of biogenesis involving transcription from extended -10 class promoters located within CRISPR repeats.

RNase III is involved in 3' end formation of *Neisseria* crRNAs. The observation that crRNA 5' ends correspond to sites of transcription initiation in *N. lactamica* suggests a reduced dependence on processing relative to other CRISPR systems. To determine whether this reduced dependence extends to crRNA 3' end formation as well or if 3' processing still occurs, and to extend our studies to other *Neisseria* strains, we deleted the *rnc* gene (which encodes RNase III) in *N. meningitidis* WUE2594, and then compared the tracrRNA and crRNA populations from this Δ*rnc* mutant with wild-type bacteria by northern analysis. As shown in Figure 2D, the Δ*rnc* strain exhibited a complete loss of the shorter (91 nt), TEX-sensitive tracrRNA, indicating that the 5' end of this RNA is generated by RNase III-dependent processing as observed previously in a Type II-A system (Deltcheva et al., 2011). We also observed dramatic differences in the crRNA population in the Δ*rnc* mutant: the 48-nt population is virtually abolished, and longer pre-crRNAs accumulate. This result strongly suggests that *Neisseria* spp. crRNA 3' end formation depends upon RNase III rather than direct transcription termination.

Repeat/spacer organization and potential targets of *Neisseria* Type II-C CRISPR loci. Having defined unique features of CRISPR/Cas systems in neisseriae, we turned our attention towards functional analyses, beginning with an examination of CRISPR organization and spacer content. Of the 103 spacers found in the seven CRISPR-positive genomes (Figure 3A and Table 2), one is 29 nt long while all others are 30 nt. All seven CRISPRs have the same 36-nt repeat consensus, with only a few repeats that deviate from this consensus (Figure 3A and Table 3). Intriguingly, the polarity of spacer conservation is opposite to that generally observed in other CRISPR loci. Conserved spacers that are shared among multiple strains in *Neisseria* spp. (color-coded in Figure 3A) are enriched at the upstream end of the array (relative to the direction of transcription) but are far less common at the downstream end. In contrast, other CRISPRs described thus far have the most recently derived and therefore least conserved spacers at the upstream end, i.e., proximal to the promoter (Makarova et al., 2011b). This observation suggests that new spacer acquisition in neisseriae, and perhaps in other Type II-C loci, occurs at the downstream end. In addition, deviations from the repeat consensus are most common at the upstream end in neisseriae (Table 3), whereas other CRISPRs most frequently exhibit the opposite tendency. Although technical limitations have thus far precluded direct tests of spacer acquisition in neisseriae, several considerations make it likely that these CRISPRs are competent for the adaptive component of the pathway. First, Type II-C loci are relatively common, with many more apparent examples among sequenced bacterial genomes than Type II-B loci (Chylinski et al., 2013). It is unlikely that type II-C systems would be so widespread if they were unable to provide adaptive protection to their hosts. Second, despite the conservation of the Type II-C loci in closely related *Neisseria* strains, there are many differences in spacer content (Figure 3A). This implies that these CRISPR loci are fluid, i.e. can adapt by adding and deleting spacers. Third, reports from other systems indicate that *cas1* and *cas2* can suffice for spacer acquisition (Yosef et al., 2012). It is therefore plausible that *cas1* and *cas2* likewise suffice for adaptation in Type II-C systems.

BLASTN searches with the 83 unique spacer sequences for similar sequences in the NCBI database allowed us to identify at least one potential target for 35 (∼42%) of them. For simplicity we required either a perfect match, or at most a single mismatch in the 10 protospacer nucleotides furthest from the PAM (i.e., well outside of the presumptive "seed" sequence that requires perfect complementarity for interference) (Sapranauskas et al., 2011; Semenova et al., 2011; Wiedenheft et al., 2011; Jinek et al., 2012). Figure 3B shows representative protospacer sequences that match CRISPR spacers from *N. meningitidis* strain 8013; representative protospacers that match spacers from all strains are shown in Figure 9. Protospacer alignments reveal an apparent PAM of 5'-NNNNGATT-3' (Figures 3B and 9). Of the 325 distinct candidate protospacers that match these 35 CRISPR spacers, all are in *Neisseria* sequences: 248 (76%) in *N. meningitidis* genomes, 69 (21%) in *N. gonorrhoeae* genomes, 6 (2%) in *N. lactamica* plasmids, and 1 each in *N. meningitidis* and *N. flavescens* plasmids. In some cases (shown in Figures 3B and 9), potential protospacers are present in the same genome as the targeting CRISPR spacer, suggesting the potential for autoimmunity (Stern et al., 2010). However, these protospacers all include significant deviations from the PAM consensus, perhaps explaining the apparent lack of self-targeting that is implied by the persistence of the matching protospacer. Intriguingly, 22 out of 35 CRISPR spacers (shown in Figures 3B and 9) with identifiable targets match at least one potential prophage sequence (Table 4), including the meningococcal disease-associated (MDA) island that is associated with invasiveness and pathogenicity in young adult patients (Bille et al., 2005, 2008).

CRISPR interference blocks natural transformation in *N. meningitides.* The preponderance of protospacers in *Neisseria* spp. genomes suggests that the CRISPR/*cas* loci could interfere with natural transformation. For our functional analyses addressing this possibility, we focused on *N. meningitidis* 8013, primarily because it exhibits the most robust transformation competence in our hands. For transformation assays we used the vector pGCC2, which contains an erythromycin resistance gene (*ermC*) and polylinker inserted into sequences from the gonococcal *lctP*/*aspC* locus (Figure 4A). Upon transformation into *N. meningitidis,* homologous recombination into the meningococcal *lctPlaspC* locus leads to *ermC* insertion and erythromycin-resistant (Erm^{R}) transformants. We selected potential natural target sequences, including ten or more nucleotides on both sides of the protospacer, for seven of the spacers of *N. meningitidis* 8013, namely spacers 8, 9, 16, 17, 18, 23, and 25 (Figures 3B and 10). Upon cloning them into pGCC2, the resulting vectors were used in liquid-medium natural transformation assays into wild-type 8013 cells, and transformation frequencies (antibiotic-resistant cfu ml⁻¹/total cfu ml⁻¹) were determined. For comparison we also cloned a spacer 1 target without any flanking sequences. The results are shown in Figure 4B. Empty pGCC2 exhibited a transformation frequency of 3.9 x 10⁻⁶ (Table 5), consistent with previous reports (Rusniok et al., 2009). Plasmids carrying targets for spacers 1, 16, 18 and 23 all exhibited transformation frequencies of 2-4 x 10⁻⁶ (Table 5), i.e. comparable to that of the empty vector. The cloned protospacers in these plasmids either lack flanking *Neisseria* sequences or have flanking sequences that deviate significantly from the PAM consensus (Figures 3B and 10). In contrast, protospacers 8, 9, 17 and 25, all four of which have flanking sequences that conform to the PAM consensus, consistently failed to yield transformants, indicating that they likely elicited CRISPR interference. Protospacers matching CRISPR spacers 9 and 25 (Figure 10) cloned into a different transformation vector, pYZEJS040 (which confers chloramphenicol resistance), for targeted integration into the distinct capsule locus also yielded no transformants (Figure 11), demonstrating that the observed effect was independent of the vector, the integration locus, and the selectable marker.

To examine targeting requirements further, we generated a series of mutations in the protospacer or flanking sequences of the pGCC2-derived plasmid targeted by spacer 9 (Figure 4C). Substitutions of two consecutive nucleotides within the PAM or the seed sequence of the protospacer yielded plasmids with transformation frequencies comparable to that of the empty vector (mutants 3 and 5; Figure 4C and Table 5), indicating that interference was abolished. In contrast, two-nucleotide substitutions in a non-PAM flanking region, substitutions in non-seed protospacer positions, small deletions at the PAM-distal end of the spacer, or a wild-type protospacer cloned in the opposite orientation all had no effect on interference (mutants 1, 2, 4, 6, 7 and 8). All of these observations are consistent with previously defined characteristics of functional Type II CRISPR/Cas systems (Deveau et al., 2008; Sapranauskas et al., 2011; Gasiunas et al., 2012; Jinek et al., 2012), indicating that CRISPR interference is indeed responsible for the observed effects on transformation frequencies. The effects of single-nucleotide mutations in this apparent 4-nt PAM remain to be defined.

Genetic analysis of the *N. meningitidis* CRISPR/*cas* locus. In other Type II CRISPR/Cas systems, Cas9 is the only Cas protein that is necessary for interference specified by existing spacers (Barrangou et al., 2007; Deltcheva et al., 2011; Sapranauskas et al., 2011; Jinek et al., 2012). To investigate if Type II-C CRISPR/Cas systems exhibit the same Cas protein requirements, we introduced transposon insertion mutations in the three *cas* genes - *cas1, cas2,* and *cas9* - in *N. meningitidis* 8013 (Figure 5A). We also generated an unmarked, in-frame *cas9* deletion strain to avoid potential polar effects and to generate a guaranteed null allele (Figure 5A). We transformed wild-type and mutant strains with a pYZEJS040 construct carrying protospacer 25 (as in Figure 11B), and compared their transformation frequencies with those of empty pYZEJS040. As expected, the empty vector readily transformed all strains, with transformation frequencies in the range of ∼0.5-7 x 10⁻⁵ (Figure 5B and Table 5). No transformants were observed when the protospacer 25 construct was used with wild-type cells, indicating effective CRISPR interference. Similarly, transposon insertion mutants in *cas1, cas2,* or a control irrelevant gene (*cas1::Tn, cas2::Tn* and *1851::Tn,* respectively) exhibited complete interference, consistent with previous results in Type II-A systems (Sapranauskas et al., 2011). In contrast, CRISPR function is abolished in both the transposon-induced (*cas9::Tn*) and deletion (*Δcas9*) mutations in *cas9.* The CRISPR interference defect of both alleles could be complemented with wild-type *cas9* under the control of its native promoter (integrated chromosomally via pGCC2), whereas empty pGCC2 had no effect (Figure 5B).

Previous studies of *cas9* orthologs from *S. thermophilus* and *S. pyogenes* identified two distinct active sites in RuvC-like and HNH nuclease motifs that are important for Cas9 function *in vivo* and *in vitro* (Sapranauskas et al., 2011; Gasiunas et al., 2012; Jinek et al., 2012). We engineered alanine mutants in corresponding catalytic residues (D16 in the RuvC domain and H588 in the HNH domain) and tested the abilities of both single mutants to complement the interference defect of the *cas9::Tn* mutant. Both alanine mutants failed to restore interference (Figure 5B). Altogether these analyses demonstrate that the *Neisseria* Type II-C CRISPR/Cas system requires *cas9* but not *cas1* or *cas2* for interference of natural transformation, and that the presence of intact RuvC-like and HNH motifs are essential for *cas9* function.

RNase III-catalysed pre-crRNA processing is dispensable for Type II-C CRISPR interference. Two additional loci - *tracrRNA* and *rnc* (the gene encoding RNase III) - have been shown to be essential for crRNA processing and interference in the Type II-A system of *S. pyogenes* SF370 (Deltcheva et al., 2011). The unique *Neisseria* biogenesis pathway described above, in which repeat-driven promoters yield crRNAs with unprocessed 5' ends, raises questions about the roles of tracrRNA and RNase III in this Type II-C system. To examine this issue, we generated *N. meningitidis* 8013 derivatives carrying a transposon-induced allele of *rnc,* or a complete deletion of either *rnc* or *tracrRNA* (Figure 5A). These strains were tested in liquid transformation assays as described above, using pYZEJS040 and its protospacer 25-bearing derivative. The results are shown in Figure 5C. The Δ*tracrRNA* strain was completely defective in CRISPR interference, but the defect was restored upon integration of a *tracrRNA* gene with its native promoter in a distinct chromosomal locus. These results are consistent with the strict requirement for *S. pyogenes* tracrRNA for pre-crRNA processing and interference *in vivo* (Deltcheva et al., 2011), as well as crRNA-directed, Cas9-catalyzed DNA cleavage *in vitro* (Jinek et al., 2012) and in eukaryotes (Cong et al., 2013).

Intriguingly, despite the previously demonstrated importance of the tracrRNA as a guide for RNase III-mediated processing, we detected no interference defect in either the *rnc::Tn* or Δ*rnc* mutants (Figure 5C). This result is in stark contrast to that observed in the Type II-A system in *S. pyogenes* SF370 (Deltcheva et al., 2011). The lack of an interference defect was observed with a vector that is targeted by an internal spacer (spacer 9; Table 5) as well as a terminal spacer (spacer 25; Figure 5C and Table 5). Northern analyses revealed clear processing defects in both crRNA (Figure 5D) and tracrRNA (Figure 5E) for the *N. meningitidis* 8013 *rnc::Tn* mutant, consistent with the results described above with a Δ*rnc* mutant in the WUE2594 strain background (Figure 2D). Mature, 48 nt crRNAs are virtually absent in the *rnc::Tn* mutant, and longer precursors accumulate (Figures 5D and 12). CrRNAs are also strongly depleted in *cas9* mutants, but unlike in the *rnc::Tn* mutant, pre-crRNAs do not accumulate. The latter observation suggests that Cas9 is important for crRNA stability but not processing, or that Cas9 functions in processing and also stabilizes unprocessed precursors. We conclude that the *N. meningitidis* Type II-C CRISPR/Cas system, unlike all other CRISPR/Cas systems characterized to date, does not require pre-crRNA processing for interference activity. RNase III-catalyzed pre-crRNA processing occurs within the bacterial cell but is dispensable for interference. The tracrRNA requirement likely reflects its involvement in target DNA binding and cleavage by crRNA-programmed Cas9, as observed *in vitro* (Jinek et al., 2012).

CRISPR interference limits transformation by *Neisseria* genomic DNA. Plasmids are rare in *N. meningitidis* (van Passel et al., 2006), and *Neisseria* genomic DNA (gDNA) is thought to be the most frequent substrate for natural transformation (Hamilton and Dillard, 2006). To test whether our results with *E*. *coli*-isolated plasmids extend to *Neisseria* gDNA, we generated strains carrying a selectable marker tightly linked to a validated target (protospacer 25). We used the *cas9::Tn* strain to enable transformation and integration of both empty pGCC2 (Figure 4A) and protospacer 25-containing pGCC2 into the meningococcal chromosome (Figure 6A). We then isolated gDNA from these strains and used them as donors in liquid-medium transformation assays with wildtype *N*. *meningitidis* 8013 cells. Transformants (transformation frequency of 1.6 x 10⁻⁵; Table 5) were readily obtained using DNA that lacked protospacer 25 adjacent to the *ermC* marker, whereas no transformants were observed when the protospacer was present (Figure 6B, left panel). Similar results were obtained with gDNA strains carrying the *CAT* marker at the capsule locus with and without tightly-linked protospacer 25 (Figures 6 and 11A, and Table 5), indicating that the interference effect was not marker- or locus-specific. We conclude that CRISPR interference is effective against the most common natural substrate for transformation in *N. meningitidis.*

The potential target spectrum of *Neisseria* CRISPR loci. *In silico* target analysis for *N. meningitidis* 8013 CRISPR spacers is summarized in Figure 3B and in much greater detail as part of Figure 9 and 12. Nine out of the 25 spacers have potential targets that match genomic sequences of *N. meningitidis* and *N. gonorrhoeae* strains. Some of these spacers have potential targets either located within known Nf (Neisserial filamentous) prophages or in genes annotated to encode putative phage-associated proteins. For example, spacer 8 matches to genes (NMB1628 and others) encoding putative surface antigen tspB proteins that are phage adsorption protein homologues; spacer 9 has twelve protospacer matches that are all in the intergenic regions between genes encoding phage assembly proteins (NGO1138 and others) and the transposases PivNM/irg (NGO1137 and others) in *N. gonorrhoeae* genomes; spacer 17 targets multiple genes (NMB1749 and others) encoding zonula occludens toxin family proteins known to be Nf prophage assembly/structural proteins (Figure 3B and Table 4) (Kawai et al., 2005; Skaar et al., 2005). In addition, spacers 21 and 23 both have a single match to genes encoding phage-associated proteins (Figure 3B and Table 4). Importantly, there are spacers that match to *Neisseria* genomic sequences(s) not related to known phage or mobile elements at all (to the best of our knowledge), such as spacer 14 (two matched loci, one a hypothetical protein and the other an intergenic region), spacer 25 (a predicted DNA binding protein), spacer 16 (a predicted deacetylase), and spacer 18 (hemagglutinin/hemolysin family proteins).

We also performed *in silico* target analyses in a more prophage-directed way: we examined the available literature for reported *Neisseria* prophage and mobile element sequences (Masignani et al., 2001; Braid et al., 2004; Bille et al., 2005; Kawai et al., 2005; Skaar et al., 2005; Joseph et al., 2011) and then searched them for matches to any of the 325 *Neisseria* protospacers. Overall, among all the 35 unique *Neisseria* spacers with potential targets, 63% (22/35) match a phage-related protospacer by this criterion (Table 4). We noted that apparent prophage targeting by the *N. meningitidis* WUE2594 CRISPR is particularly extensive, accounting for 69% (36/52) of all the phage-related potential matches shown in Table 4. We speculate that the presence of a functional Type II-C CRISPR/Cas system with a dozen prophage-matched spacers has contributed to the lack of Nf prophages in the *N. meningitidis* WUE2594 genome (Joseph et al., 2011 and our observations). The genes most frequently matched (67 out of the 325 protospacers) by *Neisseria* spacers are those encoding homologues of PivNG/PivNM/irg putative transposases and recombinases (Kawai et al., 2005; Skaar et al., 2005) The fact that these genes are usually adjacent to and probably functionally associated with putative Nf prophage and insertion sequences (Skaar et al., 2005; Kawai et al., 2006) suggests that *Neisseria* Type II-C CRISPR/Cas system interferes with the acquisition of Nf prophages by targeting their PivNG/PivNM/irg transposase-encoding loci.

We also observed that the candidate phage-related CRISPR targets almost exclusively belong to filamentous prophages (Table 4), including the 8kb MDA (Meningococcal Disease Associated) island associated with invasiveness and pathogenicity (Bille et al., 2005). In contrast, several Mu-like prophages (Masignani et al., 2001; Braid et al., 2004; Joseph et al., 2011) from *Neisseria* genomes had no CRISPR spacer matches (Table 4). The reasons for the difference in apparent CRISPR targeting of filamentous and Mu-like prophages are not known.

### Discussion

CRISPR interference and the third major pillar of horizontal gene transfer. CRISPR/Cas pathways have been revealed as RNA-directed immune systems that protect bacteria and archaea from phage infection and HGT (Karginov and Hannon, 2010; Marraffini and Sontheimer, 2010; Terns and Terns, 2011; Wiedenheft et al., 2012). Several dozen bacterial species are known to be competent for HGT via natural transformation. Of this subset of bacteria, *Neisseria* spp. are unusual in that their transformation competence is constitutive (Hamilton and Dillard, 2006). Only a few phages are known to infect *N. meningitidis,* and although conjugative plasmids are present in some meningococcal isolates (van Passel et al., 2006), transformation is the major mechanism for mobilization of meningococcal chromosomal loci (Moxon and Jansen, 2005). *Neisseria* genomic sequences are preferred substrates for natural transformation, given that DNA uptake is strongly promoted by a short DNA uptake sequence (DUS) that is highly overrepresented in *Neisseria* spp. chromosomes (Budroni et al., 2011). DNA exchange is so frequent that the population structures of most neisseriae are effectively panmictic, with little propensity for the emergence of clonal subpopulations (Smith et al., 1993). Frequent HGT in *N. meningitidis* is thought to promote capsule switching and other forms of antigenic variation, homology-based DNA repair, and other functions (Hamilton and Dillard, 2006). Native CRISPR/Cas systems have previously been shown to prevent phage infection (and, by inference, phage transduction) and conjugation, which constitute two of the primary routes of HGT. Our results reveal a role for a native CRISPR/Cas system in preventing the third main route of HGT, natural transformation. This is consistent with recent reports that CRISPR/Cas systems can target loci that are already established in bacterial or archaeal chromosomes (Edgar and Qimron, 2010; Gudbergsdottir et al., 2011; Jiang et al., 2013), indicating that interference does not depend on the invasive DNA's route of entry. Similarly, an engineered, heterologous CRISPR/Cas system introduced into *Streptococcus pneumoniae* can block natural transformation during active infection in mice (Bikard et al., 2012). We find that a native CRISPR/Cas system in *N. meningitidis* can block the transformation events that can be so important for immune evasion and other critical aspects of invasiveness and pathogenicity. Intriguingly, the ability of native CRISPR systems to block natural transformation would be expected to enable the selection of spacers that discriminate against specific chromosomal loci that negatively affect the fitness of certain strains or under certain conditions.

Although relatively few phages are known to infect *N. meningitidis,* they are not unknown (Kawai et al., 2005). Several genomic islands have been identified that resemble phages and could therefore represent prophage sequences (Bille et al., 2005, 2008; Joseph et al., 2011). One such sequence, the MDA island, correlates with invasiveness and pathogenicity in young adults (Bille et al., 2005, 2008). The existence of numerous CRISPR spacers with the potential to target these sequences suggests that CRISPR interference plays a role in shaping prophage content and serves phage defense functions in *N. meningitidis,* as elsewhere. CRISPR interference could limit the spread of prophages via either transformation or infection. Accordingly, CRISPR interference could negatively correlate with meningococcal pathogenicity, as suggested in enterococci (Palmer and Gilmore, 2010) and streptococci (Bikard et al., 2012). Alternatively, meningococcal Cas9 could participate in other regulatory events that contribute to pathogenicity, as suggested very recently (Sampson et al., 2013).

It is noteworthy that many *N. meningitidis and N. lactamica* strains encode CRISPR systems, while strains of the closely related *N. gonorrhoeae* with clearly functional CRISPR systems have not been identified. It is believed that these organisms split in relatively recent times (< 100,000 years ago), evolutionarily speaking, but exact estimates have been stymied by the large recombination frequencies in these species (Bennett et al., 2010). It is equally possible that the nasopharyngeal-localized species gained the system after the split, or that *N. gonorrhoeae* lost the CRISPR system after the split. Both pathogens have been suggested not to establish long-lasting clones and tend towards linkage equilibrium (Buckee et al., 2008). It may not be coincidental that *N*. *meningitidis* carries a CRISPR system and can develop semi-clonal lineages (Bart et al., 2001), given that the CRISPR system could provide a short-term barrier to HGT. It is also possible that the co-existence of commensal *Neisseria* species such as *N. lactamica* and *N. meningitidis* in the nasal pharynx (Feil and Spratt, 2001) selects for a system that limits genetic exchange, and intriguingly, some *N. lactamica* and *N. meningitidis* isolates show large amounts of exchange while others show lower signatures of exchange (Hanage et al., 2005; Corander et al., 2012). In contrast, *N. gonorrhoeae* inhabits a niche that is devoid of other closely related bacteria that encode the DUS to allow efficient transformation of their sequences (Vazques et al., 1993; Cehovin et al., 2013).

Towards a minimal CRISPR/Cas system. In CRISPR/Cas systems investigated to date, crRNAs are transcribed from an external promoter, generating a multimeric precursor. The pre-crRNA is processed by endonucleolytic cleavage to generate mature crRNAs (Carte et al., 2008; Haurwitz et al., 2010; Gesner et al., 2011), and processing is essential for interference *in vivo* (Brouns et al., 2008; Deltcheva et al., 2011; Hale et al., 2012; Westra et al., 2012). The potential presence of minimal and apparently fortuitous promoter elements has been noted within certain CRISPRs of *Sulfolobus solfataricus* P2, though they are not thought to account for the functional expression of crRNA and in fact appear to be suppressed by the repeat-binding protein Cbp1 (Deng et al., 2012). The results presented here reveal that streamlined CRISPR/Cas systems exist in which pre-crRNA processing is not essential (Figure 7). In CRISPR-containing strains of *N. meningitidis* and *N. lactamica,* as well as other species such as C. *jejuni,* the CRISPR repeats each contain an extended -10 box that drives transcription initiation within the downstream spacer. Thus, many crRNAs contain 5'-triphosphate ends that are not subject to further 5'-processing.

Like other Type II CRISPR/Cas systems, neisseriae produce a tracrRNA that apparently anneals to pre-crRNA and enables binding and cleavage by a RNase III. This reaction generates crRNA 3' ends, and *rnc* mutants accumulate multimeric crRNA precursors. However, these *rnc* mutants exhibit no interference defect, indicating that processing is not essential. In addition, while the tracrRNA is essential for interference, its role in directing processing is not, since processing is itself dispensable. This provides the first clear indication that the tracrRNA is required for post-processing events such as target DNA binding and cleavage in bacterial cells, as it is *in vitro* (Jinek et al., 2012).

Among the three main types of CRISPR/Cas pathways, the Type II systems are the simplest ones characterized thus far, as judged by the number of components and essential steps. Both Type II-A and Type II-B systems include the CRISPR array itself, a tracrRNA, four protein-coding genes encoded within the *cas* locus, and the host factor RNase III (Deltcheva et al., 2011; Makarova et al., 2011b; Magadán et al., 2012; Chylinski et al., 2013). The *Neisseria* systems that we have characterized are even more streamlined: they do not require a separate leader sequence to drive crRNA transcription, they lack one of the four *cas*/*csn* genes present in Type II-A or II-B systems, and they do not require RNase III or crRNA processing. The *Neisseria* systems are among the founding members of a new CRISPR/Cas subtype (Type II-C) that is characterized by a smaller number of *cas*/*csn* proteins (Koonin and Makarova, 2013; Chylinski et al., 2013), and in at least some cases by repeat-embedded promoters and processing independence.

Importantly, recent reports have shown that Type II CRISPR/Cas systems can be ported into eukaryotic cells and employed for RNA-directed genome editing and genome binding, including multiplexed applications specified by multiple spacers (Jinek et al., 2012, 2013; Cho et al., 2013; Cong et al., 2013; DiCarlo et al., 2013; Hwang et al., 2013; Mali et al., 2013; Qi et al., 2013). The Cas9 effector proteins from neisseriae share the conserved features observed in the *S. pyogenes* and *S. thermophilus* Cas9 enzymes used in these studies (Chylinski et al., 2013). The fewer the functional requirements for the operation of such systems, the greater their versatility and applicability will be. Separately encoded crRNAs and tracrRNAs are more efficient *in vivo* than single-guide RNAs that combine essential crRNA and tracrRNA domains in the same transcript (Cong et al., 2013). Although endogenous eukaryotic activities can substitute for bacterial RNase III to process tracrRNA/pre-crRNA hybrids in human and mouse cells (Cong et al., 2013), it is not known whether RNase III will be dispensable in other eukaryotic contexts, or indeed in all mammalian cell types. Accordingly, the identification of processing-independent CRISPR/Cas systems could increase the range of applications in eukaryotic genome editing, especially in light of the potential toxicity of bacterial RNase III expression (Pines et al., 1988). Such applications will benefit from further analysis of meningococcal Cas9 activity, including the definition of the presumptive cleavage sites relative to the PAM.

### Experimental Procedures

Bacterial Strains, Plasmids, and Oligonucleotides. *N. lactamica* 020-06, *N. meningitidis* WUE2594 and 8013, and mutant derivatives thereof that were used in this study are listed in Supplemental Experimental Procedures, as are complete lists of all plasmids and DNA oligonucleotides.

Mutant Strain Construction. All mutants were confirmed by PCR and DNA sequencing. Most mutant strains were generated by transformation with appropriately constructed plasmids. For generation of the *cas9, rnc,* and control NMV_1851 transposon-induced alleles in the 8013 strain background, we used gDNA from the corresponding mutant in the NeMeSys collection (Rusniok et al., 2009) to transform 8013. For generation of the Δ*rnc* derivative of 8013, we used gDNA from the WUE2594 Δ*rnc* derivative that was initially made by a plasmid-based approach. For complementation of *cas9::Tn,* Δ*cas9,* and Δ*tracrRNA* mutants, we cloned wildtype copies of the relevant gene into plasmid pGCC2 and transformed the resulting plasmids into the parental mutant strain.

RNA Extraction and Depletion of Processed RNAs. For 020-06, WUE2594 and its mutant derivatives, total RNA was extracted from frozen cell pellet lysates using the hot-phenol method described previously (Blomberg *et al.,* 1990). For depletion of processed transcripts, equal amounts of total RNA were incubated with Terminator™ exonuclease (TEX) (Epicentre) or in buffer alone as described (Sharma et al., 2010). For 8013 and its mutant derivatives, total RNAs were extracted from frozen cell pellets using miRNeasy Mini Kit (Qiagen) with two additional steps: a ten minute initial cell lysis with lysozyme and Proteinase K, and a later on-column DNase digestion step (the RNase-Free DNase Set, Qiagen).

dRNA-seq. Libraries for Solexa sequencing (HiSeq) of cDNA were constructed by vertis Biotechnology AG, Germany (http://www.vertis-biotech.com/), as described previously for eukaryotic microRNA (Berezikov *et al.,* 2006) but omitting the RNA size-fractionation step prior to cDNA synthesis. cDNA libraries were sequenced using a HiSeq 2000 machine (Illumina) in single read mode at the Max Planck Genome Centre Cologne (Cologne, Germany). Data analysis was done as described (Chao et al., 2012), with the only exception being that the minimal read length after trimming and clipping was 12 nt instead of 20 nt.

Transcriptional *gfp* Fusions. The inserts used for the construction of the transcriptional *gfp* fusion plasmids were generated with synthetic DNA oligonucleotides. *E. coli* cells were transformed with these plasmids and grown on agar plates for fluorescence imaging. To measure GFP fluorescence, single colonies were grown in LB broth, fixed, and analyzed by flow cytometry.

*In vitro* Transcription. Templates for *in vitro* transcription assays were PCR-generated, gel-purified 210 bp DNA fragments amplified from pNH13, pNH14, or pNH15. Transcription reactions with sigma-saturated *E. coli* RNA Polymerase holoenzyme (Epicentre) included α-[³²P]-ATP.

Natural Transformation. Natural transformation assays were performed as described (Duffin and Seifert, 2012). Transformation frequencies were reported as antibiotic-resistant cfu/ml divided by total cfu/ml from at least three independent experiments (mean ±s.e.m.).

Accession Numbers. The Gene Expression Ominbus (GEO) accession number for the dRNA-Seq data reported in this paper is GSE44582.

Bacterial Strains and Growth Conditions. *N. lactamica* 020-06, *N. meningitidis* WUE2594 and 8013, and mutant derivatives thereof that were used in this study are listed below.

| **Strain names** | **Relevant genotypes** | **Source** |
|---|---|---|
| *N. lactamica* 020-06 | Wild type | Dr. Julia Bennett |
| *N. meningitidis* WUE2594 | Wild type | Dr. Christoph Schoen lab collection |
| | Δ*rnc* | This study |
| *N. meningitidis* 8013 | Wild type | Dr. Hank Seifert lab collection |
| | *1851::Tn* | Dr. Vladimir Pelicic, genomic DNA |
| | *cas9::Tn* | Dr. Vladimir Pelicic, genomic DNA |
| | *rnc::Tn* | Dr. Vladimir Pelicic, genomic DNA |
| | Δ*rnc* | This study |
| | *cas1::Tn* | This study |
| | *cas2::Tn* | This study |
| | cas*9::Tn*/pGCC2 | This study |
| | *cas9::Tn*/pGCC2-*cas9* wt | This study |
| | *cas9::Tn*/pGCC2*-cas9* D16A | This study |
| | *cas9::Tn*/pGCC2*-cas9* H588A | This study |
| | Δ*cas9* | This study |
| | Δ*cas9*/pGCC2 | This study |
| | Δ*cas9*/pGCC2-*cas9* wt | This study |
| | Δ*tracr* | This study |
| | Δ*tracr*/pGCC2 | This study |
| | Δ*tracr*/pGCC2-*tracr* | This study |
| | *cas9::Tn*/pGCC2-protospacer25 | This study |
| | *cas9::Tn*/pYZEJS040 | This study |
| | *cas9::Tn*/pYZEJS040-protospacer25 | This study |

Strain 8013 and its derivatives were grown on GC Medium Base (GCB) (Difco) plates with appropriate antibiotics and Kellogg's supplements I and II (22.2 mM glucose, 0.68 mM glutamine, 0.45 mM co-carboxylase, 1.23 mM Fe(NO₃)₃; all from Sigma). Antibiotic concentrations used for 8013 were 2.5 µg/ml for erythromycin; 50 µg/ml for kanamycin; 50 µg/ml for streptomycin; and 2.5 µg/ml for chloramphenicol. 020-06, WUE2594, and derivatives thereof were grown on GC agar (Difco) with PolyViteX (bioMerieux), and with 7 µg/ml chloramphenicol when appropriate. All solid cultures were incubated at 37°C in a 5% CO₂ humidified atmosphere.

Liquid cultures of 020-06, WUE2594 and its derivatives were grown in a 37°C shaker-incubator at 220 rpm without added CO₂. Bacteria grown on Columbia agar plates with 5% sheep blood (bioMerieux) were harvested and a starter culture was inoculated to a final OD₆₀₀ of 0.4 in a flask containing 10 ml of Proteose Peptone Media (PPM⁺) medium supplemented with PolyViteX (bioMerieux). After one hour the starter culture was used to inoculate a flask containing 25 ml PPM⁺/PolyViteX to a final OD₆₀₀ of 0.05. When the cultures reached mid-log (OD₆₀₀ 0.5) or early stationary (OD₆₀₀ 1.0) phase, 10 ml of culture were harvested. The cell samples were immediately centrifuged for 10 min at 4,000 rpm. The cell pellet was frozen in liquid N₂ and stored at -80°C until RNA extraction.

Mutant Strain Construction. All mutants were confirmed by PCR and DNA sequencing. PCRs for verifying strains or transformants were performed with Taq or OneTaq DNA Polymerases (NEB) using either 10 ng of genomic DNA (25 cycles) or 0.5 µl CLS extracts (35 cycles) as templates. Chromosomal DNAs were isolated using QIAamp DNA Mini Kit (Qiagen).

The *cas1* and *cas2* transposon-induced alleles were made by transforming 8013 with the plasmids pCR2.1/cas1-Kan and pCR2.1/cas2-Kan, respectively, followed by Kan^{R} selection. For generation of the *cas9, rnc,* and control NMV_1851 transposon-induced alleles in the 8013 strain background, we used chromosomal DNA from the corresponding mutant in the NeMeSys collection (Rusniok et al., 2009) to transform 8013, and then selected Kan^{R} transformants. The *cas9::Tn* mutant strain with a transposon insertion after the 604^{th} nucleotide of the ORF was constructed with NeMeSys mutant 23/6. The *rnc::Tn* mutant strain with a transposon insertion after 574^{th} nucleotide of the ORF was constructed with NeMeSys mutant 6/47. A control strain with a transposon insertion after the 22^{nd} ORF nucleotide of gene NMV_1851 (which encodes a hypothetical protein) was constructed using NeMeSys mutant 73/5. The *kan*-marked Δ*tracrRNA* strain was made by transforming 8013 with plasmid pSMARTHCamp/Δtracr+Kan, followed by Kan^{R} selection.

The WUE2594 Δ*rnc* derivative was constructed by replacing the *rnc* gene with a kanamycin resistance cassette. WUE2594 was then transformed with the plasmid pBJ1 and Kan^{R} colonies were selected. The Δ*rnc* derivative of 8013 was made by transforming 8013 with genomic DNA from the WUE2594 Δ*rnc* derivative, followed by Kan^{R} selection.

To create the unmarked, in-frame Δ*cas9* allele (removing all ORF nts except for the five N-terminal and five C-terminal codons), we first selected a spontaneous streptomycin-resistant (Sm^{R}) mutant of 8013 by plating 3 x 10⁹ wt cells on GCB plates with 50 µg/ml streptomycin, and selecting an Sm^{R} colony. We confirmed that it carried an A128G substitution in *rpsL,* resulting in a K34R missense mutation. We then transformed this Sm^{R} derivative with plasmid *pSTblue-*1/Δcas9/*CAT-rpsL,* in which a dual-marker cassette [*CAT* (chloramphenicol acetyltransferase) and wild-type *rpsL*] replaced most of the *cas9* ORF. The resulting Cm^{R} transformants are also streptomycin-sensitive (Sm^{S}), since the Sm^{S} phenotype conferred by the wildtype *rpsL* is dominant over the Sm^{R} phenotype conferred by the *rpsL^{A128G}* allele at the native locus. Sm^{S} Cm^{R} transformants were then transformed with plasmid pSTblue-1/Δcas9. Sm^{R} Cm^{S} colonies from this transformation were screened by PCR to confirm replacement of the dual marker cassette with the unmarked *cas9* deletion.

For complementation of *cas9::Tn,* Δ*cas9,* and Δ*tracrRNA* mutants, we cloned wildtype copies of the relevant gene into plasmid pGCC2, transformed the resulting plasmids into the parental mutant strain, and selected erythromycin-resistant (Erm^{R}) transformants.

To generate strains carrying a selectable marker tightly linked to a target protospacer (as a source of chromosomal DNA for genomic transformation experiments), plasmids pGCC2 or pGCC2-MC8013spacer25 were transformed into the *cas9::Tn* strain, and Erm^{R} transformants were selected. Similarly, pYZEJS040 or pYZEJS040-MC8013spacer25 were transformed into the *cas9::Tn* strain, and Cm^{R} transformants were selected.

Plasmids. A complete list of all plasmids, as well as information on their construction, is provided at the end of this section. *E. coli* Top10 cells (Invitrogen) were used for all cloning procedures. All plasmid constructions were sequence-verified. PCR reactions for cloning were performed with Platinum Pfx DNA Polymerase (Invitrogen).

The inserts used for the construction of transcriptional *gfp* fusion plasmids pNH13, pNH14, pNH15 and pNH18 were generated by duplex formation of complementary DNA oligonucleotides. Oligonucleotide pairs were JVO9535/JVO9536 and JVO9537/JVO9538 for *Neisseria* spp. wildtype (pNH13) and mutant (pNH14) CRISPR repeat constructs, respectively; JVO9599/JVO9601 for the wildtype CRISPR repeat from *Campylobacter jejunii* NCTC11168 (pNH18); and JVO9539/JVO9540 for the -10/-35 positive control promoter from T7A1 phage (pNH15). For each DNA duplex insert, 100 nM sense oligonucleotides were annealed with equimolar amounts of antisense oligonucleotides at 95°C for 3 min, followed by slow cooling to room temperature. DNA duplexes were digested with *Aat*II/*Nhe*I and cloned into *Aat*II/*Nhe*I-digested pAS093. For construction of *3x CRISPR-repeat-spacer unit-gfp* transcriptional fusion plasmid pNH17, the plasmid pAS093 was digested with *Aat*II/*Nhe*I and ligated to *Aat*II/*Nhe*I-digested PCR products amplified from *N. lactamica* 020-06 chromosomal DNA with primer pairs JVO9585/JVO9548.

To generate the pBJ1 plasmid used for creating the Δ*rnc* mutation in WUE2594, ∼600 bp upstream and downstream of the *rnc* gene were amplified with the primer pairs rnc1/rnc2 and rnc3/rnc4, respectively, using WUE2594 genomic DNA as template. The oligonucleotides were modified so as to introduce *Bam*HI/*Eco*RI site at the 5' and 3' ends (respectively) of the upstream fragment, and *Eco*RI/*Hind*III sites at the 5' and 3' ends (respectively) of the downstream fragment. These fragments were cloned into the pBluescript II SK(+) vector (Invitrogen) along with an *Eco*RI-digested fragment of pUC4K (GE Healthcare) containing the kanamycin cassette, yielding the knock-out plasmid (pBJ1) that contains the kanamycin cassette flanked on either side by the upstream and downstream regions of *rnc.*

Short putative targets for strain 8013 CRISPR spacers 1 (30 nts), 16 (50 nts), 23 (50 nts), and 25 (50 nts) were created by annealing synthetic oligonucleotide pairs OYZ001/OYZ002, OYZ007/OYZ008, OYZ011/OYZ012, and OYZ015/OYZ016, respectively. Longer (208, 350, 305, and 203 nt) putative targets for spacers 8, 9, 17, 18 of 8013 were PCR-amplified from the chromosomal DNAs of *N. meningitidis* strain MC58, *N. gonorrhoea* strain FA1090, and *N. meningitidis* strains MC58 and Z2491 respectively, and digested with *Aat*II and *Pac*I. Primer pairs for these PCRs were OYZ003/OYZ004, OYZ005/OYZ006, OYZ009/0YZO10, and OYZ013/OYZ014, respectively. All eight of these putative targets were ligated into pGCC2 via *Aat*II and *Pac*I sites, to create pGCC2 derivatives for interference tests.

pYZEJS040 (pSTblue-1/*siaA*+*CAT*+*ctrA*) was constructed by PCR-amplifying three individual fragments: a 562nt *siaA* fragment from 8013 chromosomal DNA using primers OYZ036/OYZ037; a 561nt *ctrA* fragment from 8013 chromosomal DNA using primers OYZ040/OYZ041; and a 1239nt *CAT* cassette from the pGCC5 vector using primers OYZ038/OYZ039. 100ng of each of the three fragments were added to a 50 µl PCR reaction without any primers. After 15 cycles of PCR, outside primers OYZ036/OYZ041 were added and 20 more cycles were performed. The ends of the 2.3 kb fusion product *siaA-CAT-ctrA* were blunted, and the fragment was ligated into the *EcoRV* site of pSTblue-1 to yield pYZEJS040. The pYZEJS040 derivatives used in interference tests were generated by ligating potential targets for 8013 CRISPR spacers 9 and 25 into pYZEJS040 via the *Aat*II and *Pac*I sites.

To construct plasmid *pCR*2.1/*cas1*-Kan, a 2.4 kb insert was PCR-amplified from the chromosomal DNA of NeMeSys strain 10/4 (Rusniok et al., 2009) using primers OYZ060/OYZ061. This insert, which contains a 1.6 kb Kan^{R} transposon inserted into the *cas1* gene, was cloned using Original TA Cloning Kit pCR2.1 (Invitrogen) according to the manufacturer's instructions. Similarly, plasmid pCR2.1/*cas2*-Kan was created by amplifying a 2.45 kb insert from the chromosomal DNA of NeMeSys strain 71/27 using primers OYZ052/OYZ055, and cloning that fragment into pCR2.1.

To create plasmids to be used in generating the unmarked Δ*cas9* mutant, genomic sequences upstream and downstream of *cas9* gene were PCR amplified, fused together via overlapping PCR and cloned into pSTblue-1. A 662 nt region containing the first 15 nt of the *cas9* ORF and 632 nt upstream of *cas9* was PCR-amplified from 8013 genomic DNA using primers OYZ066/OYZ068. Similarly, a 517nt region containing the last 15 nt of the *cas9* ORF and 487nt downstream of *cas9* was amplified using primers OYZ069/OYZ071. 100 ng of both PCR fragments were added to a 50 µl PCR reaction without any primers. After 15 cycles of PCR, outside primers OYZ066/OYZ071 were added and 20 more PCR cycles were performed. The resulting 1.2 kb fusion product included internal *Sal*I and *Spe*I sites (originally incorporated in the primers). The ends of the fragment were blunted, and the product was ligated into the *Eco*RV site of pSTblue-1 to create plasmid pSTblue-1/Δ*cas9+Sal*I*-Spe*I*.* The *Sal*I and *Spe*I sites of this plasmid were used to introduce a 1.6kb *CAT-rpsL* dual marker cassette, and resulted in pSTblue-1/Δ*cas9*/*CAT-rpsL.* The plasmid pSTblue-1/Δ*cas9* was generated similarly: 647 nt and 502 nt genomic fragments upstream and downstream of the *cas9* gene, including the 15 nts at each terminus of the ORF, were amplified using primers OYZ066/OYZ067 and OYZ070/OYZ071, respectively, and then fused together by overlapping PCR. The 1.2 kb fusion product was blunted and ligated into the *Eco*RV site of pSTblue-1.

To create pSMARTHCAmp/Δ*tracr*+*Pme*I, genomic sequences upstream and downstream of the tracrRNA region were PCR amplified and fused together via overlapping PCR. The 638nt upstream region and the 598nt downstream region were amplified from 8013 chromosomal DNA using primer pairs OYZ081/OYZ082 and OYZ083/OYZ084, respectively. 100 ng of both fragments were added to a 50 µl PCR reaction without any primers, and after 15 cycles, outside primers OYZ081/OYZ084 were added and 20 more cycles were performed. The 1.2 kb fusion product included an internal *Pme*I site (designed in the primers). The fragment was blunted and ligated into vector pSMARTHCAMP according to the instructions for the CloneSmart Cloning Kit (Lucigen). The *Pme*I site was used to insert a 1.2 kb Kan^{R} cassette that had been amplified from NeMeSys mutant 23/6 chromosomal DNA using primer pair OYZ085/OYZ086. This yielded plasmid pSMARTHCAmp/Δ*tracr*+Kan.

Complementation plasmid pGGC2/promoter+*cas9*wt was created by amplifying the *cas9* ORF and its native promoter from 8013 genomic DNA using primer pair OYZ072/OYZ073, digesting the PCR product with *Aat*II and *Pac*I*,* and then ligating it into pGCC2 via the *Aat*II/*Pac*I sites. pGGC2/promoter+tracr was created by amplifying the tracrRNA locus with its native promoter from 8013 genomic DNA using primer pair OYZ091/OYZ092, digesting the PCR product with *Aat*II and *Pac*I*,* then ligating it into pGCC2 via the *Aat*II/*Pac*I sites.

| **Plasmids** | **Relevant characteristics** | **Source** |
|---|---|---|
| **Plasmids for *in vivo* interference assays** | | |
| pYZEJS001 | pGCC2 empty vector | Dr. Hank Seifert lab collection |
| pYZEJS010 | pGCC2-MC8013 spacer1 target | This study |
| pYZEJS011 | pGCC2-MC8013 spacer8 target | This study |
| pYZEJS012 | pGCC2-MC8013 spacer9 target | This study |
| pYZEJS014 | pGCC2-MC8013 spacer16 target | This study |
| pYZEJS015 | pGCC2-MC8013 spacer17 target | This study |
| pYZEJS016 | pGCC2-MC8013 spacer18 target | This study |
| pYZEJS017 | pGCC2-MC8013 spacer23 target | This study |
| pYZEJS018 | pGCC2-MC8013 spacer25 target | This study |
| pYZEJS019 | pGCC2-MC8013 spacer9 target mut1 | This study |
| pYZEJS020 | pGCC2-MC8013 spacer9 target mut2 | This study |
| pYZEJS021 | pGCC2-MC8013 spacer9 target mut3 | This study |
| pYZEJS022 | pGCC2-MC8013 spacer9 target mut4 | This study |
| pYZEJS023 | pGCC2-MC8013 spacer9 target mut5 | This study |
| pYZEJS024 | pGCC2-MC8013 spacer9 target mut6 | This study |
| pYZEJS025 | pGCC2-MC8013 spacer9 target mut7 | This study |
| pYZEJS026 | pGCC2-MC8013 spacer9 target mut8 | This study |
| pYZEJS028 | pGCC5 empty vector | Dr. Hank Seifert lab collection |
| pYZEJS032 | pSTblue-1 empty vector | Novagen |
| pYZEJS040 | pSTblue-1/*sia*A+*CAT*+*ctrA* | This study |
| pYZEJS042 | pYZEJS040-protospacer9 | This study |
| pYZEJS043 | pYZEJS040protospacer25 | This study |

| **Plasmids for creating the Δ*cas9* strain** | | |
|---|---|---|
| pYZEJS033 | pSTblue-1/Δ*cas9*/SalI+SpeI | This study |
| pYZEJS034 | pSTblue-1/Δ*cas9* | This study |
| pYZEJS035 | pSTblue-1/Δ*cas9*/*CAT*+*rpsL* | This study |

| **Plasmids for creating *cas1::Tn* and *cas2::Tn* strains** | | |
|---|---|---|
| pYZEJS037 | pCR2.1/*cas1*-Kan | This study |
| pYZEJS038 | pCR2.1/cas2-Kan | This study |

| **Plasmid for creating the *cas9* complementation strain** | | |
|---|---|---|
| pYZEJS044 | pGCC2-promoter+cas9 wt | This study |

| **Plasmids for creating Δtracr strain** | | |
|---|---|---|
| pYZEJS061 | pSMARTHCAmp/Δ*tracr*+PmeI | This study |
| pYZEJS062 | pSMARTHCAmp/Δ*tracr*+Kan | This study |

| **Plasmid for creating tracr complementation strain** | | |
|---|---|---|
| pYZEJS064 | pGCC2-promoter+*tracr* | This study |

| **Plasmids for creating the Δ*rnc* strain in *N. meningitidis* WUE2594** | | |
|---|---|---|
| pBJ1 | Δ*rnc* | This study |

| **Plasmids for promoter cloning** | | |
|---|---|---|
| pAS093 (P_{invR}-gfp) | *invR* transcriptional *gfp* fusion plasmid, expressing constitutive *gfp* | (Sittka et al., 2008) |
| pAS0046 '*gfp* (P*ₙᵤₗₗ*) | background control plasmid for transcriptional *gfp* fusion plasmid | (Sittka et al., 2007) |
| pNH13 (P_{wt}) | *Neisseria: CRISPR-repeat* transcriptional *gfp* fusion plasmid | This study |
| pNH14 (Pₘᵤₜ) | *Neisseria: mutant CRISPR-repeat* transcriptional *gfp* fusion plasmid | This study |
| pNH15 (P_{ctrl}) | *T7 phage promoter A1* transcriptional *gfp* fusion plasmid | This study |
| pNH17 (P₃ₓ) | *Neisseria: 3x CRISPR-repeat-spacer unit* transcriptional *gfp* fusion plasmid | This study |
| pNH18 (P*_{C.jejuni}*) | *Campylobacter jejuni: CRISPR-repeat* transcriptional *gfp* fusion plasmid | This study |

RNA Extraction, Depletion of Processed RNAs, and Northern Blots. For 020-06, WUE2594 and its mutant derivatives, frozen cell pellets from liquid cultures were resuspended in lysis solution containing 800 µl of 0.5 mg/ml lysozyme in TE buffer (pH 8.0) and 80 µl 10% SDS. Bacterial cells were lysed by placing the samples for 1-2 minutes at 65°C in a water bath. Afterwards, total RNA was extracted from the lysates using the hot-phenol method described previously (Blomberg *et al.,* 1990). For depletion of processed transcripts, total RNA was freed of residual genomic DNA by DNase I treatment, and equal amounts of *Neisseria* RNA were incubated with Terminator 5'-phosphate-dependent exonuclease (TEX) (Epicentre) or in buffer alone as previously described (Sharma et al., 2010). For northern blot analysis, 5 µg total RNA freed of residual genomic DNA or 3 µg of TEX treated RNA was loaded per sample. After separation by electrophoresis in 8% polyacrylamide/8.3 M urea/1x TBE gels, RNA was transferred onto Hybond-XL membranes, and membranes were hybridized with γ-³²P-ATP end-labeled oligodeoxyribonucleotide probes.

For 8013 and its mutant derivatives, cells grown overnight on GCB plates were collected, immediately treated with RNAprotect Bacteria Reagent (Qiagen), and frozen at -80°C for storage. Total RNAs were extracted using miRNeasy Mini Kit (Qiagen) with two additional steps: a 10 min initial cell lysis in 30 mM Tris-HCl (pH 8.0)/1 mM EDTA containing 1.5 mg/ml lysozyme (Invitrogen) and 2 mg/ml Proteinase K (Fermentas), and a later on-column DNase digestion step (The RNase-Free DNase Set, Qiagen). For northern analysis, 8-10 µg of total RNA for each sample was separated by electrophoresis in a 10% polyacrylamide/8 M urea/1x TBE gel. RNAs were electroblotted overnight at 14V to a Genescreen Plus membrane (PerkinElmer) in 1x TBE, cross-linked to the membrane by UV irradiation and then soaking in 0.16M N-(3-dimethylaminopropyl)-N'-ethylcarbodiimide hydrochloride/0.13 M 1-methylimidazole (both from Sigma) (pH 8.0) at 60°C for 2h. Blots were prehybridized with 8 ml ULTRAhyb buffer (Ambion) at 60°C for 30 min, then probed at 37°C overnight with 5 x 10⁶ cpm/ml of the appropriate DNA oligonucleotide probe. Northern probes were 5' end-labeled with γ-³²P-ATP (PerkinElmer) and T4 polynucleotide kinase (NEB). The membranes were washed at room temperature twice with 2x SSC/0.1% SDS for 5 min and then twice with 1x SSC/0.1% SDS for 15 min. RNAs were then visualized by PhosphorImager detection. Similarly end-labeled *Msp*I-digested pBR322 DNAs (NEB) were used as size markers.

Construction of cDNA Libraries for dRNA-seq. Total RNA was freed of residual genomic DNA by DNase I treatment. For depletion of processed transcripts, equal amounts of *Neisseria* RNA were incubated with Terminator™ 5'-phosphate dependent exonuclease (TEX) (Epicentre) as previously described (Sharma et al., 2010). Libraries for Solexa sequencing (HiSeq) of cDNA were constructed by vertis Biotechnology AG, Germany (http://www.vertis-biotech.com/), as described previously for eukaryotic microRNA (Berezikov *et al.,* 2006) but omitting the RNA size-fractionation step prior to cDNA synthesis. In brief, equal amounts of RNA samples were poly(A)-tailed using poly(A) polymerase. Then, the 5'-triphosphate structures were removed using tobacco acid pyrophosphatase (TAP). Afterwards, an RNA adapter was ligated to the 5'-phosphate of the RNA. First-strand cDNA was synthesized by an oligo(dT)-adapter primer and MMLV reverse transcriptase. A PCR-based amplification step with a high-fidelity DNA polymerase was then used to increase the cDNA concentration to 20-30 ng/µl. A library-specific barcode for sequence multiplexing was included in the 3'-sequencing adapter. cDNA libraries were sequenced using a HiSeq 2000 machine (Illumina) in single read mode at the Max Planck Genome Centre Cologne (Cologne, Germany).

Read Mapping and Coverage Plot Construction. Sample preparation, sequencing (Illumina GAIIx) and data analysis was done as described (Chao et al., 2012), with the only exception being that the minimal read length after trimming and clipping was 12 nt instead of 20 nt.

Transcriptional gfp Fusions. *E. coli* cells were transformed with transcriptional *gfp* fusion plasmids and grown on agar plates for fluorescence imaging. To measure GFP fluorescence, single colonies were inoculated in LB broth and grown for 12 h. Cells were then fixed in 4% paraformaldehyde/1x PBS and analysed by flow cytometry.

*In vitro* Transcription. Templates for *in vitro* transcription assays were PCR-generated, gel-purified 210 bp DNA fragments amplified from pNH13, pNH14, or pNH15. Primer pairs were the forward primers used for construction of each DNA duplex insert (see above), together with reverse primer JVO155. Templates (100 ng) were incubated at 37°C in transcription buffer (40 mM Tris-HCl (pH 7.5)/100 mM KCl/10 mM MgCl₂/0.01% Triton/1 mM DTT) together with 1.5 Units sigma-saturated *E. coli* RNA Polymerase Holoenzyme (Epicentre), α-[³²P]-ATP (30 µCi; Hartmann-Analytic Braunschweig), and NTP mix (10 µM ATP and 200 µM each CTP, GTP, UTP). A negative control reaction used water in place of DNA template. 25 µl reactions were incubated for 1, 5, 10 and 30 min. Aliquots were phenol-extracted, precipitated, denatured by heating in formamide loading dye, separated by electrophoresis in 12% sequencing gels, and analyzed with a PhosphorImager.

CRISPR prediction and *in silico* Analysis of Natural Targets. CRISPRs in sequenced *Neisseria* genomes were predicted using CRISPRfinder (http://crispr.u-psud.fr/Server/) (Grissa et al., 2007) and CRISPRI (http://crispi.genouest.org/) (Rousseau et al., 2009). Our initial predictions of *Neisseria* CRISPRs were consistent with those of CRISPRdb (http://crispr.u-psud.fr/crispr/). Spacers were subjected to blastn (Basic Local Alignment Search Tool) search against the nr/nt database (http://www.ncbi.nlm.nih.gov/). Multiple Sequence Alignments were performed using WebLogo (http://weblogo.berkeley.edu/logo.cgi).

Natural Transformation. Natural transformation assays were performed in *N. meningitidis* 8013 and its mutant derivatives as described for *N. gonorrhoeae* (Duffin and Seifert, 2012). 150 ng plasmids or 100 ng chromosomal DNA was used per transformation reaction. 10 µl of serial 10-fold dilutions were spotted on GCB plate in triplicates in the presence and absence of appropriate antibiotics. 200 µl from the undiluted final transformation mixture were also plated on GCB plates with appropriate antibiotics to enhance detection. Eight representative transformants per reaction were verified by restreaking on selective plates twice and then by PCR from CLS extract (i.e., from cells lysed in 1% Triton/20 mM Tris-HCl (pH 8.3)/2mM EDTA at 94°C for 15 min and then 20°C for 5 min). Transformation frequencies were reported as antibiotic-resistant cfu/ml divided by total cfu/ml from at least three independent experiments (mean ± s.e.m.).

### References for Example 1

Barrangou, R., Fremaux, C., Deveau, H., Richards, M., Boyaval, P., Moineau, S., Romero, D.A., and Horvath, P. (2007). CRISPR provides acquired resistance against viruses in prokaryotes. Science 315, 1709-1712.
Bart, A., Barnabé, C., Achtman, M., Dankert, J., van der Ende, A., and Tibayrenc, M. (2001). The population structure of Neisseria meningitidis serogroup A fits the predictions for clonality. Infect. Genet. Evol. 1, 117-122.
Bennett, J.S., Bentley, S.D., Vernikos, G.S., Quail, M.A., Cherevach, I., White, B., Parkhill, J., and Maiden, M.C.J. (2010). Independent evolution of the core and accessory gene sets in the genus Neisseria: insights gained from the genome of Neisseria lactamica isolate 020-06. BMC Genomics 11, 652.
Bhaya, D., Davison, M., and Barrangou, R. (2011). CRISPR-Cas systems in bacteria and archaea: versatile small RNAs for adaptive defense and regulation. Annu. Rev. Genet. 45, 273-297.
Bikard, D., Hatoum-Aslan, A., Mucida, D., and Marraffini, L.A. (2012). CRISPR interference can prevent natural transformation and virulence acquisition during in vivo bacterial infection. Cell Host & Microbe 12, 177-186.
Bille, E., Ure, R., Gray, S.J., Kaczmarski, E.B., McCarthy, N.D., Nassif, X., Maiden, M.C.J., and Tinsley, C.R. (2008). Association of a bacteriophage with meningococcal disease in young adults. PloS One 3, e3885.
Bille, E., Zahar, J.-R., Perrin, A., Morelle, S., Kriz, P., Jolley, K.A., Maiden, M.C.J., Dervin, C., Nassif, X., and Tinsley, C.R. (2005). A chromosomally integrated bacteriophage in invasive meningococci. J. Exp. Med. 201, 1905-1913.
Bolotin, A., Quinquis, B., Sorokin, A., and Ehrlich, S.D. (2005). Clustered regularly interspaced short palindrome repeats (CRISPRs) have spacers of extrachromosomal origin. Microbiology 151, 2551-2561.
Braid, M.D., Silhavy, J.L., Kitts, C.L., Cano, R.J., and Howe, M.M. (2004). Complete genomic sequence of bacteriophage B3, a Mu-like phage of Pseudomonas aeruginosa. J. Bact. 186, 6560-6574.
Bratcher, H.B., Bennett, J.S., and Maiden, M.C.J. (2012). Evolutionary and genomic insights into meningococcal biology. Future Microbiol. 7, 873-885.
Brouns, S.J.J., Jore, M.M., Lundgren, M., Westra, E.R., Slijkhuis, R.J.H., Snijders, A.P.L., Dickman, M.J., Makarova, K.S., Koonin, E. V., and Van der Oost, J. (2008). Small CRISPR RNAs guide antiviral defense in prokaryotes. Science 321, 960-964.
Buckee, C.O., Jolley, K.A., Recker, M., Penman, B., Kriz, P., Gupta, S., and Maiden, M.C.J. (2008). Role of selection in the emergence of lineages and the evolution of virulence in Neisseria meningitidis. Proc. Natl. Acad. Sci. USA 105, 15082-15087.
Budroni, S., Siena, E., Dunning Hotopp, J.C., Seib, K.L., Serruto, D., Nofroni, C., Comanducci, M., Riley, D.R., Daugherty, S.C., Angiuoli, S.V., et al. (2011). Neisseria meningitidis is structured in clades associated with restriction modification systems that modulate homologous recombination. Proc. Natl. Acad. Sci. USA 108, 4494-4499.
Carte, J., Wang, R., Li, H., Terns, R.M., and Terns, M.P. (2008). Cas6 is an endoribonuclease that generates guide RNAs for invader defense in prokaryotes. Genes Dev. 22, 3489-3496.
Cehovin, A., Simpson, P.J., McDowell, M.A., Brown, D.R., Noschese, R., Pallett, M., Brady, J., Baldwin, G.S., Lea, S.M., Matthews, S.J., and Pelicic, V. (2013). Specific DNA recognition mediated by a type IV pilin. Proc. Natl. Acad. Sci. USA 110, 3065-3070.
Chao, Y., Papenfort, K., Reinhardt, R., Sharma, C.M., and Vogel, J. (2012). An atlas of Hfq-bound transcripts reveals 3' UTRs as a genomic reservoir of regulatory small RNAs. EMBO J. 31, 4005-4019.
Chen, I., Christie, P.J., and Dubnau, D. (2005). The ins and outs of DNA transfer in bacteria. Science 310, 1456-1460.
Cho, S.W., Kim, S., Kim, J.M., and Kim, J.-S. (2013). Targeted genome engineering in human cells with the Cas9 RNA-guided endonuclease. Nature Biotech. 31, 230-232.
Chylinski, K., Le Rhun, A., and Charpentier, E. (2013). The tracrRNA and Cas9 families of type II CRISPR-Cas immunity systems. RNA Biol., in press [ePub ahead of print (doi:10.4161/rna24321)].
Cong, L., Ran, F.A., Cox, D., Lin, S., Barretto, R., Habib, N., Hsu, P.D., Wu, X., Jiang, W., Marraffini, L.A., et al. (2013). Multiplex genome engineering using CRISPR/Cas systems. Science 339, 819-823.
Corander, J., Connor, T.R., O'Dwyer, C.A., Kroll, J.S., and Hanage, W.P. (2012). Population structure in the Neisseria, and the biological significance of fuzzy species. J. Royal Soc. Interface 9, 1208-1215.
Deltcheva, E., Chylinski, K., Sharma, C.M., Gonzales, K., Chao, Y., Pirzada, Z.A., Eckert, M.R., Vogel, J., and Charpentier, E. (2011). CRISPR RNA maturation by trans-encoded small RNA and host factor RNase III. Nature 471, 602-607.
Deng, L., Kenchappa, C.S., Peng, X., She, Q., and Garrett, R.A. (2012). Modulation of CRISPR locus transcription by the repeat-binding protein Cbp1 in Sulfolobus. Nucleic Acids Res. 40, 2470-2480.
Deveau, H., Barrangou, R., Garneau, J.E., Labonté, J., Fremaux, C., Boyaval, P., Romero, D.A., Horvath, P., and Moineau, S. (2008). Phage response to CRISPR-encoded resistance in Streptococcus thermophilus. J. Bact. 190, 1390-1400.
DiCarlo, J.E., Norville, J.E., Mali, P., Rios, X., Aach, J., and Church, G.M. (2013). Genome engineering in Saccharomyces cerevisiae using CRISPR-Cas systems. Nucleic Acids Res. 41, 4336-4343.
Duffin, P.M., and Seifert, H.S. (2012). Genetic transformation of Neisseria gonorrhoeae shows a strand preference. FEMS Microbiol. Letters 334, 44-48.
Edgar, R., and Qimron, U. (2010). The Escherichia coli CRISPR system protects from 1 lysogenization, lysogens, and prophage induction. J. Bact. 192, 6291-6294.
Feil, E.J., and Spratt, B.G. (2001). Recombination and the population structures of bacterial pathogens. Annu. Rev. Microbiol. 55, 561-590.
Garneau, J.E., Dupuis, M.-È., Villion, M., Romero, D.A., Barrangou, R., Boyaval, P., Fremaux, C., Horvath, P., Magadán, A.H., and Moineau, S. (2010). The CRISPR/Cas bacterial immune system cleaves bacteriophage and plasmid DNA. Nature 468, 67-71.
Gasiunas, G., Barrangou, R., Horvath, P., and Siksnys, V. (2012). Cas9-crRNA ribonucleoprotein complex mediates specific DNA cleavage for adaptive immunity in bacteria. Proc. Natl. Acad. Sci. USA 109, 2579-2586.
Gesner, E.M., Schellenberg, M.J., Garside, E.L., George, M.M., and Macmillan, A.M. (2011). Recognition and maturation of effector RNAs in a CRISPR interference pathway. Nature Struct. Mol. Biol. 18, 688-692.
Grissa, I., Vergnaud, G., and Pourcel, C. (2007). The CRISPRdb database and tools to display CRISPRs and to generate dictionaries of spacers and repeats. BMC Bioinformatics 8, 172.
Gudbergsdottir, S., Deng, L., Chen, Z., Jensen, J.V.K., Jensen, L.R., She, Q., and Garrett, R.A. (2011). Dynamic properties of the Sulfolobus CRISPR/Cas and CRISPR/Cmr systems when challenged with vector-borne viral and plasmid genes and protospacers. Mol. Microbiol. 79, 35-49.
Haft, D.H., Selengut, J., Mongodin, E.F., and Nelson, K.E. (2005). A guild of 45 CRISPR-associated (Cas) protein families and multiple CRISPR/Cas subtypes exist in prokaryotic genomes. PLoS Comp. Biol. 1, e60.
Hale, C., Kleppe, K., Terns, R.M., and Terns, M.P. (2008). Prokaryotic silencing (psi)RNAs in Pyrococcus furiosus. RNA 14, 2572-2579.
Hale, C.R., Majumdar, S., Elmore, J., Pfister, N., Compton, M., Olson, S., Resch, A.M., Glover, C.V.C., Graveley, B.R., Terns, R.M., et al. (2012). Essential features and rational design of CRISPR RNAs that function with the Cas RAMP module complex to cleave RNAs. Mol. Cell 45, 292-302.
Hale, C.R., Zhao, P., Olson, S., Duff, M.O., Graveley, B.R., Wells, L., Terns, R.M., and Terns, M.P. (2009). RNA-guided RNA cleavage by a CRISPR RNA-Cas protein complex. Cell 139, 945-956.
Hamilton, H.L., and Dillard, J.P. (2006). Natural transformation of Neisseria gonorrhoeae: from DNA donation to homologous recombination. Mol. Microbiol. 59, 376-385.
Hanage, W.P., Fraser, C., and Spratt, B.G. (2005). Fuzzy species among recombinogenic bacteria. BMC Biol. 3, 6.
Haurwitz, R.E., Jinek, M., Wiedenheft, B., Zhou, K., and Doudna, J.A. (2010). Sequence- and structure-specific RNA processing by a CRISPR endonuclease. Science 329, 1355-1358.
Hook-Barnard, I.G., and Hinton, D.M. (2007). Transcription initiation by mix and match elements: flexibility for polymerase binding to bacterial promoters. Gene Reg. Systems Biol. 1, 275-293.
Horvath, P., Romero, D.A., Coûté-Monvoisin, A.-C., Richards, M., Deveau, H., Moineau, S., Boyaval, P., Fremaux, C., and Barrangou, R. (2008). Diversity, activity, and evolution of CRISPR loci in Streptococcus thermophilus. J. Bact. 190, 1401-1412.
Hwang, W.Y., Fu, Y., Reyon, D., Maeder, M.L., Tsai, S.Q., Sander, J.D., Peterson, R.T., Yeh, J.-R.J., and Joung, J.K. (2013). Efficient genome editing in zebrafish using a CRISPR-Cas system. Nature Biotech. 31, 227-229.
Jiang, W., Bikard, D., Cox, D., Zhang, F., and Marraffini, L.A. (2013). RNA-guided editing of bacterial genomes using CRISPR-Cas systems. Nature Biotech. 31, 233-239.
Jinek, M., Chylinski, K., Fonfara, I., Hauer, M., Doudna, J.A., and Charpentier, E. (2012). A programmable dual-RNA-guided DNA endonuclease in adaptive bacterial immunity. Science 337, 816-821.
Jinek, M., East, A., Cheng, A., Lin, S., Ma, E., and Doudna, J. (2013). RNA-programmed genome editing in human cells. eLife 2, e00471.
Jolley, K.A., and Maiden, M.C.J. (2010). BIGSdb: Scalable analysis of bacterial genome variation at the population level. BMC Bioinformatics 11, 595.
Joseph, B., Schwarz, R.F., Linke, B., Blom, J., Becker, A., Claus, H., Goesmann, A., Frosch, M., Müller, T., Vogel, U., et al. (2011). Virulence evolution of the human pathogen Neisseria meningitidis by recombination in the core and accessory genome. PloS One 6, e18441.
Karginov, F. V, and Hannon, G.J. (2010). The CRISPR system: small RNA-guided defense in bacteria and archaea. Mol. Cell 37, 7-19.
Kawai, M., Nakao, K., Uchiyama, I., and Kobayashi, I. (2006). How genomes rearrange: genome comparison within bacteria Neisseria suggests roles for mobile elements in formation of complex genome polymorphisms. Gene 383, 52-63.
Kawai, M., Uchiyama, I., and Kobayashi, I. (2005). Genome comparison in silico in Neisseria suggests integration of filamentous bacteriophages by their own transposase. DNA Research 12, 389-401.
Koonin, E. V., and Makarova, K.S. (2013). CRISPR-Cas: evolution of an RNA-based adaptive immunity system in prokaryotes. RNA Biol., in press [ePub ahead of print (doi:10.4161/rna24022)].
Magadán, A.H., Dupuis, M.-È., Villion, M., and Moineau, S. (2012). Cleavage of phage DNA by the Streptococcus thermophilus CRISPR3-Cas system. PLoS ONE 7, e40913.
Makarova, K.S., Aravind, L., Wolf, Y.I., and Koonin, E. V (2011a). Unification of Cas protein families and a simple scenario for the origin and evolution of CRISPR-Cas systems. Biol. Direct 6, 38.
Makarova, K.S., Grishin, N. V, Shabalina, S.A., Wolf, Y.I., and Koonin, E. V (2006). A putative RNA-interference-based immune system in prokaryotes: computational analysis of the predicted enzymatic machinery, functional analogies with eukaryotic RNAi, and hypothetical mechanisms of action. Biol. Direct 1, 7.
Makarova, K.S., Haft, D.H., Barrangou, R., Brouns, S.J.J., Charpentier, E., Horvath, P., Moineau, S., Mojica, F.J.M., Wolf, Y.I., Yakunin, A.F., et al. (2011b). Evolution and classification of the CRISPR-Cas systems. Nature Rev. Microbiol. 9, 467-477.
Mali, P., Yang, L., Esvelt, K.M., Aach, J., Guell, M., DiCarlo, J.E., Norville, J.E., and Church, G.M. (2013). RNA-guided human genome engineering via Cas9. Science 339, 823-826.
Marraffini, L.A. and Sontheimer, E.J. (2008). CRISPR interference limits horizontal gene transfer in staphylococci by targeting DNA. Science 322, 1843-1845.
Marraffini, L.A. and Sontheimer, E.J. (2010). CRISPR interference: RNA-directed adaptive immunity in bacteria and archaea. Nature Rev. Genet. 11, 181-190.
Masignani, V., Giuliani, M.M., Tettelin, H., Comanducci, M., Rappuoli, R., and Scarlato, V. (2001). Mu-like Prophage in serogroup B Neisseria meningitidis coding for surface-exposed antigens. Infect. Immun. 69, 2580-2588.
Mojica, F.J.M., Díez-Villaseñor, C., García-Martínez, J., and Almendros, C. (2009). Short motif sequences determine the targets of the prokaryotic CRISPR defence system. Microbiology 155, 733-740.
Mojica, F.J.M., Díez-Villaseñor, C., García-Martínez, J., and Soria, E. (2005). Intervening sequences of regularly spaced prokaryotic repeats derive from foreign genetic elements. J. Mol. Evol. 60, 174-182.
Moxon, E.R., and Jansen, V.A.A. (2005). Phage variation: understanding the behaviour of an accidental pathogen. Trends Microbiol. 13, 563-565.
Palmer, K., and Gilmore, M. (2010). Multidrug-resistant enterococci lack CRISPR-cas. mBio 1, e00227-10.
Peng, J., Yang, L., Yang, F., Yang, J., Yan, Y., Nie, H., Zhang, X., Xiong, Z., Jiang, Y., Cheng, F., et al. (2008). Characterization of ST-4821 complex, a unique Neisseria meningitidis clone. Genomics 91, 78-87.
Pines, O., Yoon, H., and Inouye, M. (1988). Expression of double-stranded-RNA-specific RNase III of Escherichia coli is lethal to Saccharomyces cerevisiae. J. Bact. 170, 2989-2993.
Pourcel, C., Salvignol, G., and Vergnaud, G. (2005). CRISPR elements in Yersinia pestis acquire new repeats by preferential uptake of bacteriophage DNA, and provide additional tools for evolutionary studies. Microbiology 151, 653-663.
Qi, L.S., Larson, M.H., Gilbert, L.A., Doudna, J.A., Weissman, J.S., Arkin, A.P., and Lim, W.A. (2013). Repurposing CRISPR as an RNA-guided platform for sequence-specific control of gene expression. Cell 152, 1173-1183.
Rousseau, C., Gonnet, M., Le Romancer, M., and Nicolas, J. (2009). CRISPRi: a CRISPR interactive database. Bioinformatics 25, 3317-3318.
Rusniok, C., Vallenet, D., Floquet, S., Ewles, H., Mouzé-Soulama, C., Brown, D., Lajus, A., Buchrieser, C., Médigue, C., Glaser, P., et al. (2009). NeMeSys: a biological resource for narrowing the gap between sequence and function in the human pathogen Neisseria meningitidis. Genome Biol. 10, R110.
Sampson, T.R., Saroj, S.D., Llewellyn, A.C., Tzeng, Y.-L., and Weiss, D.S. (2013). A CRISPR/Cas system mediates bacterial innate immune evasion and virulence. Nature, in press [ePub ahead of print (doi:10.1038/nature12048)].
Sapranauskas, R., Gasiunas, G., Fremaux, C., Barrangou, R., Horvath, P., and Siksnys, V. (2011). The Streptococcus thermophilus CRISPR/Cas system provides immunity in Escherichia coli. Nucleic Acids Res. 39, 9275-9282.
Sashital, D.G., Wiedenheft, B., and Doudna, J.A. (2012). Mechanism of foreign DNA selection in a bacterial adaptive immune system. Mol. Cell 46, 606-615.
Semenova, E., Jore, M.M., Datsenko, K.A., Semenova, A., Westra, E.R., Wanner, B., van der Oost, J., Brouns, S.J.J., and Severinov, K. (2011). Interference by clustered regularly interspaced short palindromic repeat (CRISPR) RNA is governed by a seed sequence. Proc. Natl. Acad. Sci. U.S.A. 108, 10098-10103.
Sharma, C.M., Hoffmann, S., Darfeuille, F., Reignier, J., Findeiss, S., Sittka, A., Chabas, S., Reiche, K., Hackermüller, J., Reinhardt, R., et al. (2010). The primary transcriptome of the major human pathogen Helicobacter pylori. Nature 464, 250-255.
Sittka, A., Lucchini, S., Papenfort, K., Sharma, C.M., Rolle, K., Binnewies, T.T., Hinton, J.C.D., and Vogel, J. (2008). Deep sequencing analysis of small noncoding RNA and mRNA targets of the global post-transcriptional regulator, Hfq. PLoS Genet. 4, e1000163.
Sittka, A., Pfeiffer, V., Tedin, K., and Vogel, J. (2007). The RNA chaperone Hfq is essential for the virulence of Salmonella typhimurium. Molec. Microbiol. 63, 193-217.
Skaar, E.P., Lecuyer, B., Lenich, A.G., Lazio, M.P., Perkins-Balding, D., Seifert, H.S., and Karls, A.C. (2005). Analysis of the Piv recombinase-related gene family of Neisseria gonorrhoeae. J. Bact. 187, 1276-1286
Smith, J.M., Smith, N.H., O'Rourke, M., and Spratt, B.G. (1993). How clonal are bacteria? Proc. Natl. Acad. Sci. USA 90, 4384-4388.
Stephens, D.S., Greenwood, B., and Brandtzaeg, P. (2007). Epidemic meningitis, meningococcaemia, and Neisseria meningitidis. Lancet 369, 2196-2210.
Stern, A., Keren, L., Wurtzel, O., Amitai, G., and Sorek, R. (2010). Self-targeting by CRISPR: gene regulation or autoimmunity? Trends Genet. 26, 335-340.
Terns, M.P., and Terns, R.M. (2011). CRISPR-based adaptive immune systems. Curr. Opin. Microbiol. 14, 321-327.
Tobiason, D.M., and Seifert, H.S. (2010). Genomic content of Neisseria species. J. Bact. 192, 2160-2168.
van Passel, M.W.J., Van der Ende, A., and Bart, A. (2006). Plasmid diversity in neisseriae. Infect. Immun. 74, 4892-4899.
Vázquez, J.A., de la Fuente, L., Berron, S., O'Rourke, M., Smith, N.H., Zhou, J., and Spratt, B.G. (1993). Ecological separation and genetic isolation of Neisseria gonorrhoeae and Neisseria meningitidis. Curr. Biol. 3, 567-572.
Westra, E.R., Van Erp, P.B.G., Künne, T., Wong, S.P., Staals, R.H.J., Seegers, C.L.C., Bollen, S., Jore, M.M., Semenova, E., Severinov, K., et al. (2012). CRISPR immunity relies on the consecutive binding and degradation of negatively supercoiled invader DNA by Cascade and Cas3. Mol. Cell 46, 595-605.
Wiedenheft, B., Van Duijn, E., Bultema, J.B., Bultema, J., Waghmare, S.P., Waghmare, S., Zhou, K., Barendregt, A., Westphal, W., Heck, A.J.R., et al. (2011). RNA-guided complex from a bacterial immune system enhances target recognition through seed sequence interactions. Proc. Natl. Acad. Sci. USA 108, 10092-10097.
Wiedenheft, B., Sternberg, S.H., and Doudna, J.A. (2012). RNA-guided genetic silencing systems in bacteria and archaea. Nature 482, 331-338.
Yosef, I., Goren, M.G., and Qimron, U. (2012). Proteins and DANN elements essential for the CRISPR adaptation process in Escherichia coli. Nucleic Acids Res. 40, 5569-5576.
Zhang, J., Rouillon, C., Kerou, M., Reeks, J., Brugger, K., Graham, S., Reimann, J., Cannone, G., Liu, H., Albers, S.-V., et al. (2012). Structure and mechanism of the CMR complex for CRISPR-mediated antiviral immunity. Mol. Cell 45, 303-313.

### Example 2

Reference is made to Hou et al., "Efficient genome engineering in human pluripotent stem cells using Cas9 from Neisseria meningitidis, PNAS, vol. 110, no. 39, pp 15644-15649, September 24, 2013.

### Abstract

Genome engineering in human pluripotent stem cells holds great promise for biomedical research and regenerative medicine. Recently, an RNA-guided, DNA-cleaving interference pathway from bacteria [the Type II clustered, regularly interspaced, short palindromic repeats (CRISPR)-CRISPR-associated (Cas) pathway] has been adapted for use in eukaryotic cells, greatly facilitating genome editing. Only two CRISPR-Cas systems (from *Streptococcus pyogenes* and *Streptococcus thermophilus*)*,* each with their own distinct targeting requirements and limitations, have been developed for genome editing thus far. Furthermore, limited information exists about homology-directed repair (HDR)-mediated gene targeting using long donor DNA templates in human pluripotent stem cells (hPSCs) with these systems. Here, using a distinct CRISPR-Cas system from *Neisseria meningitidis,* we demonstrate efficient targeting of an endogenous gene in three hPSC lines using HDR. The Cas9 RNA-guided endonuclease from *N. meningitidis* (NmCas9) recognizes a 5'-NNNNGATT-3' protospacer adjacent motif (PAM) different from those recognized by Cas9 proteins from *S. pyogenes* and *S. thermophilus* (SpCas9 and StCas9, respectively). Similar to SpCas9, NmCas9 is able to use a single-guide RNA (sgRNA) to direct its activity. Due to its distinct PAM, the *N. meningitidis* CRISPR-Cas machinery increases the sequence contexts amenable to RNA-directed genome editing.

### Introduction

Human pluripotent stem cells (hPSCs) can proliferate indefinitely while maintaining the potential to give rise to virtually all human cell types (1). They are therefore invaluable for regenerative medicine, drug screening, and biomedical research. However, to realize the full potential of hPSCs, it will be necessary to manipulate their genomes in a precise, efficient manner. Historically, gene targeting in hPSCs has been extremely difficult (2). The development of zinc-finger nucleases (ZFNs) and transcription activator-like endonucleases (TALENs) (reviewed in refs. (3) and (4)) has facilitated gene targeting in hPSCs (5-7). Nonetheless, they require the design, expression, and validation of a new pair of proteins for every targeted locus, rendering both of these platforms time-consuming and labor-intensive (8-10).

Clustered, regularly interspaced, short palindromic repeat (CRISPR) loci, along with CRISPR-associated (*cas*) genes, underlie an adaptive immune system of bacteria and archaea that defends against bacteriophages (11) and limits horizontal gene transfer (12-14). "Protospacer" sequences from invading nucleic acids are incorporated as "spacers" within CRISPRs, conferring immunity and providing a genomic memory of past invasions. CRISPR-Cas systems have been classified into three types (Types I, II and III) and numerous subtypes (15). All use short CRISPR RNAs (crRNAs) (16, 17) to specify genetic interference via the destruction of invading nucleic acids (18). The target nucleic acids are recognized by crRNA Watson-Crick pairing. Importantly, most CRISPR-Cas subtypes target DNA directly (13, 19, 20), suggesting the possibility of engineered, RNA-directed gene targeting/editing systems. The use of RNA guides for gene targeting would confer many advantages over ZFNs and TALENs, especially by obviating the need for repeated protein design/optimization. Recently, this vision has become a reality (21-31).

Type II CRISPR-Cas systems are noteworthy in that the essential targeting activities - crRNA binding, target DNA binding, R-loop formation, and double-stranded DNA cleavage - are all executed by a single polypeptide, Cas9 (32-35). In addition to crRNA and Cas9, an additional RNA, trans-acting CRISPR RNA (tracrRNA), is essential for interference in bacteria (14, 32, 36) and *in vitro* (34, 36). The tracrRNA is partially complementary to pre-crRNA repeats, leading to the formation of duplexes that are cleaved by the host factor ribonuclease III (RNase III) (32). The Type II crRNA maturation pathway was originally characterized in strains of *Streptococcus pyogenes* (32) and *Streptococcus thermophilus* (35, 36), and RNase III-catalyzed pre-crRNA processing is essential for interference in both native systems. Recent studies of a Type II CRISPR-Cas locus from *Neisseria meningitidis* revealed an intrinsically RNase III- and processing-independent system, which nonetheless requires tracrRNA (14). Importantly, crRNA-directed DNA cleavage was reconstituted *in vitro* with recombinant *S. pyogenes* Cas9 (SpCas9) (34) or *S. thermophilus* Cas9 (StCas9) (33, 36). The SpCas9 *in vitro* system enabled the development of fused crRNA-tracrRNA chimeras called single-guide RNAs (sgRNAs) that bypass processing (34). Subsequent development of eukaryotic genome editing applications has focused on sgRNAs (21-30), though separately encoded pre-crRNAs and tracrRNAs are also effective (21).

Target cleavage by many CRISPR/Cas systems, including those from Type II, require proximity to a 2-5 nucleotide (nt) sequence called a protospacer adjacent motif (PAM) (37) (38-40). Genome editing applications reported thus far have focused almost exclusively on SpCas9, which has a 5'-NGG-3' PAM. StCas9 (from the CRISPR1 locus of strain LMD-9) has also been used in eukaryotes (21), and that system has a 5'-NNAGAAW-3' PAM (W = A or T). Eukaryotic editing capabilities will benefit from the increased frequency of target sites stemming from the development of additional Cas9s with distinct PAMs.

Targeting by sgRNAs usually relies on either of two approaches. First, double-strand break (DSB) repair by nonhomologous end joining (NHEJ) can be used to generate insertions or deletions (indels) that induce frame shifts. Second, the addition of a homologous repair template can allow Cas9-induced DSBs or nicks to be repaired by homology-directed repair (HDR). The latter strategy is useful for making precise changes such as repairing mutations or inserting transgenes. Most studies thus far have relied on either NHEJ, or on HDR using short DNA fragments or oligos (24-26, 29, 31). Currently there is very limited information available on gene targeting using long DNA donor templates in hPSCs (23).

Here, we report the development of *N. meningitidis* Cas9 (NmCas9) (14) as a genome editing platform, and its application to high-efficiency targeting of an endogenous gene in hPSCs. This system uses a 24 nt proto-spacer for targeting and requires a PAM that is different from those of SpCas9 or StCas9. We have achieved -60% targeting efficiency with two human embryonic stem cell (hESC) lines and one human induced pluripotent stem (iPS) cell line. Our work demonstrates the feasibility of using the *N. meningitidis* CRISPR/Cas system in genome editing in hPSCs using long DNA donor templates. This work also provides an alternative to the *S. pyogenes* and *S. thermophilus* CRISPR-Cas system and expands the genomic contexts that are amenable to RNA-directed genome editing in eukaryotes.

### Results

Functional Expression of NmCas9 in Mammalian Cells. Our recent work has shown that *N. meningitidis* strain 8013 has a functional type II-C CRISPR/Cas system (14), and that Cas9 is the only Cas protein required for interference activity. We set out to test whether this system could be used for efficient gene targeting in hPSCs. We cloned the open reading frame (ORF) from the 3.25 kb *cas9* gene, along with a C-terminal FLAG tag, into a mammalian expression plasmid under the control of an EF1α promoter (Fig. 13A). This NmCas9-containing vector was transfected into 293FT cells and the expression of NmCas9 protein was analyzed by anti-FLAG western blot. As shown in Fig. 13A, full-length NmCas9 was efficiently expressed in 293FT cells. We then assayed the nuclease activity of NmCas9 expressed in mammalian cells by *in vitro* plasmid cleavage. Cell extract was prepared from 293FT cells two days after transfection with the NmCas9-containing vector (the same one as in Fig. 13A). We assembled cleavage reactions using cell extract, various *in vitro*-synthesized small RNAs, and the plasmid ptdTomato pre-linearized by NdeI (Fig. 13B). tdTomato is a fusion of two copies of the dTomato gene, each of which has one consensus PAM sequence (5'-NNNNGATT-3') (Fig. 13B). As shown in Fig. 13C, we achieved efficient plasmid cleavage only in the presence of both tracrRNA and a cognate crRNA (Fig. 13C, lane 3). The pattern of the cleavage products was consistent with two predicted cleavage sites in the PAM-proximal regions (Fig. 13C, right panel). Importantly, a non-cognate crRNA (N), which contains sequences from EGFP, did not direct NmCas9-mediated cleavage (Fig. 13C, lane 4), indicating that the specificity of the NmCas9 nuclease is indeed guided by the spacer-derived sequence in crRNAs. Additionally, plasmid cleavage is deficient when tracrRNA is absent, even in the presence of a cognate crRNA (Fig. 13C, lane 5), suggesting that tracrRNA is necessary for NmCas9 function *in vitro.* This is consistent with the tracrRNA requirement for NmCas9-mediated interference in bacterial cells (14).

Two Cas9 orthologs, SpoCas9 and StCas9, were previously demonstrated to induce blunt double-strand breaks (DSB) in their DNA targets, between the third and fourth nucleotide counting from the PAM-proximal end of protospacers (34) (19, 33). We hypothesized that NmCas9 cleaves the DNA target in a similar way, and we tested this by mapping the NmCas9 cleavage site on ptdTomato by Sanger sequencing. Two cleavage products in Fig. 13C (the 1.5 kb and the 2.5 kb fragments) were gel-extracted and sequenced to identify the NmCas9 cleavage sites on the sense strand and the antisense strand, respectively. As expected, NmCas9 induced a blunt-end DSB between the third and fourth nucleotides counting from the PAM-proximal end of the proto-spacer (Fig. 13D).

NmCas9 Functions in RNA-Directed Gene Disruption in hPSCs. Knowing that NmCas9, without any codon optimization, can be efficiently expressed in mammalian cells and is functional *in vitro,* we next tested its utility in genome editing in hPSCs. We first monitored its localization. We transfected 293FT cells with several NmCas9 constructs with various nuclear localization signal (NLS) arrangements, and analyzed NmCas9 protein localization by either GFP fluorescence or anti-HA immunostaining. NmCas9 with NLSs on both N- and C-termini localized efficiently to the nucleus (Fig. 14C), while NmCas9 constructs with just one NLS did not (Fig. 14A and 14B). In addition, the same NmCas9 construct with two NLSs also localized to the nucleus of hESCs (Fig. 14D). We noticed that in hESCs, NmCas9, without any crRNA/tracrRNA, displayed a punctate pattern similar to the organization of the nucleolus in hESCs. It is not yet clear if this phenomenon is related to the organization of the double NLS on the protein.

To test the genome editing activity of NmCas9, we used an hESC cell reporter line that has a single copy of the tdTomato fluorescent protein gene knocked into the highly expressed DNMT3b locus (H9 DNMT3b-tdTomato), leading to tdTomato fluorescence. If NmCas9 is able to introduce a DSB in the tdTomato sequence in the genome, repair by NHEJ would likely lead to indels that disrupt tdTomato expression. Accordingly, the appearance of tdTomato-negative cells would be predicted to reflect genome-editing activity.

Human ESCs are known to have low transfection efficiencies. To achieve maximum genome-editing efficiency in hPSCs, we assembled expression cassettes of all the necessary components (NmCas9, tracrRNA and crRNA) onto one single plasmid that contains an OriP sequence (Fig. 14E). OriP was reported to increase the transfection efficiency and plasmid stability in hPSCs if co-transfected with an RNA expressing the EBNA protein (41, 42). The encoded tracrRNA and crRNA both corresponded to the mature, processed forms as they exist in *N. meningitidis* cells (14). The resulting all-in-one plasmids were electroporated into H9 DNMT3b-tdTomato cells, and tdTomato fluorescence was monitored by FACS 4-6 days after electroporation. As shown in Fig. 14F, a sub-population (5.4%) of tdTomato negative cells became detectable only when a tdTomato-targeting crRNA was encoded on the plasmid. Importantly, for the control plasmid expressing non-targeting crRNA, only background levels (∼0.1%) of tdTomato negative cells appeared (Fig. 14F), likely due to the low level of spontaneous differentiation in the culture, leading to repression of the DNMT3b promoter. The increased frequency of non-fluorescent cells in the presence of the cognate crRNA suggests successful genome editing by NmCas9.

To confirm that NmCas9 introduced a DSB at the intended genomic site, we FACS-sorted the tdTomato negative population, PCR-amplified the genomic region flanking the predicted cutting site in the 5' copy of dTomato, cloned the resulting PCR fragments and sequenced 22 of the resulting plasmids (selected at random). The sequencing results showed both insertions and deletions in the tdTomato sequence (Fig. 14G, only unique indels are shown) in 95% of the sequenced clones. Most importantly, all of these indels were centered around the NmCas9 cleavage site, indicating that the DSB occurred at the intended position (Fig. 14G).

A Chimeric sgRNA is Effective for Gene Editing in hPSCs. To simplify the NmCas9 genome editing system, we explored the possibility of substituting both crRNA and tracrRNA with a chimeric sgRNA. We fused the 5' end of the 91 nt processed tracrRNA sequence with the 3' end of the 48 nt mature crRNA using a 6 nt linker (Supp. Fig. 13A). This sgRNA was cloned under the control of the U6 promoter and electroporated into the H9 DNMT3b-tdTomato reporter cell line together with a plasmid expressing NmCas9. FACS analysis showed that this sgRNA indeed resulted in tdTomato-negative cells (Fig. 17B) at a level comparable to that achieved by the all-in-one plasmid expressing separate crRNA and tracrRNA (see Fig. 14E). These results indicated that an sgRNA could substitute for separate crRNA and tracrRNA in directing NmCas9-mediated gene editing in hESCs.

Specificity of NmCas9 in hPSCs. We next tested the specificity of NmCas9 in mammalian cells by mutational analysis. We introduced single-nucleotide mutations at every odd-numbered position from the 1^{st} to the 17^{th} nt in the PAM-proximal end [spanning the cleavage site (see Figs. 13D and 14G) and the functionally critical "seed" sequence] of the spacer in the tdTomato-targeting crRNA construct (Fig. 15A, upper panel). We then measured the ability of those constructs to give rise to tdTomato-negative cells in the H9 DNMT3b-tdTomato cell line. As shown in Fig. 15A, mutations at position 1 through 9 led to background levels of tdTomato-negative cells, indicating that mismatches at these positions in the crRNA/target duplex are not tolerated by NmCas9. As for mutations at positions 11, 13, 15, some tdTomato negative cells appeared, but with an efficiency of only 10-25% of that observed with wildtype crRNA (Fig. 15A lower panel). The mismatch at position 17 was -40% as efficient as wildtype. These results imply a crRNA/target specificity comparable to that of the SpCas9 system in mammalian cells (21).

We also investigated PAM sequence requirements for NmCas9 in human ES cells. We designed five crRNAs that use different sequences as the PAM in the tdTomato coding region (Fig. 15B) and then tested their ability to disrupt tdTomato expression in H9 DNMT3b-tdTomato cells. Four of the sites were associated with a PAM that varied from the 5'-NNNNGATT-3' consensus by only a single nt. Only a GCTT variant site was efficiently targeted, while the other four variants were severely deficient (Fig. 15B, lower panel). Our results indicate that an A-to-C mutation at the 2^{nd} nt of the PAM could be tolerated, whereas a G-to-C mutation at the 1^{st} position, T-to-C at the 3^{rd}, and T-to-G at the 4^{th} likely render the PAM variants non-functional. Interestingly, C is the second most frequent residue at the 2^{nd} nt of the PAM in candidate bacterial protospacers (14), suggesting that GCTT might also be a natural PAM.

NmCas9 Increases Gene Targeting Efficiency in hPSCs. We next explored whether NmCas9 can increase gene-targeting efficiency in hPSCs compared to the traditional method in which no DSB was intentionally introduced at the target site. We used a donor DNA template previously used to target the endogenous *POU5F1* (*OCT4*) gene (6) (Fig. 16A), creating a fusion of *OCT4* with *EGFP.* We designed the crRNA using the consensus PAM sequence located ∼84bp downstream of the *OCT4* stop codon (Fig. 16A). Two human ES cell lines, H1 and H9, and one human iPS cell line, iPS005 (43), were used in the experiment. After puromycin selection, we were able to obtain clones for all three cell lines when plasmid expressing the *OCT4*-targeting crRNA was used. Of these clones, -60% were correctly targeted with single insertion events (Table 7), comparable to the efficiency obtained using TALENs in a previous report with the same donor DNA (6). Fluorescent images of the targeted clones revealed the expected nuclear localization of EGFP signal due to the fusion with Oct4 protein (Fig. 16B). Southern blots using a probe outside the targeting vector's homology arm confirmed the correct integration of the donor sequence in the *OCT4* locus (Fig. 16C). Most importantly, the EGFP signals respond to differentiation cues as the endogenous Oct4 would (Fig. 16D). In a control experiment with an all-in-one plasmid expressing a non-targeting crRNA, no puromycin-resistant clones were obtained with the H1 ESC line. Only one puromycin-resistant clone each was obtained from H9 ESCs and iPS005 iPSCs, and neither clone was correctly targeted (Table 7). All of the above results indicated that the CRISPR-Cas system from *N. meningitidis* was able to generate accurately targeted clones in hPSCs with much increased efficiency compared to the traditional method.

### Discussion

Genome Editing by *N. meningitidis* Cas9. In this report, we have successfully used the Type II-C CRISPR-Cas system from *N. meningitidis* to achieve both NHEJ-mediated gene editing and long DNA donor-directed gene targeting of an endogenous locus in hPSCs. The targeting efficiency we obtain with NmCas9 is comparable to that achieved with TALENs. Using the same donor construct, we were able to get ∼60% targeting efficiency in all three different hPSC lines tested (Table 7), whereas the targeting efficiency of a TALEN was 48% in the one hESC line tested (6). A previous report using SpCas9 in human iPSCs achieved a targeting efficiency of 43%, close to what we observed with NmCas9 (6). However, that report only identified seven clones and did not perform further analysis to confirm the correct integration of the donor DNA sequence only at the intended site. Therefore additional work will be needed to compare the efficiency of mammalian gene targeting using these two CRISPR-Cas systems.

CrRNA/Target Mismatch Tolerance by NmCas9 in Mammalian Cells. One potential advantage of NmCas9, relative to SpCas9, is that it might offer better targeting specificity by virtue of its longer crRNA spacer (24 vs. 20 nts) and its longer PAM (14). We chose 24 nt as the crRNA spacer length for NmCas9 because that is the length of the crRNA spacer in *N. meningitidis.* CrRNA-target mismatches distant from the PAM were tolerated to various extents for both NmCas9 (Fig. 15B) and SpCas9 (21) in mammalian cells. However, NmCas9 was more sensitive than SpCas9 to mismatches at the 13^{th}, 15^{th}, and 17^{th} nts (counting from the PAM-proximal end of the proto-spacer). NmCas9 gene editing efficiencies with mismatches at those positions were no higher than 10-40% of those observed with the perfectly matched crRNA (Fig. 15B), whereas with SpCas9, mismatches at equivalent positions retained 60-90% of the non-mismatched efficiency (21).

PAM requirements in mammalian cells. One hallmark of Type II CRISPR-Cas systems is the requirement of a nearby PAM on the target sequence. This sequence varies between different Cas9 orthologs. Among Cas9 proteins validated for mammalian genome editing, PAM functional requirements have been defined for three: those from S. *pyogenes* SF370 (21-23, 32, 34), *S.thermophilus* LMD-9 (the CRISPR1 locus) (19, 21, 38), and *N. meningitidis* 8013 (Fig. 15B) (14). On one hand, the PAM requirement adds a second layer of specificity for gene targeting, beyond that afforded by spacer/protospacer complementarity. For longer PAMs (such as the NmCas9 PAM, 5'-NNNNGATT-3'), the frequency of off-target cutting events should potentially drop significantly compared to SpCas9, which requires a 5'-NGG-3' PAM. On the other hand, longer PAM requirements also constrain the frequency of targetable sites. By developing genome-editing systems using a range of Cas9 proteins with distinct PAM requirements, the genomic regions that can be targeted by CRISPR-Cas editing would expand significantly.

The results in Fig. 15B show that NmCas9 does allow limited deviation from the 5'-NNNNGATT-3' PAM. Having a variable PAM can potentially increase the flexibility during the design of targeting construct. However, it also increases the potential of off-target cleavage. Due to the limited options afforded by the sequence of tdTomato, we only tested one nucleotide substitution in each position of the PAM domain. It is possible that additional nucleotide substitutions will also be tolerated. A detailed mutational analysis will be needed to fully understand the PAM requirements of NmCas9 in mammalian cells.

Editing the Genomes of hPSCs. Compared to two other widely used systems for enhancing gene targeting efficiency (ZFNs and TALENs), the CRISPR-Cas system offers a much simpler and more user-friendly design. For each different genomic locus to be targeted, one only needs to design a small RNA by applying simple Watson-Crick base-pairing rules. This system's ease of use will make gene targeting in hPSCs, once considered a difficult project, a routine lab technique. This simple and high efficiency gene targeting system for hPSC will also have a tremendous impact on personalized regenerative medicine. One concern with using CRISPR/Cas in human genome editing is off-target cleavage. Our work (Fig. 15A) and that of others (21, 44) has shown that the CRISPR/Cas system can tolerate mismatches within the crRNA, especially in the PAM-distal region. This raises concerns that other regions in the genome might be cleaved unintentionally. Indeed, recent work has shown various off-target cleavage rates in the human genome using SpCas9 with different sgRNAs (44). To fully understand this issue, whole-genome sequencing of cells targeted by different Cas9 proteins with different crRNA/sgRNA constructs will be needed. A potential way to get around this problem is to use a nickase, a Cas9 variant in which one nuclease domain is inactivated by a mutation (21, 34), so that off-target cleavage will have a much lower chance of generating unwanted mutations in the genome while HDR will still be stimulated.

### Materials and Methods

Cell Culture. Human ESCs and iPS cells were cultured in E8™ medium (43) on Matrigel-coated tissue culture plates with daily media change at 37°C with 5% CO₂. Cells were split every 4-5 days with 0.5 mM EDTA in 1x PBS. 293FT cells were cultured similarly in DMEM/F12 media supplemented with 10% FBS.

NmCas9 DNA Transfection and *In Vitro* Plasmid Digestion. All transfections with 293FT cells were done using Fugene HD (Promega) following the manufacturer's instructions. Cell lysate was prepared two days after transfection. Plasmid digestion using cell lysate was carried out at 37°C for 1-4 hours in digestion buffer (1x PBS with 10 mM MgCl₂). See supplemental method for a detailed procedure. To map the cleavage site of NmCas9, the digested plasmid DNA was excised from the agarose gel and purified using Gel Extraction Kit (Qiagen). The purified fragments were then sequenced to map the cleavage site.

Gene Editing in hPSCs. All plasmids used in this experiment were purified using the MaxiPrep Kit from Qiagen. Human PSCs were passaged two or three days before the experiments. Immediately before the experiment, hPSCs were individualized by Accutase® treatment, washed once with E8™ medium, and resuspended at densities of 2.5 - 6.2 x 10⁶ cells/ml in E8™ medium with 10 mM HEPES buffer (pH 7.2 - 7.5) (Life Technologies). For electroporation, 400 µl of cell suspension, 15 µg of pSimple-Cas9-Tracr-CrRNA plasmid, 5 µg of EBNA RNA, and (for those experiments involving gene targeting by HDR) 5 µg of linearized DNA template plasmid (Addgene 31939) were mixed in a 4mm cuvette (BioRad) and immediately electroporated with a BioRad Gene Pulser. Electroporation parameters were 250V, 500 µF, and infinite resistance. Cells were then plated into appropriate Matrigel coated culture dishes in E8™ medium supplemented with 10 µM ROCK inhibitor Y-27632. Media was changed the next day to E8™ medium. For those experiments involving gene editing by HDR, puromycin selection was started 4 days after electroporation. Surviving colonies were picked 4 to 6 days after selection and expanded in E8™ medium.

Plasmid Construction. The *cas9* gene from *Neisseria meningitidis* strain 8013 was PCR-amplified and cloned into the pSimpleII plasmid (an OriP containing plasmid) under the control of the EF1α promoter. Nuclear localization signals and HA tag sequences were incorporated via the PCR primers. An *N. meningitidis* BsmBI-crRNA cassette and the *N. meningitidis* tracrRNA, both under the control of U6 RNA polymerase III promoters, were synthesized as gene blocks (Integrated DNA Technologies) and cloned into pSimpleII-Cas9 via blunt end cloning, generating the pSimple-Cas9-Tracr-BsmBI plasmid that includes all elements needed for targeting. To insert specific spacer sequences into the crRNA cassette, synthetic oligonucleotides containing the desired spacer sequences were annealed to generate a duplex with overhangs compatible with those generated by BsmBI digestion of the pSimple-Cas9-Tracr-BsmBI plasmid. The insert was then ligated into the BsmBI-digested plasmid.

NmCas9 DNA Transfection and *In Vitro* Plasmid Digestion. All transfections with 293FT cells were done using Fugene® HD (Promega) following the manufacture's instructions. Roughly 2µg plasmids and 6µl of Fugene HD were used for one well of a 6-well plate. Two days after transfection, 293FT cells expressing NmCas9 were harvested by TrypLE (Life Technologies), washed once in PBS, and then lysed in PBS by sonication. Cellular debris was cleared by centrifugation and the supernatant was used in plasmid digestion assays. For the digestions, lug tdTomato plasmid (Clontech) linearized by *Nde*I (New England Biolabs) was mixed with *in vitro*-transcribed tracrRNA, crRNA and 293FT cell lysate and incubated at 37°C for 1-4 hours in digestion buffer (1x PBS with 10 mM MgCl₂). DNA from the reaction mix was then purified with a PCR clean-up kit (Qiagen) and resolved by agarose gel electrophoresis. To map the cleavage site of NmCas9, the digested plasmid DNA was excised from the agarose gel and purified using Gel Extraction Kit (Qiagen). The purified fragments were then sequenced to map the cleavage site.

*In Vitro* Transcription. Synthetic oligonucleotides (Integrated DNA Technologies) containing the T7 promoter sequence and *N. meningitidis* tracrRNA or crRNA sequences were annealed to generate dsDNA templates for run-off transcription. *In vitro* transcription was done using the MegaScript T7 In Vitro Transcription kit (Ambion) following the manufacture's specifications.

Southern Blots. Genomic DNA of targeted clones is purified using PureGene core kit (Qiagen). 5 µg of genomic DNA was digested with BamHI and then resolved on a 0.8% agarose gel. DIG-labeled DNA probe synthesis, DNA gel transfer, and blot hybridization and visualization were done according to Roche's DIG application manual.

Genome editing using single-guide RNA (sgRNA). A single-guide RNA that targets tdTomato was put under the control of a U6 promoter and cloned into the EcoRV site of pstBlue-1 (Novagen). For electroporation, 7.5 µg of pstBlue-U6-sgRNA, 7.5 µg of pSimpleII-NLS-NmCas9-HA-NLS(s) and 5µg of EBNA RNA was mixed with ∼1 x 10⁶ cells in a 4 mm cuvette (BioRad) and immediately electroporated with a BioRad Gene Pulser. Cells were then plated into appropriate Matrigel coated culture dishes in E8™ medium supplemented with 10 µM ROCK inhibitor Y-27632.\

### References for Example 2

1. Thomson JA, et al. (1998) Embryonic stem cell lines derived from human blastocysts. Science 282(5391):1145-1147.
2. Zwaka TP & Thomson JA (2003) Homologous recombination in human embryonic stem cells. Nat Biotechnol 21(3):319-321.
3. Urnov FD, Rebar EJ, Holmes MC, Zhang HS, & Gregory PD (2010) Genome editing with engineered zinc finger nucleases. Nat Rev Genet 11(9):636-646.
4. Joung JK & Sander JD (2013) TALENs: a widely applicable technology for targeted genome editing. Nat Rev Mol Cell Biol 14(1):49-55.
5. Hockemeyer D, et al. (2009) Efficient targeting of expressed and silent genes in human ESCs and iPSCs using zinc-finger nucleases. Nat Biotechnol 27(9):851-857.
6. Hockemeyer D, et al. (2011) Genetic engineering of human pluripotent cells using TALE nucleases. Nat Biotechnol 29(8):731-734.
7. Zou J, et al. (2009) Gene targeting of a disease-related gene in human induced pluripotent stem and embryonic stem cells. Cell Stem Cell 5(1):97-110.
8. Zhang L, et al. (2000) Synthetic zinc finger transcription factor action at an endogenous chromosomal site. Activation of the human erythropoietin gene. J Biol Chem 275(43):33850-33860.
9. Cermak T, et al. (2011) Efficient design and assembly of custom TALEN and other TAL effector-based constructs for DNA targeting. Nucleic Acids Res 39(12):e82.
10. Porteus MH (2006) Mammalian gene targeting with designed zinc finger nucleases. Mol Ther 13(2):438-446.
11. Barrangou R, et al. (2007) CRISPR provides acquired resistance against viruses in prokaryotes. Science 315(5819):1709-1712.
12. Bikard D, Hatoum-Aslan A, Mucida D, & Marraffini LA (2012) CRISPR interference can prevent natural transformation and virulence acquisition during in vivo bacterial infection. Cell Host Microbe 12(2): 177-186.
13. Marraffini LA & Sontheimer EJ (2008) CRISPR interference limits horizontal gene transfer in staphylococci by targeting DNA. Science 322(5909): 1843-1845.
14. Zhang Y, et al. (2013) Processing-Independent CRISPR RNAs Limit Natural Transformation in Neisseria meningitidis. Mol Cell 50(4):488-503.
15. Makarova KS, et al. (2011) Evolution and classification of the CRISPR-Cas systems. Nat Rev Microbiol 9(6):467-477.
16. Brouns SJ, et al. (2008) Small CRISPR RNAs guide antiviral defense in prokaryotes. Science 321(5891):960-964.
17. Hale C, Kleppe K, Terns RM, & Terns MP (2008) Prokaryotic silencing (psi)RNAs in Pyrococcus furiosus. RNA 14(12):2572-2579.
18. Wiedenheft B, Sternberg SH, & Doudna JA (2012) RNA-guided genetic silencing systems in bacteria and archaea. Nature 482(7385):331-338.
19. Garneau JE, et al. (2010) The CRISPR/Cas bacterial immune system cleaves bacteriophage and plasmid DNA. Nature 468(7320):67-71.
20. Westra ER, et al. (2012) CRISPR immunity relies on the consecutive binding and degradation of negatively supercoiled invader DNA by Cascade and Cas3. Mol Cell 46(5):595-605.
21. Cong L, et al. (2013) Multiplex genome engineering using CRISPR/Cas systems. Science 339(6121):819-823.
22. Jinek M, et al. (2013) RNA-programmed genome editing in human cells. Elife 2:e00471.
23. Mali P, et al. (2013) RNA-guided human genome engineering via Cas9. Science 339(6121):823-826.
24. Wang H, et al. (2013) One-Step Generation of Mice Carrying Mutations in Multiple Genes by CRISPR/Cas-Mediated Genome Engineering. Cell 153(4):910-918.
25. Cho SW, Kim S, Kim JM, & Kim JS (2013) Targeted genome engineering in human cells with the Cas9 RNA-guided endonuclease. Nat Biotechnol 31(3):230-232.
26. Chang N, et al. (2013) Genome editing with RNA-guided Cas9 nuclease in zebrafish embryos. Cell Res 23(4):465-472.
27. DiCarlo JE, et al. (2013) Genome engineering in Saccharomyce cerevisiae using CRISPR-Cas systems. Nucleic Acids Res 41(7):4336-4343.
28. Gratz SJ, et al. (2013) Genome engineering of Drosophila with the CRISPR RNA-guided Cas9 nuclease. Genetics*.*
29. Hwang WY, et al. (2013) Efficient genome editing in zebrafish using a CRISPR-Cas system. Nat Biotechnol 31(3):227-229.
30. Xiao A, et al. (2013) Chromosomal deletions and inversions mediated by TALENs and CRISPR/Cas in zebrafish. Nucleic Acids Res.
31. Ding Q, et al. (2013) Enhanced efficiency of human pluripotent stem cell genome editing through replacing TALENs with CRISPRs. Cell Stem Cell 12(4):393-394.
32. Deltcheva E, et al. (2011) CRISPR RNA maturation by trans-encoded small RNA and host factor RNase III. Nature 471(7340):602-607.
33. Gasiunas G, Barrangou R, Horvath P, & Siksnys V (2012) Cas9-crRNA ribonucleoprotein complex mediates specific DNA cleavage for adaptive immunity in bacteria. Proc Natl Acad Sci USA 109(39):E2579-2586.
34. Jinek M, et al. (2012) A programmable dual-RNA-guided DNA endonuclease in adaptive bacterial immunity. Science 337(6096):816-821.
35. Sapranauskas R, et al. (2011) The Streptococcus thermophilus CRISPR/Cas system provides immunity in Escherichia coli. Nucleic Acids Res 39(21):9275-9282.
36. Karvelis T, et al. (2013) crRNA and tracrRNA guide Cas9-mediated DNA interference in Streptococcus thermophilus. RNA Biol 10(5).
37. Shah SA, Erdmann S, Mojica FJ, & Garrett RA (2013) Protospacer recognition motifs: Mixed identities and functional diversity. RNA Biol 10(5).
38. Deveau H, et al. (2008) Phage response to CRISPR-encoded resistance in Streptococcus thermophilus. J Bacteriol 190(4):1390-1400.
39. Horvath P, et al. (2008) Diversity, activity, and evolution of CRISPR loci in Streptococcus thermophilus. J Bacteriol 190(4):1401-1412.
40. Mojica FJ, Diez-Villasenor C, Garcia-Martinez J, & Almendros C (2009) Short motif sequences determine the targets of the prokaryotic CRISPR defence system. Microbiology 155(Pt 3):733-740.
41. Kameda T, Smuga-Otto K, & Thomson JA (2006) A severe de novo methylation of episomal vectors by human ES cells. Biochem Biophys Res Commun 349(4): 1269-1277.
42. Ren C, et al. (2006) Establishment and applications of epstein-barr virus-based episomal vectors in human embryonic stem cells. Stem Cells 24(5): 1338-1347.
43. Chen G, et al. (2011) Chemically defined conditions for human iPSC derivation and culture. Nat Methods 8(5):424-429.
44. Fu Y, et al. (2013) High-frequency off-target mutagenesis induced by CRISPR-Cas nucleases in human cells. Nat Biotechnol.

### Example 3

Reference is made to Figures 18-22 and the Brief Descriptions provided above. In Figure 18, NmCas9 D16A is shown to function as a nickase in human pluripotent stem cells. Human ES cells expressing both TdTomato and EGFP were transfected with plasmids encoding SpCas9 D10A, NmCas9 D16A or both, along with their respective single guide RNAs (sgRNAs) that target TdTomato coding sequence. Sequences targeted by the sgRNAs are as indicated in Figure 18. Cellular TdTomato fluorescence was analyzed by flow cytometer 5 days after transfection. Significant amount (0.94%) of TdTomato negative cells (indicated by arrow in Figure 18) becomes detectable only in the presence of both SpCas9 D10A and NmCas9 D16A. This suggests that NmCas9 D16A is a functional nickase in human ES cells, and when paired with another properly positioned SpCas9 D10A nickase, could induce DSBs (result in 31nt 5' overhangs) and NHEJ.

Figure 19 demonstrates NmCas9 mediated homology-directed repair using either sense or antisense ssODN. A Human ES cell line that expresses both EGFP and a single mutated (deficient, 9nt deleted) copy of Tomato fluorescent protein gene was transfected with plasmids expressing NmCas9 and gRNA targeting the mutated region of tomato gene, with or without 100pmole of 160nt ssODN as repair template. The ssODNs corresponded to wild type tomato sequence, and were centered around the mutated region. Cellular fluorescence was analyzed by flow cytometry 5 days after transfection. Tomato fluorescence positive cells become detectable (0.3%-0.5%) only when sense or antisense ssODN was co-transfected with NmCas9 and gRNA.

Figure 20 illustrates that NmCas9 can be delivered in the form of mRNA instead of plasmid DNA. Human ES cells expressing both TdTomato and EGFP were transfected with NmCas9 mRNA or an NmCas9 expressing plasmid, together with a plasmid expressing sgRNA that targets tomato gene. Cellular fluorescence was analyzed by flow cytometry 5 days after transfection. The results in Figure 20 shown that the Tomato fluorescence negative cell population, which indicates cleavage of the TdTomato gene, arises when NmCas9 is transfected in the forms of either plasmid (10.8%) or mRNA (23.4%). The Nm Cas9 mRNA used in this study is 5' capped and has a 3' polyA tail. Roughly 30ug of NmCas9 mRNA was transfected.

**Table 1. Bacterial species with predicted Type II-C CRISPR/Cas systems harboring repeats with extended -10 boxes**

| STRAIN CRISPR_id | START | END | #SPACER | DR consensus -10box **[tgnTAAAAT]** | DR length |
|---|---|---|---|---|---|
| *Helicobacter mustelae* 12198^{a} | | | | | |
| NC_013949_1 | 24706 | 25400 | 10 | GTTTTAGCCACTTCATAAATATGTTTA**TG**C**TAAAAT** | 36 |

| *Campylobacter jejuni subsp- jejuni* NCTC 11168^{a,c} | | | | | |
|---|---|---|---|---|---|
| NC_002163_2 | 1455125 | 1455424 | 04 | GTTTTAGTCCCTTTTTAAATTTCTTTA**TG**G**TAAAAT** | 36 |

| *Neisseria meningitidis* Z2491^{a,c} | | | | | |
|---|---|---|---|---|---|
| NC_003116_10 | 608413 | 609504 | 16 | GTTGTAGCTCCCTTTCTCATTTCGCAG**TG**C**TACAAT** | 36 |

| *Ilyobacter polytropus* DSM 2926^{a} | | | | | |
|---|---|---|---|---|---|
| NC_014633_2 | 744582 | 745934 | 20 | GTTGTACTTCCCTAATTATTTTAGCTA**TG**T**TACAAT** | 36 |

| *Pasteurella multocida subsp- multocida* str-Pm70^{a} | | | | | |
|---|---|---|---|---|---|
| NC_002663_3 | 1322127 | 1322492 | 05 | GTTGTAGTTCCCTCTCTCATTTCGCAG**TG**C**TACAAT** | 36 |

| *Clostridium cellulolyticum* H10^{a} | | | | | |
|---|---|---|---|---|---|
| NC_011898_3 | 3652357 | 3652923 | 08 | GTTATAGCTCCAATTCAGGCTCCGATA**TG**C**TATAAT** | 36 |

| *Rhodopseudomonas palustris* BisB18^{a} | | | | | |
|---|---|---|---|---|---|
| NC_007925_4 | 4995446 | 4996735 | 19 | GCCGTGGCTTCCCTACCGATTTCCCCG**TG**G**TAGGCT** | 36 |

| *Azospirillum sp-* B510^{a} | | | | | |
|---|---|---|---|---|---|
| NC_013854_7 | 3034463 | 3034649 | 02 | GCTTCAATGAGGCCCAAGCATTTCTGCC**TG**G**GAAGAC** | 36 |
| NC_013854_8 | 3035919 | 3038413 | 33 | GCTTCAATGAGGCCCAAGCATTTCTGCC**TG**G**GAAGAC** | 36 |

| *Candidatus Puniceispirillum marinum* IMCC1322^{a} | | | | | |
|---|---|---|---|---|---|
| NC_014010_1 | 40 | 1792 | 26 | GTTGCTCTAGGCTCTCAATCACCAGAG**TG**C**TATACT** | 36 |

| *Parvibaculum lavamentivorans* DS-1^{b} | | | | | |
|---|---|---|---|---|---|
| tm_1_Crispr_1 | 101247 | 104452 | 48 | GCTGCGGATTGCGGCCGTCTCTCGATT**TG**C**TAC**T**CT** | 36 |

| | | | | | |
|---|---|---|---|---|---|
| ^{#}Type II - C CRISPR/cas prediction ^{a} Species with CRISPRdb entry (http://crispr.u-psud.fr/) ^{b}Species without CRISPRdb entry but available genome sequences to predict CRISPR repeats ^{c}experimentally confirmed extended -10 box | | | | | |

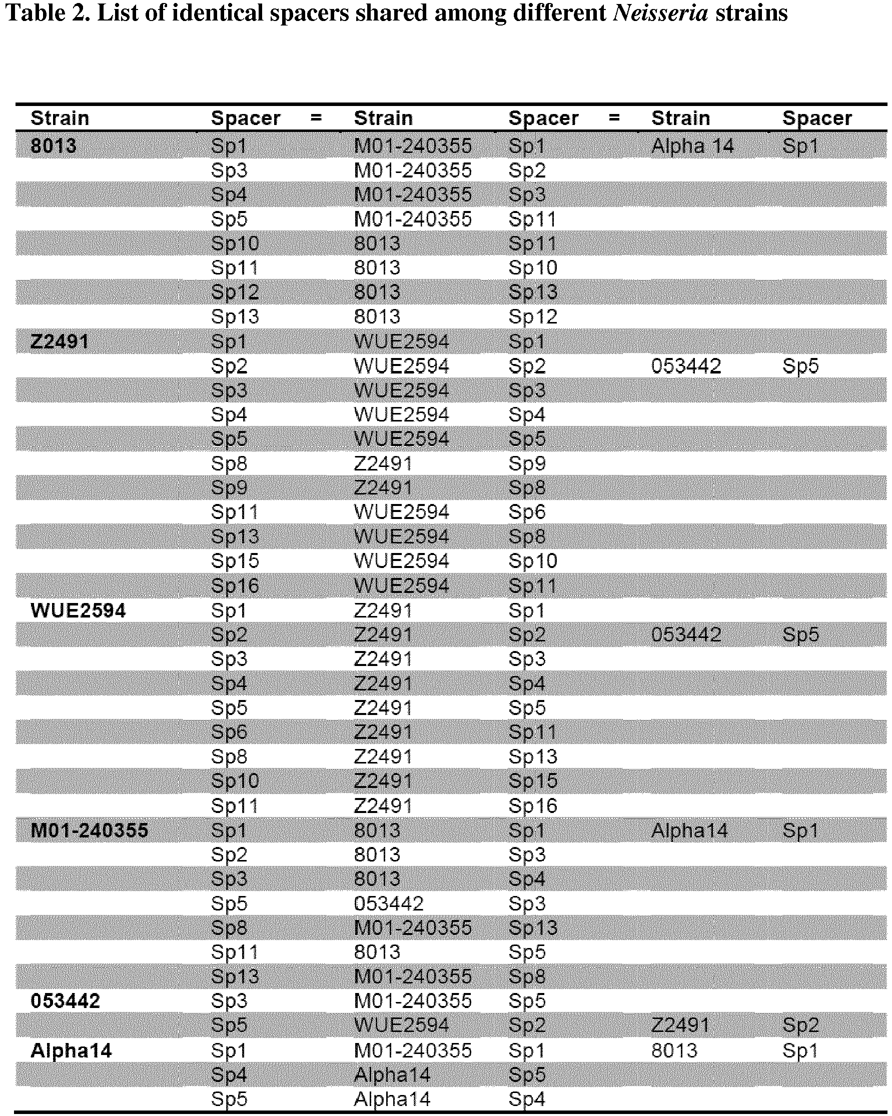

**Table 3. List of Neisseria repeat variants**

| | | |
|---|---|---|
| Consensus Repeat Sequence | | GTTGTAGCTCCCTTTCTCATTTCGCAGTGCTACAAT |

| Strain name | Repeat number | Variant Repeat Sequences* |
|---|---|---|
| *N.m.* 8013 | R1 | GTTGTAGC**G**CCC**A**TTCTCATTTCGCAGTGCTACAAT |
| *N.m.* Z2491 | R1 | GTTGTAGC**G**CCC**A**TTCTCATTTCGCAGTGCTACAAT |
| *N.m.* WUE2594 | R1 | GTTGTAGC**G**CCC**A**TTCTCATTTCGCAGTGCTACAAT |
| *N.m* M01-240335 | R1 | GTTGTAGC**G**CCC**A**TTCTCATTTCGCAGTGCTACAAT |
| | R15 | GTTGTAGCTCCCTTTCTCATTTCGCAGT**A**CTACAAT |
| *N.m.* Alpha14 | R1 | GTTGTAGC**G**CCC**A**TTCTCATTTCGCAGTGCTACAAT |
| | R4 | GTTGTAGCTCCCTTTCTCAT**AAT** |
| *N.m.* 053442 | R1 | GTTGTAGC**G**CCC**A**TTCTCATTTCGCAGTGCTACAAT |
| | R2 | GTTGTAGCTCCC**A**TTCTCATTTCGCAGTGCTACAAT |
| | R8, 9, 10 | GTTGTAGCTCCCTTTCTC**C**TTTCGCAGTGCTACAAT |
| *N.l.* 020-06 | None | |
| * Nucleotides differing from consensus repeat sequences are underlined and in bold. | | |
| *N.m., Neisseria meningitidis; N.l., Neisseria lactamica.* | | |

**Table 4. Prophage-related potential natural targets for Neisseria CRISPRs**

| **Phage-like sequences** | **References** | **Target locus_tag** | **Annotations (NCBI & NemeSys)** | **Targeted by Neisseria Strain name _Spacer number** |
|---|---|---|---|---|
| Meningococcal disease-associated (MDA) island in *N. meningitidis* Z2491 (*NMA1792-1800*), an 8kb filamentous prophage associated with hyperinvasive isolates. | (Bille et al., 2005) | *NMA1792* | putative phage replication initiation factor | WUE2594_sp21 |
| | | *NMA1799* | putative zonular occludens toxin-like protein | WUE2594_sp12 |
| | | *NMA1800* | putative pilin gene-inverting protein/transposase | WUE2594_sp18 |
| Nf (Neisseria filamentous) prophages in *N*. *meningitidis* MC58 | (Kawai et al., 2005; Joseph et al., 2011) | *NMB1543* | putative phage replication initiation factor | WUE2594_sp21 |
| (*NMB1542-1552*) | | *NMB1544* | conserved hypothetical protein | WUE2594_sp19* |
| (*NMB1625-1635*) | | *NMB1552* | putative pilin gene-inverting protein (PivML) | WUE2594_sp13 |
| | | | | WUE2594_sp18 |
| | | *NMB1625* | putative pilin gene-inverting protein (PivML) | WUE2594_sp13 |
| | | | | WUE2594_sp18 |
| | | *NMB1628* | Putative TspB protein | 8013_sp8* |
| | | *NMB1633* | conserved hypothetical protein | WUE2594_sp19 |
| Nf2 prophages in *N*. *meningitidis* | (Kawai et al., 2005; Joseph et al., 2011) | *NMB1749* | putative zonular occludens toxin-like protein | WUE2594_sp7 |
| MC58 | | | | 053442_sp8 |
| (*NMB1742-1750*) | | | | 053442_sp9 |
| | | | | 8013_sp17 |
| | | *NMB1750* | putative pilin gene-inverting protein (PivML) | 053442_sp7 |
| | | | | WUE2594_sp17 |
| | | Intergenic | Between *NMB1749* and *NMB1750* | Z2491_sp3 = WUE2594_sp3 |
| IS*Ngo2*s and nearby degenerate Nf prophages in *N*. *gonorrhoaea* FA1090: | (Kawai et al., 2005; Skaar et al., 2005) | *MGO1137* | invertase related gene 2 (*irg2*), putative phage associated protein | Z2491_sp2*= WUE2594_sp2* =053442_sp5* M01-240355_sp16 |
| (Nf4-G4: *NGO1137-NGO1147*Nf4-G3: *NGO1164-NGO1170* Nf4-G5: Nf4-G6: *NGO1641-NGO1645*) | | *NGO1138* | putative zonular occludens toxin-like, phage associated protein | 053442_sp8* |
| | | Intergenic | Between *NGO1137* and *NGO1138* | 8013_sp9 |
| | | *NGO1164* | invertase related gene 3 (*irg3*). putative phage associated protein | Z2491_sp2* = WUE2594_sp2* =053442_sp5* M01-240355_sp16 |
| | | *NGO1165* | putative zonular occludens toxin-like, phage associated protein | 053442_sp8* |
| | | Intergenic | Between *NGO1164* and *NGO1165* | 8013_ sp9 |
| | | *NGO1262* | invertase related gene 5 (*irg5*), putative phage associated protein | Z2491_sp2* = WUE2594_sp2* =053442_sp5* M01-240355_sp16 |
| | | *NGO1263* | putative zonular occludens toxin-like, phage associated protein | 053442_sp8* |
| | | Intergenic | Between *NGO1262* and *NGO1263* | 8013_sp9 |
| | | *NGO1641* | invertase related gene 6 (*irg6*), putative phage associated protein | Z2491_sp2* = WUE2594_sp2* =053442_sp5* M01-240355_sp16 |
| | | *NGO1643* | putative zonular occludens toxin-like, phage associated protein | 053442_sp8* |
| | | Intergenic | Between *NGO1641* and *NGO1643* | 8013_sp9 |
| ISN*go2*s/S*Ngo3*s with partially deleted Nf prophages in *N*.*gonorrhoaea* FA1090 | (Kawai et al., 2005; Skaar et al., 2005) | *NGO0773* | putative invertase related gene 1 (*irg1*) | M01-240355_sp16 |
| (Nf4-G1: *NGO0773*) (Nf4-G4: *NGO1200*) (Nf4-G8: *NGO1703*) | | *NGO1200* | putative invertase related gene 4 (*irg4*) | M01-240355_sp16 |
| | | *NGO1703* | putative invertase related gene 8 (*irg8*) | M01-240355_sp16 |
| Nf1 prophages in *N*. *meningitidis* FAM18 | (Kawai et al., 2005) | *NMC0022* | putative transposase, pilin gene-inverting protein (PivML) | WUE2594_sp18 |
| (Nf1-C1: *NMC0022-0031*) (Nf1-C2: *NMC0277-0288*) (Nf1-C3: *NMC1709-1718*) (Nf1-C4: *NMC1861-1869*) | | *NMC0023* | putative zonular occludens toxin-like protein | WUE2594_sp12 |
| | | *NMC0030* | conserved hypothetical protein | WUE2594_sp19 |
| | | *NMC0278* | conserved hypothetical protein | WUE2594_sp19 |
| | | *NMC0285* | putative zonular occludens toxin-like protein | WUE2594_sp12 |
| | | *NMC0286* | putative invertase / transposase, putative pilin gene-inverting protein (PivML) | WUE2594_sp18 |
| | | *NMC1710* | conserved hypothetical protein | WUE2594_sp19 |
| | | *NMC1714* | conserved hypothetical protein | WUE2594_sp20* |
| | | *NMC1717* | putative zonular occludens toxin-like protein | WUE2594_sp12 |
| | | *NMC1718* | putative transposase, putative pilin gene-inverting protein (PivML) | WUE2594_sp18 |
| | | *NMC1862* | conserved hypothetical protein | WUE2594_sp19 |
| | | *NMC1864* | hypothetical integral membrane protein | Z2491_sp15 =WU E2594_sp10 |
| | | *NMC1868* | putative zonular occludens toxin-like protein | WUE2594_sp12 |
| | | *NMC1869* | putative invertase / transposase, pilin gene-inverting protein (PivML) | WUE2594_sp18 |
| Meningococcal disease associated (MDA) island-like in *N*. *meningitidis* 053442 | (Peng et al., 2008) | *NMCC_0148* | putative invertase/transposase | WUE2594_sp13 |
| | | | | WUE2594_sp18 |
| (*NMCC_0148* to *NMCC_0156)* | | *NMCC_0149* | conserved hypothetical protein | WUE2594_sp12 |
| | | *NMCC_0153* | conserved hypothetical protein | Z2491_sp15 =WUE2594_sp10 |
| | | *NMCC_0156* | conserved hypothetical protein | WUE2594_sp21 |
| Mu-like prophages in *N*. *meningitidis* MC58 MuMenB/NeisMu1: (*NMB1078-1121*, 35kb) NeisMu2: (*NMB0985-0991,* 4kb) NMB1: (*NMB1002-1007,* 2kb) | (Braid et al., 2004)(Masi gnani et al., 2001) | None | N/A | None. |
| Mu-like prophages in *N*. *meningitidis* Z2491 Pnm1 (*NMA1821-NMA1884,* 39k) Pnm2 (*NMA1281-NMA1330,* 29kb) Pnm3 *(NMA1185-NMA1199,* 7kb) Pnm4/5 (*NMA1208-NMA1231,* 12kb) | (Masignani et al., 2001; Braid et al., 2004) | None | N/A | None |
| *Only perfect matches throughout the 30-nt spacers, or 1 mismatch within the 5'-terminal 10 nts, were considered "potential targets" and listed in this table. Protospacers with a single mismatch are denoted with an asterisk* (*). | | | | |

**Table 5. Transformation frequencies reported in this study**

| **Donor DNA** | **Recipient Strain (*N. meningitidis* 8013)** | **Antibiotic Selection^{(a)}** | **Transformation Frequencies** | |
|---|---|---|---|---|
| | | | **Mean^{(b)}** | **SEM^{(b)}** |
| **Related to** **Figure 4B** | | | | |
| pGCC2 empty | wt | Erythromycin | 3.9 X 10⁻⁶ | 1.1 X 10⁻⁷ |
| pGCC2-protospacer 1 | wt | Erythromycin | 2.5 X 10⁻⁶ | 89 X 10⁻⁸ |
| pGCC2-protospacer 8 | wt | Erythromycin | 0 | 0 |
| pGCC2-protospacer 9 | wt | Erythromycin | 0 | 0 |
| pGCC2-protospacer 16 | wt | Erythromycin | 2.2 X 10⁻⁶ | 25 X 10⁻⁷ |
| pGCC2-protospacer 17 | wt | Erythromycin | 0 | 0 |
| pGCC2-protospacer 18 | wt | Erythromycin | 2.5 X 10⁻⁶ | 51 X 10⁻⁷ |
| pGCC2-protospacer 23 | wt | Erythromycin | 37 X 10⁻⁶ | 1.6 X 10⁻⁶ |
| pGCC2-protospacer 25 | wt | Erythromycin | 0 | 0 |
| No DNA | wt | Erythromycin | 0 | 0 |

| **Related to** **Figure 4C** | | | | |
|---|---|---|---|---|
| pGCC2 empty | wt | Erythromycin | 3.8 X 10⁻⁶ | 15 X 10⁻⁷ |
| pGCC2-Ps9wildtype | wt | Erythromycin | 0 | 0 |
| pGCC2-Ps9Mutant1 | wt | Erythromycin | 0 | 0 |
| pGCC2-Ps9Mutant2 | wt | Erythromycin | 0 | 0 |
| pGCC2-Ps9Mutant3 | wt | Erythromycin | 1.6 X 10⁻⁶ | 5.3 X 10⁻⁷ |
| pGCC2-Ps9Mutant4 | wt | Erythromycin | 0 | 0 |
| pGCC2-Ps9Mutant5 | wt | Erythromycin | 1.2 X 10⁻⁶ | 2.4 X 10⁻⁷ |
| pGCC2-Ps9Mutant6 | wt | Erythromycin | 0 | 0 |
| pGCC2-Ps9Mutant7 | wt | Erythromycin | 0 | 0 |
| pGCC2-Ps9Mutant8 | wt | Erythromycin | 0 | 0 |
| No DNA | wt | Erythromycin | 0 | 0 |

| **Related to** **Figure 5B** | | | | |
|---|---|---|---|---|
| PYZEJS040 | wt | Chloramphenicol | 6.0 X 10⁻⁶ | 1.6 X 10⁻⁶ |
| PYZEJS040-protospacer25 | wt | Chloramphenicol | 0 | 0 |
| PYZEJS040 | *cas9::Tn* | Chloramphenicol | 3.8 X 10⁻⁵ | 1.9 x 10⁻⁵ |
| PYZEJS040-protospacer25 | *cas9::Tn* | Chloramphenicol | 2.9 X 10⁻⁵ | 4.4 X 10⁻⁶ |
| PYZEJS040 | *cas9::Tn* + *cas9* wt | Chloramphenicol | 1.1 X 10⁻⁵ | 4.9 X 10⁻⁶ |
| PYZEJS040-protospacer25 | *cas9::Tn* + *cas9wt* | Chloramphenicol | 0 | 0 |
| PYZEJS040 | *cas9::Tn* + *cas9* D16A | Chloramphenicol | 5.2 X 10⁻⁵ | 1.4 X 10⁻⁵ |
| PYZEJS040-protospacer25 | *cas9::Tn* + *cas9* D16A | Chloramphenicol | 2.5 X 10⁻⁵ | 7.7 X 10⁻⁶ |
| PYZEJS040 | *cas9::Tn* + *cas9* H588A | Chloramphenicol | 2.0 X 10⁻⁵ | 2.9 X 10⁻⁶ |
| PYZEJS040-protospacer25 | *cas9::Tn* + *cas9* H588A | Chloramphenicol | 4.0 X 10⁻⁵ | 1.7 X 10⁻⁵ |
| PYZEJS040 | *cas9::Tn* + empty | Chloramphenicol | 6.8 X 10⁻⁵ | 2.5 X 10⁻⁵ |
| PYZEJS040-protospacer25 | *cas9::Tn* + empty | Chloramphenicol | 6.2 X 10⁻⁵ | 1.2 X 10⁻⁵ |
| PYZEJS040 | Δ*cas9* | Chloramphenicol | 9.5 X 10⁻⁶ | 1.6 X 10⁻⁶ |
| PYZEJS040-protospacer25 | Δ*cas9* | Chloramphenicol | 7.2 X 10⁻⁶ | 2.6 X 10⁻⁶ |
| PYZEJS040 | Δ*cas9* + *cas9* wt | Chloramphenicol | 1.9 X 10⁻⁵ | 6.0 X 10⁻⁶ |
| PYZEJS040-protospacer25 | Δ*cas9* + *cas9* wt | Chloramphenicol | 0 | 0 |
| PYZEJS040 | Δ*cas9* + empty | Chloramphenicol | 90 X 10⁻⁶ | 5.7 X 10⁻⁷ |
| PYZEJS040-protospacer25 | Δ*cas9* + empty | Chloramphenicol | 8.9 X 10⁻⁶ | 3.1 X 10⁻⁶ |
| PYZEJS040 | *cas1::Tn* | Chloramphenicol | 3.8 X 10⁻⁵ | 1.3 X 10⁻⁵ |
| PYZEJS040-protospacer25 | *cas1::Tn* | Chloramphenicol | 0 | 0 |
| PYZEJS040 | *cas2::Tn* | Chloramphenicol | 5.0 X 10⁻⁶ | 1.7 X 10⁻⁶ |
| PYZEJS040-protospacer25 | *cas2::Tn* | Chloramphenicol | 0 | 0 |
| PYZEJS040 | *1851::Tn* | Chloramphenicol | 1.3 X 10⁻⁵ | 7.9 X 10⁻⁶ |
| PYZEJS040-protospacer25 | *1851::Tn* | Chloramphenicol | 0 | 0 |

| **Related to** **Figure 5C** | | | | |
|---|---|---|---|---|
| PYZEJS040 | wt | Chloramphenicol | 2.7 X 10⁻⁵ | 1.0 X 10⁻⁵ |
| PYZEJS040-protospacer25 | wt | Chloramphenicol | 0 | 0 |
| PYZEJS040 | *rnc::Tn*^{(c)} | Chloramphenicol | 1.9 X 10⁻⁵ | 8.2 X 10⁻⁶ |
| PYZEJS040-protospacer25 | *rnc::Tn*^{(c)} | Chloramphenicol | 0 | 0 |
| PYZEJS040 | Δ*rnc* | Chloramphenicol | 6.0 X 10⁻⁶ | 2.3 X 10⁻⁶ |
| PYZEJS040-protospacer25 | Δ*rnc* | Chloramphenicol | 0 | 0 |
| PYZEJS040 | Δ*tracr* | Chloramphenicol | 4.7 X 10⁻⁵ | 2.4 X 10⁻⁵ |
| PYZEJS040-protospacer25 | Δ*tracr* | Chloramphenicol | 1.4 X 10⁻⁵ | 4.5 X 10⁻⁶ |
| PYZEJS040 | Δ*tracr* + *tracr* | Chloramphenicol | 4.3 X 10⁻⁶ | 1.8 X 10⁻⁶ |
| PYZEJS040-protospacer25 | Δ*tracr* + *tracr* | Chloramphenicol | 0 | 0 |
| PYZEJS040 | Δ*tracr* + empty | Chloramphenicol | 3.7 X 10⁻⁶ | 2.4 X 10⁻⁶ |
| PYZEJS040-protospacer25 | Δ*tracr* + empty | Chloramphenicol | 6.7 X 10⁻⁶ | 1.3 X 10⁻⁶ |

| **Related to** **Figure 6B** | | | | |
|---|---|---|---|---|
| gDNA of *cas9::Tn* | wt | Erythromycin | 0 | 0 |
| gDNA of *cas9::Tn*/pGCC2 empty | wt | Erythromycin | 1.6 X 10⁻⁵ | 6.1 X 10⁻⁶ |
| gDNA of *cas9::Tn*/pGCC2-Ps25 | wt | Erythromycin | 0 | 0 |

| **Related to** **Figure 6B** | | | | |
|---|---|---|---|---|
| gDNA of *cas9::Tn* | wt | Chloramphenicol | 0 | 0 |
| gDNA of *cas9::Tn*/pYZEJS040 | wt | Chloramphenicol | 5.1 X 10⁻⁶ | 2.6 X 10⁻⁶ |
| gDNA of *cas9::Tn*/pYZEJS040-Ps25 | wt | Chloramphenicol | 0 | 0 |

| **Related to Figure S4** | | | | |
|---|---|---|---|---|
| PYZEJS040 | wt | Chloramphenicol | 6.0 X 10⁻⁶ | 1.6 X 10⁻⁶ |
| PYZEJS040-protospacer 9 | wt | Chloramphenicol | 0 | 0 |
| PYZEJS040-protospacer 25 | wt | Chloramphenicol | 0 | 0 |

| **Related to interference assays testing internal protospacer 9 in** *rnc::Tn* and Δ*rnc* strains. | | | | |
|---|---|---|---|---|
| PYZEJS040 | wt | Chloramphenicol | 7.3 X 10⁻⁶ | 1.5 X 10⁻⁶ |
| PYZEJS040-protospacer 9 | wt | Chloramphenicol | 0 | |
| PYZEJS040 | *rnc::Tn* | Chloramphenicol | 2.2 X 10⁻⁵ | 2.5 X 10⁻⁶ |
| PYZEJS040-protospacer 9 | *rnc::Tn* | Chloramphenicol | 0 | |
| PYZEJS040 | Δ*rnc* | Chloramphenicol | 6.9 X 10⁻⁶ | 3.7 X 10⁻⁶ |
| PYZEJS040-protospacer 9 | Δ*rnc* | Chloramphenicol | 0 | |
| ^{(a)} Indicates antibiotic used to select transformants. | | | | |
| ^{(b)} The average and standard error of the mean (s.e.m.) of transformation frequencies (ratios comparing transformants cfu/ml vs. total cfu/ml) from at least three independent experiments. | | | | |
| ^{(c)} The *rnc::Tn* mutant of *N. meningitidis* 8013 exhibited obvious slow-growth defects. | | | | |

**Table 6. Characteristics of the seven CRISPR/Cas-containing Neisseria strains**

| **Strain name** | **Serogroup**^{(a)} | **Country/Year**^{(a)} | **ST**^{(a)} | **Clonal Complex**^{(a)} | **CRISPR_id**^{(b)} |
|---|---|---|---|---|---|
| ***N*. *meningitidis*** | | | | | |
| Z2491 | A | Gambia /1983 | 4 | ST-4 complex | NC_003116_10 |
| 8013 | C | France /1989 | 177 | ST-18 complex | NC_017501_11 |
| WUE2594 | A | Germany /1991 | 5 | ST-5 complex | NC_017512_9 |
| M01-240355 | B | UK /2001 | 213 | ST-213 complex | NC_017517_9 |
| 053442 | C | China/2004 | 4821 | ST-4821 complex | NC_010120_5 |
| alpha14 | NG | Germany/1999 | 53 | ST-53 complex | NC_013016_1 to NC_013016_2^{(c)} |

| ***N*. lactamica** | | | | | |
|---|---|---|---|---|---|
| 020-06 | N/A | UK / 1997 | 640 | ST-640 complex | NC_014752_11 |
| ^{(a)} according to Neisseria PubMLST database (Jolley and Maiden, 2010) | | | | | |
| ^{(b)} according to CRISPRdb | | | | | |
| ^{(c)} NC_013016_1 and NC_013016_2 each constitute part of our predicted CRISPR in *N. meningitidis* alpha14. | | | | | |

**Table 7. Summary of gene targeting efficiency using NmCas9 in hPSCs**

| Cell line | crRNA | Clone analyzed | Nontargeted | Targeted with additional insertions | Targeted | Targeting efficiency (%) |
|---|---|---|---|---|---|---|
| H1 (ES) | Nontargeting | 0 | 0 | 0 | 0 | 0 |
| | Targeting | 20 | 5 | 3 | 12 | 60 |
| H9 (ES) | Nontargeting | 1 | 1 | 0 | 0 | 0 |
| | Targeting | 39 | 9 | 7 | 23 | 59 |
| iPS005 (iPS) | Nontargeting | 1 | 1 | 0 | 0 | 0 |
| | Targeting | 10 | 1 | 3 | 6 | 60 |

**Table 8. Plasmids used in this study**

| Plasmid no. | Plasmid name | Description |
|---|---|---|
| 1 | pSimplell-NmCas9-FLAG | Flag tagged NmCas9 without NLS |
| 2 | pSimplell-NLS-NmCas9-EGFP | NmCas9 EGFP fusion with N-terminal NLS |
| 3 | pSimpleII-NmCas9-HA-NLS | NmCas9 with C-terminal HA tag and NLS |
| 4 | pSimpleII-NLS-NmCas9-HA-NLS(s) | NmCas9 with dual NLS and HA tags |
| 5 | pSimpleII-U6-tracrRNA-U6-BsmBI-NLS-NmCas9-HA-NLS(s) | All-in-one plasmid containing NmCas9, tracrRNA expression cassette and U6-BsmBI cassette |
| 6 | pSimpleII-U6-tracrRNA-U6-crRNA(tdTomato)-NLS-NmCas9-HA-NLS(s) | All-in-one plasmid containing NmCas9, tracrRNA expression cassette and tdTomato-targeting crRNA expression cassette |
| 7 | pSimpleII-U6-tracrRNA-U6-crRNA(EGFP)-NLS-NmCas9-HA-NLS(s) | All-in-one plasmid containing NmCas9, tracrRNA expression cassette and EGFP-targeting crRNA expression cassette |
| 8 | pSimpleII-U6-tracrRNA-U6-crRNA(OCT4)-NLS-NmCas9-HA-NLS(s) | All-in-one plasmid containing NmCas9, tracrRNA expression cassette and OCT4-targeting crRNA expression cassette |
| 9 | pSTBIue-1-U6-sgRNA (tdTomato) | U6-driven sgRNA targeting tdTomato |

**Table 9. crRNA-encoding DNA sequences used in this study**

| crRNA | Encoding DNA sequence |
|---|---|
| EGFP targeting crRNA | gttcagcgtgtccggcgagggcgaGTTGTAGCTCCCTTTCTCATTTCG |
| OCT4 targeting crRNA | GacctggagtttgtgccagggtttGTTGTAGCTCCCTTTCTCATTTCG |
| tdTomato targeting crRNA (GATT PAM) | gtacgtgaagcaccccgccgacatGTTGTAGCTCCCTTTCTCATTTCG |
| tdTomato targeting crRNA (GATG PAM) | GccccgagggcttcaagtgggagcGTTGTAGCTCCCTTTCTCATTTCG |
| tdTomato targeting crRNA (GACT PAM) | ggacggcggtctggtgaccgtgacGTTGTAGCTCCCTTTCTCATTTCG |
| tdTomato targeting crRNA (GCTT PAM) | gattacaagaagctgtccttccccGTTGTAGCTCCCTTTCTCATTTCG |
| tdTomato targeting crRNA (CATT PAM) | GggcctcccagcccatggtcttctGTTGTAGCTCCCTTTCTCATTTCG |
| tdTomato targeting crRNA (CCAA PAM) | ggccgcccctacgagggcacccagGTTGTAGCTCCCTTTCTCATTTCG |

| | |
|---|---|
| All sequences are 5' to 3', left to right. Spacer regions are in lowercase and underlined, and CRISPR repeat regions are in uppercase. In some cases, the first nucleotide of the spacer is changed to a G to satisfy the requirement of the U6 promoter. | |

**Table 10. Primers used in this study**

| Name | Forward | Reverse |
|---|---|---|
| For cleavage site mapping in tdTomato | ATGGTGAGCAAGGGCGAGGAG | CCGGTGCTGCCGGTGCCATGCCCCAG |
| For tdTomato indel mapping | AACACTGTCCCTCTCATGTCCCTGCTTC | CCGGTGCTGCCGGTGCCATGCCCCAG |
| For making a Southern blot probe for OCT4 | GTGATGCCACCAAGAACCTT | ACAGCAGCGAGCAAATAGGT |

| | | |
|---|---|---|
| All sequences are 5' to 3', left to right. | | |

**Table 11. Sequences of unprocessed and processed tracrRNA and crRNA in N. meningitidis**

| tracrRNA and crRNA | Sequence |
|---|---|
| Unprocessed tracrRNA | |
| Processed tracrRNA | |
| Full-length spacer+repeat unit in CRISPR locus | |
| Processed crRNA | 5'NNNNNNNNNNNNNNNNNNNNNNNNGTTGTAGCTCCCTTTCTCATTTCG 3' |

| | |
|---|---|
| Sequences that are cleaved off during processing are underlined. | |

## Claims

1. A method for modifying a target DNA sequence in a cell, the method comprising:
(a) expressing an exogenous Cas9 protein from a *Neisseria* species or a variant protein thereof having at least 80% amino acid sequence identity relative to SEQ ID NO: 1 in the cell by introducing a DNA molecule or an RNA molecule that expresses the Cas9 protein; wherein the Cas9 protein or variant has nuclease activity and the ability to bind to a target polynucleotide sequence in the cell; and
(b) expressing or transfecting RNA comprising a CRISPR RNA (crRNA) and a trans-activating CRISPR RNA (tracrRNA) in the cell, wherein the RNA binds to the Cas9 protein or variant, and the RNA hybridizes to the target DNA sequence
with the proviso that the method is not a method for modifying the germ line genetic identity of human beings; and with the proviso that the method is:
i) performed *in vitro* or *ex vivo;* or
ii) not a method for treatment of the human or animal body by therapy.

2. The method of claim 1, wherein the Cas9 protein or variant protein:
(i) cleaves both strands of the target DNA sequence; or
(ii) nicks a single strand of the target DNA sequence,
optionally wherein the method further comprises contacting the target DNA sequence with a homologous DNA fragment.

3. The method of any previous claim, wherein the Cas9 protein or variant protein is expressed from a nucleic acid having a codon sequence that is optimized for expression in the cell.

4. The method of any previous claim, wherein the Cas9 protein is from *Neisseria meningitidis.*

5. The method of any previous claim, wherein expressing a Cas9 protein from a *Neisseria* species or a variant protein thereof in the cell comprises transfecting the cell with an expression vector that expresses the Cas9 protein from a eukaryotic promoter.

6. The method of any of claims 1 to 4, wherein expressing a Cas9 protein from a *Neisseria* species or a variant protein thereof in the cell comprises transfecting the cell with an mRNA that encodes the Cas9 protein

7. The method of any previous claim, wherein expressing an RNA in the cell that binds to the Cas9 protein or variant and hybridizes to the target DNA sequence comprises transfecting the cell with an expression vector that expresses the RNA from a eukaryotic promoter.

8. The method of any of claims 1 to 4 or 6, wherein the cell is a prokaryotic cell.

9. The method of any of claims 1 to 7, wherein the cell is a eukaryotic cell.

10. The method of claim 9, wherein the cell is a stem cell.

11. The method of claim 10, wherein the cell is an embryonic stem cell or an induced pluripotent stem cell.

12. The method of any previous claim, wherein the RNA comprises:
(i) two molecules of duplexed RNA; or
(ii) a single RNA molecule forming a hairpin structure; or
(iii) an RNA mimic of green fluorescent protein (GFP); or
(iv) Xist RNA.

13. The method of any previous claim, further comprising contacting the target DNA sequence with 4-hydroxybenzylidene, 3,5-dimethoxy-4-hydroxybenzylidene, or a 3,5-difluoro-4-hydroxybenzylidene.

14. A kit for performing the method of claim 1 comprising:
(a) a vector for expressing a Cas9 protein from a *Neisseria* species or a variant protein thereof having at least 80% amino acid sequence identity relative to SEQ ID NO: 1, wherein the Cas9 protein or variant has nuclease activity and the ability to bind to a target polynucleotide sequence in the cell; and
(b) a vector for expressing an RNA in the cell, wherein the RNA comprises a CRISPR RNA (crRNA) and a trans-activating CRISPR RNA (tracrRNA) wherein the RNA binds to the Cas9 protein or variant, and the RNA hybridizes to the target DNA sequence.

15. The method of claim 1, wherein:
(a) expressing a Cas9 protein from a *Neisseria* species or a variant protein thereof having nuclease activity and the ability to bind to a target polynucleotide sequence in the cell comprises non-viral delivery of RNA encoding the protein to the cell; and/or
(b) expressing or transfecting RNA comprising a CRISPR RNA (crRNA) and a trans-activating CRISPR RNA (tracrRNA) in the cell, wherein the RNA binds to the Cas9 protein or variant, and the RNA hybridizes to the target DNA sequence comprises non-viral delivery of the RNA.

## Patentansprüche

1. Verfahren zum Modifizieren einer Ziel-DNA-Sequenz in einer Zelle, wobei das Verfahren umfasst:
(a) Exprimieren eines exogenen Cas9-Proteins aus einer Neisseria-Spezies oder einer Proteinvariante davon mit mindestens 80 % Aminosäuresequenzidentität relativ zu SEQ ID NO: 1 in der Zelle durch Einführen eines DNA-Moleküls oder eines RNA-Moleküls, das das Cas9-Protein exprimiert, wobei das Cas9-Protein oder die Cas9-Variante Nukleaseaktivität und die Fähigkeit zur Bindung an eine Ziel-Polynukleotidsequenz in der Zelle aufweist, und
(b) Exprimieren oder Transfizieren von RNA, die eine CRISPR-RNA (cNA) und eine trans-aktivierende CRISPR-RNA (tracNA) umfasst, in der Zelle, wobei die RNA an das Cas9-Protein oder die Cas9-Variante bindet, und die RNA mit der Ziel-DNA-Sequenz hybridisiert;
mit der Maßgabe, dass das Verfahren kein Verfahren zur Veränderung der genetischen Identität der Keimbahn des Menschen ist; und mit der Maßgabe, dass das Verfahren:
i) in vitro oder ex vivo durchgeführt wird; oder
ii) kein Verfahren zur Behandlung des menschlichen oder tierischen Körpers durch Therapie ist.

2. Verfahren nach Anspruch 1, bei dem das Cas9-Protein oder die Cas9 -Proteinvariante:
(i) beide Stränge der Ziel-DNA-Sequenz spaltet; oder
(ii) einen einzelnen Strang der Ziel-DNA-Sequenz anschneidet,
wobei das Verfahren optional ferner das Inkontaktbringen der Ziel-DNA-Sequenz mit einem homologen DNA-Fragment umfasst.

3. Verfahren nach einem der vorhergehenden Ansprüche, bei dem das Cas9-Protein oder die Cas9-Proteinvariante von einer Nukleinsäure mit einer Codonsequenz exprimiert wird, die für die Expression in der Zelle optimiert ist.

4. Verfahren nach einem der vorstehenden Ansprüche, bei dem das Cas9-Protein aus Neisseria meningitidis stammt.

5. Verfahren nach einem der vorhergehenden Ansprüche, bei dem die Expression eines Cas9-Proteins aus einer Neisseria-Spezies oder einer Proteinvariante davon in der Zelle das Transfizieren der Zelle mit einem Expressionsvektor umfasst, der das Cas9-Protein von einem eukaryotischen Promotor exprimiert.

6. Verfahren nach einem der Ansprüche 1 bis 4, bei dem das Exprimieren eines Cas9-Proteins aus einer Neisseria-Spezies oder einer Proteinvariante davon in der Zelle das Transfizieren der Zelle mit einer mRNA umfasst, die für das Cas9-Protein kodiert.

7. Verfahren nach einem der vorhergehenden Ansprüche, bei dem das Exprimieren einer RNA in der Zelle, die an das Cas9-Protein oder die Variante davon bindet und mit der Ziel-DNA-Sequenz hybridisiert, das Transfizieren der Zelle mit einem Expressionsvektor umfasst, der die RNA von einem eukaryotischen Promotor exprimiert.

8. Verfahren nach einem der Ansprüche 1 bis 4 oder 6, bei dem die Zelle eine prokaryotische Zelle ist.

9. Verfahren nach einem der Ansprüche 1 bis 7, bei dem die Zelle eine eukaryotische Zelle ist.

10. Verfahren nach Anspruch 9, bei dem die Zelle eine Stammzelle ist.

11. Verfahren nach Anspruch 10, bei dem die Zelle eine embryonale Stammzelle oder eine induzierte pluripotente Stammzelle ist.

12. Verfahren nach einem der vorhergehenden Ansprüche, bei dem die RNA umfasst:
(i) zwei Moleküle doppelsträngiger RNA; oder
(ii) ein einzelnes RNA-Molekül, das eine Haarnadelstruktur bildet; oder
(iii) eine RNA-Nachbildung von grün fluoreszierende Protein (GFP); oder
(iv) Xist RNA.

13. Verfahren nach einem der vorhergehenden Ansprüche, ferner umfassend
Inkontaktbringen der Ziel-DNA-Sequenz mit 4-Hydroxybenzyliden, 3,5-Dimethoxy-4-hydroxybenzyliden oder einem 3,5-Difluor-4-hydroxybenzyliden.

14. Kit zur Durchführung des Verfahrens nach Anspruch 1, umfassend:
(a) einen Vektor zum Exprimieren eines Cas9-Proteins aus einer Neisseria-Spezies oder einer Proteinvariante davon mit mindestens 80 % Aminosäuresequenzidentität in Bezug auf SEQ ID NO: 1, wobei das Cas9-Protein oder die Cas9-Proteinvariante Nukleaseaktivität und die Fähigkeit zur Bindung an eine Zielpolynukleotidsequenz in der Zelle aufweist; und
(b) einen Vektor zum Exprimieren einer RNA in der Zelle, wobei die RNA eine CRISPR-RNA (cNA) und eine trans-aktivierende CRISPR-RNA (tracNA) umfasst, wobei die RNA an das Cas9-Protein oder die Cas9-Variante bindet und die RNA mit der Ziel-DNA-Sequenz hybridisiert.

15. Verfahren nach Anspruch 1, bei dem:
(a) das Exprimieren eines Cas9-Proteins aus einer Neisseria-Spezies oder einer Proteinvariante davon mit Nuklease-Aktivität und der Fähigkeit, an eine Ziel-Polynukleotidsequenz in der Zelle zu binden, eine nicht-virale Verabreichung von das Protein kodierender RNA an die Zelle umfasst; und/ oder
(b) das Exprimieren oder Transfizieren von RNA, die eine CRISPR-RNA (cNA) und eine trans-aktivierende CRISPR-RNA (tracNA) umfasst, in der Zelle, wobei die RNA an das Cas9-Protein oder die Cas9-Variante bindet und die RNA mit der Ziel-DNA-Sequenz hybridisiert, eine nicht-virale Verabreichung der RNA umfasst.

## Revendications

1. Procédé pour modifier une séquence d'ADN cible dans une cellule, le procédé comprenant les étapes consistant à :
(a) exprimer une protéine Cas9 exogène d'une espèce de *Neisseria* ou une protéine variante de celle-ci ayant au moins 80 % d'identité de séquence d'acides aminés par rapport à SEQ ID NO : 1 dans la cellule en introduisant une molécule d'ADN ou une molécule d'ARN qui exprime la protéine Cas9 ; dans lequel la protéine Cas9 ou la variante a une activité nucléase et la capacité de se lier à une séquence polynucléotidique cible dans la cellule ; et
(b) exprimer ou transfecter de l'ARN comprenant un ARN CRISPR (ARNc) et un ARN CRISPR trans-activateur (ARNtracr) dans la cellule, dans lequel l'ARN se lie à la protéine Cas9 ou la variante, et l'ARN s'hybride à la séquence d'ADN cible
à condition que le procédé ne soit pas un procédé pour modifier l'identité génétique de lignée germinale d'êtres humains ; et à condition que le procédé :
i) soit réalisé *in vitro* ou *ex vivo* ; ou
ii) ne soit pas un procédé de traitement du corps humain ou animal par thérapie.

2. Procédé selon la revendication 1, dans lequel la protéine Cas9 ou la protéine variante :
(i) clive les deux brins de la séquence d'ADN cible ; ou
(ii) coupe un brin unique de la séquence d'ADN cible,
facultativement dans lequel le procédé comprend en outre la mise en contact de la séquence d'ADN cible avec un fragment d'ADN homologue.

3. Procédé selon l'une quelconque des revendications précédentes, dans lequel la protéine Cas9 ou la protéine variante est exprimée à partir d'un acide nucléique ayant une séquence de codons qui est optimisée pour expression dans la cellule.

4. Procédé selon l'une quelconque des revendications précédentes, dans lequel la protéine Cas9 provient de *Neisseria meningitidis.*

5. Procédé selon l'une quelconque des revendications précédentes, dans lequel l'expression d'une protéine Cas9 d'une espèce de *Neisseria* ou d'une protéine variante de celle-ci dans la cellule comprend la transfection de la cellule avec un vecteur d'expression qui exprime la protéine Cas9 à partir d'un promoteur eucaryote.

6. Procédé selon l'une quelconque des revendications 1 à 4, dans lequel l'expression d'une protéine Cas9 provenant d'une espèce de *Neisseria* ou d'une protéine variante de celle-ci dans la cellule comprend la transfection de la cellule avec un ARNm qui code pour la protéine Cas9.

7. Procédé selon l'une quelconque des revendications précédentes, dans lequel l'expression d'un ARN dans la cellule qui se lie à la protéine Cas9 ou la variante et s'hybride à la séquence d'ADN cible comprend la transfection de la cellule avec un vecteur d'expression qui exprime l'ARN à partir d'un promoteur eucaryote.

8. Procédé selon l'une quelconque des revendications 1 à 4 ou 6, dans lequel la cellule est une cellule procaryote.

9. Procédé selon l'une quelconque des revendications 1 à 7, dans lequel la cellule est une cellule eucaryote.

10. Procédé selon la revendication 9, dans lequel la cellule est une cellule souche.

11. Procédé selon la revendication 10, dans lequel la cellule est une cellule souche embryonnaire ou une cellule souche pluripotente induite.

12. Procédé selon l'une quelconque des revendications précédentes, dans lequel l'ARN comprend :
(i) deux molécules d'ARN duplexé ; ou
(ii) une molécule d'ARN unique formant une structure en épingle à cheveux ; ou
(iii) un ARN imitant une protéine fluorescente verte (GFP) ; ou
(iv) de l'ARN Xist.

13. Procédé selon l'une quelconque des revendications précédentes, comprenant en outre la mise en contact de la séquence d'ADN cible avec du 4-hydroxybenzylidène, du 3,5-diméthoxy-4-hydroxybenzylidène ou du 3,5-difluoro-4-hydroxybenzylidène.

14. Kit pour exécuter le procédé de la revendication 1, comprenant :
(a) un vecteur pour exprimer une protéine Cas9 provenant d'une espèce de *Neisseria* ou une protéine variante de celle-ci ayant au moins 80 % d'identité de séquence d'acides aminés par rapport à SEQ ID NO : 1, dans lequel la protéine Cas9 ou la variante a une activité nucléase et la capacité de se lier à une séquence polynucléotidique cible dans la cellule ; et
(b) un vecteur pour exprimer un ARN dans la cellule, dans lequel l'ARN comprend un ARN CRISPR (ARNcr) et un ARN CRISPR trans-activateur (ARNtracr), dans lequel l'ARN se lie à la protéine Cas9 ou la variante, et l'ARN s'hybride à la séquence d'ADN cible.

15. Procédé selon la revendication 1, dans lequel :
(a) l'expression d'une protéine Cas9 provenant d'une espèce de *Neisseria* ou d'une protéine variante de celle-ci ayant une activité nucléase et la capacité de se lier à une séquence polynucléotidique cible dans la cellule comprend la délivrance non virale d'ARN codant la protéine à la cellule ; et/ou
| PYZEJS040-protospacer25 | Δ*tracr* + empty | Chloramphenicol | 6.7 X 10⁻⁶ | 1.3 X 10⁻⁶ |
|---|---|---|---|---|
| **Related to Figure 6B** | | | | |
| gDNA of *cas9::Tn* | wt | Erythromycin | 0 | 0 |
| gDNA of *cas9::Tn*/pGCC2 empty | wt | Erythromycin | 16 X 10⁻⁵ | 6.1 X 10⁻⁶ |
| gDNA of *cas9::Tn*/pGCC2-Ps25 | wt | Erythromycin | 0 | 0 |
| **Related to Figure 6B** | | | | |
|---|---|---|---|---|
| gDNA of *cas9::Tn* | wt | Chloramphenicol | 0 | 0 |
| gDNA of *cas9::Tn*/ pYZEJS040 | wt | Chloramphenicol | 5.1 X 10⁻⁶ | 2.6 X 10⁻⁶ |
| gDNA of *cas9::Tn*/ pYZEJS040-Ps25 | wt | Chloramphenicol | 0 | 0 |
| **Related to Figure S4** | | | | |
|---|---|---|---|---|
| pYZEJS040 | wt | Chloramphenicol | 6.0 X 10⁻⁶ | 1.6 X 10⁻⁶ |
| pYZEJS040-protospacer 9 | wt | Chloramphenicol | 0 | 0 |
| pYZEJS040-protospacer 25 | wt | Chloramphenicol | 0 | 0 |
| **Related to Interference assays testing internal protospacer 9 in** *rnc::Tn* and Δ*rnc* strains. | | | | |
|---|---|---|---|---|
| pYZEJS040 | wt | Chloramphenicol | 7.3 X 10⁻⁶ | 1.5 X 10⁻⁶ |
| pYZEJS040-protospacer 9 | wt | Chloramphenicol | 0 | |
| pYZEJS040 | *rnc::Tn* | Chloramphenicol | 2.2 X 10⁻⁵ | 2.5 X 10⁻⁶ |
| pYZEJS040-protospacer 9 | *rnc::Tn* | Chloramphenicol | 0 | |
| pYZEJS040 | Δ*rnc* | Chloramphenicol | 6.9 X 10⁻⁶ | 3.7 X 10⁻⁶ |
| pYZEJS040-protospacer 9 | Δ*rnc* | Chloramphenicol | 0 | |
| ^{(a)} Indicates antibiotic used to select transformants. | | | | |
| ^{(b)} The average and standard error of the mean (s.e.m.) of transformation frequencies (ratios comparing transformants cfu/ml vs. total cfu/ml) from at least three independent experiments. | | | | |
| ^{(c)} The *rnc::Tn* mutant of *N*. *meningitidis* 8013 exhibited obvious slow-growth defects. | | | | |


### SEQUENCE LISTING

<110> Northwestern University Wisconsin Alumni Research Foundation Sontheimer, Erik J. Zhang, Yan Mondragon, Alfonso Rajan, Rakhi Thomson, James Hou, Zhonggang
<120> RNA-DIRECTED DNA CLEAVAGE AND GENE EDITING BY CAS9 ENZYME FROM NEISSERIA MENINGITIDIS
<130> 5369-00075
<150> US 61/826,338
   <151> 2013-05-22
<160> 9
<170> PatentIn version 3.5
<210> 1
   <211> 1082
   <212> PRT
   <213> Neisseria meningitidis
<400> 1
   Met Ala Ala Phe Lys Pro Asn Pro Ile Asn Tyr Ile Leu Gly Leu Asp
   1               5                   10                  15
   Ile Gly Ile Ala Ser Val Gly Trp Ala Met Val Glu Ile Asp Glu Asp
               20                  25                  30
   Glu Asn Pro Ile Cys Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
   130 135 140
   Lys Asn Glu Gly Glu Thr Ala Asp Lys Glu Leu Gly Ala Leu Leu Lys
   145 150 155 160
   Gly Val Ala Asp Asn Ala His Ala Leu Gln Thr Gly Asp Phe Arg Thr
   165 170 175
   Pro Ala Glu Leu Ala Leu Asn Lys Phe Glu Lys Glu Ser Gly His Ile
   180 185 190
   Arg Asn Gln Arg Gly Asp Tyr Ser His Thr Phe Ser Arg Lys Asp Leu
   195 200 205
   Gln Ala Glu Leu Ile Leu Leu Phe Glu Lys Gln Lys Glu Phe Gly Asn
   210 215 220
   Pro His Val Ser Gly Gly Leu Lys Glu Gly Ile Glu Thr Leu Leu Met
   225 230 235 240
   Thr Gln Arg Pro Ala Leu Ser Ser Asp Ala Val Gln Lys Met Leu Gly
   245 250 255
   His Cys Thr Phe Glu Pro Ala Glu Pro Lys Ala Ala Lys Asn Thr Tyr
   260 265 270
   Thr Ala Glu Arg Phe Ile Trp Leu Thr Lys Leu Asn Asn Leu Arg Ile
   275 280 285
   Leu Glu Gln Gly Ser Glu Arg Pro Leu Thr Asp Thr Glu Arg Ala Thr
   290 295 300
   Leu Met Asp Glu Pro Tyr Arg Lys Ser Lys Leu Thr Tyr Ala Gln Ala
   305 310 315 320
   Arg Lys Leu Leu Gly Leu Glu Asp Thr Ala Phe Phe Lys Gly Leu Arg
   325 330 335
   Tyr Gly Lys Asp Asn Ala Glu Ala Ser Thr Leu Met Glu Met Lys Ala
   340 345 350
   Tyr His Ala Ile Ser Arg Ala Leu Glu Lys Glu Gly Leu Lys Asp Lys
   355 360 365
   Lys Ser Pro Leu Asn Leu Ser Pro Glu Leu Gln Asp Glu Ile Gly Thr
   370 375 380
   Ala Phe Ser Leu Phe Lys Thr Asp Glu Asp Ile Thr Gly Arg Leu Lys
   385                 390                 395                 400
   Asp Arg Ile Gln Pro Glu Ile Leu Glu Ala Leu Leu Lys His Ile Ser
                   405                 410                 415
   Phe Asp Lys Phe Val Gln Ile Ser Leu Lys Ala Leu Arg Arg Ile Val
               420                 425                 430
   Pro Leu Met Glu Gln Gly Lys Arg Tyr Asp Glu Ala Cys Ala Glu Ile
           435                 440                 445
   Tyr Gly Asp His Tyr Gly Lys Lys Asn Thr Glu Glu Lys Ile Tyr Leu
       450                 455                 460
   Pro Pro Ile Pro Ala Asp Glu Ile Arg Asn Pro Val Val Leu Arg Ala
   465                 470                 475                 480
   Leu Ser Gln Ala Arg Lys Val Ile Asn Gly Val Val Arg Arg Tyr Gly
                   485                 490                 495
   Ser Pro Ala Arg Ile His Ile Glu Thr Ala Arg Glu Val Gly Lys Ser
               500                 505                 510
   Phe Lys Asp Arg Lys Glu Ile Glu Lys Arg Gln Glu Glu Asn Arg Lys
           515                 520                 525
   Asp Arg Glu Lys Ala Ala Ala Lys Phe Arg Glu Tyr Phe Pro Asn Phe
       530                 535                 540
   Val Gly Glu Pro Lys Ser Lys Asp Ile Leu Lys Leu Arg Leu Tyr Glu
   545                 550                 555                 560
   Gln Gln His Gly Lys Cys Leu Tyr Ser Gly Lys Glu Ile Asn Leu Gly
                   565                 570                 575
   Arg Leu Asn Glu Lys Gly Tyr Val Glu Ile Asp His Ala Leu Pro Phe
               580                 585                 590
   Ser Arg Thr Trp Asp Asp Ser Phe Asn Asn Lys Val Leu Val Leu Gly
           595                 600                 605
   Ser Glu Asn Gln Asn Lys Gly Asn Gln Thr Pro Tyr Glu Tyr Phe Asn
       610                 615                 620
   Gly Lys Asp Asn Ser Arg Glu Trp Gln Glu Phe Lys Ala Arg Val Glu
   625                 630                 635                 640
   Thr Ser Arg Phe Pro Arg Ser Lys Lys Gln Arg Ile Leu Leu Gln Lys
                   645                 650                 655
   Phe Asp Glu Asp Gly Phe Lys Glu Arg Asn Leu Asn Asp Thr Arg Tyr
               660                 665                 670
   Val Asn Arg Phe Leu Cys Gln Phe Val Ala Asp Arg Met Arg Leu Thr
           675                 680                 685
   Gly Lys Gly Lys Lys Arg Val Phe Ala Ser Asn Gly Gln Ile Thr Asn
       690                 695                 700
   Leu Leu Arg Gly Phe Trp Gly Leu Arg Lys Val Arg Ala Glu Asn Asp
   705                 710                 715                 720
   Arg His His Ala Leu Asp Ala Val Val Val Ala Cys Ser Thr Val Ala
                   725                 730                 735
   Met Gln Gln Lys Ile Thr Arg Phe Val Arg Tyr Lys Glu Met Asn Ala
               740                 745                 750
   Phe Asp Gly Lys Thr Ile Asp Lys Glu Thr Gly Glu Val Leu His Gln
           755                 760                 765
   Lys Thr His Phe Pro Gln Pro Trp Glu Phe Phe Ala Gln Glu Val Met
       770                 775                 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
               820                 825                 830
   Ala Pro Asn Arg Lys Met Ser Gly Gln Gly His Met Glu Thr Val Lys
           835                 840                 845
   Ser Ala Lys Arg Leu Asp Glu Gly Val Ser Val Leu Arg Val Pro Leu
       850                 855                 860
   Thr Gln Leu Lys Leu Lys Asp Leu Glu Lys Met Val Asn Arg Glu Arg
   865                 870                 875                 880
   Glu Pro Lys Leu Tyr Glu Ala Leu Lys Ala Arg Leu Glu Ala His Lys
                   885                 890                 895
   Asp Asp Pro Ala Lys Ala Phe Ala Glu Pro Phe Tyr Lys Tyr Asp Lys
               900                 905                 910
   Ala Gly Asn Arg Thr Gln Gln Val Lys Ala Val Arg Val Glu Gln Val
           915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser  Leu His Pro Asn Asp  Leu Val Glu
           995                 1000                1005
   Val Ile  Thr Lys Lys Ala Arg  Met Phe Gly Tyr Phe  Ala Ser Cys
       1010                 1015                1020
   His Arg  Gly Thr Gly Asn Ile  Asn Ile Arg Ile His  Asp Leu Asp
       1025                 1030                1035
   His Lys  Ile Gly Lys Asn Gly  Ile Leu Glu Gly Ile  Gly Val Lys
       1040                 1045                1050
   Thr Ala  Leu Ser Phe Gln Lys  Tyr Gln Ile Asp Glu  Leu Gly Lys
       1055                 1060                1065
   Glu Ile  Arg Pro Cys Arg Leu  Lys Lys Arg Pro Pro  Val Arg
       1070                 1075                1080
<210> 2
   <211> 3249
   <212> DNA
   <213> Neisseria meningitidis
<400> 2
   atggctgcct tcaaacctaa ttcaatcaac tacatcctcg gcctcgatat cggcatcgca       60
   tccgtcggct gggcgatggt agaaattgac gaagaagaaa accccatccg cctgattgat      120
   ttgggcgtgc gcgtatttga gcgtgccgaa gtaccgaaaa caggcgactc ccttgccatg      180
   gcaaggcgtt tggcgcgcag tgttcgccgc ctgacccgcc gtcgcgccca ccgcctgctt      240
   cggacccgcc gcctattgaa acgcgaaggc gtattacaag ccgccaattt tgacgaaaac 300
   ggcttgatta aatccttacc gaatacacca tggcaacttc gcgcagccgc attagaccgc 360
   aaactgacgc ctttagagtg gtcggcagtc ttgttgcatt taatcaaaca tcgcggctat 420
   ttatcgcaac ggaaaaacga gggcgaaact gccgataagg agcttggcgc tttgcttaaa 480
   ggcgtagccg gcaatgccca tgccttacag acaggcgatt tccgcacacc ggccgaattg 540
   gctttaaata aatttgagaa agaaagcggc catatccgca atcagcgcag cgattattcg 600
   catacgttca gccgcaaaga tttacaggcg gagctgattt tgctgtttga aaaacaaaaa 660
   gaatttggca atccgcatgt ttcaggcggc cttaaagaag gtattgaaac cctactgatg 720
   acgcaacgcc ctgccctgtc cggcgatgcc gttcaaaaaa tgttggggca ttgcaccttc 780
   gaaccggcag agccgaaagc cgctaaaaac acctacacag ccgaacgttt catctggctg 840
   accaagctga acaacctgcg tattttagag caaggcagcg agcggccatt gaccgatacc 900
   gaacgcgcca cgcttatgga cgagccatac agaaaatcca aactgactta cgcacaagcc 960
   cgtaagctgc tgggtttaga agataccgcc tttttcaaag gcttgcgcta tggtaaagac 1020
   aatgccgaag cctcaacatt gatggaaatg aaggcctacc atgccatcag ccgtgcactg 1080
   gaaaaagaag gattgaaaga caaaaaatcc ccattaaacc tttctcccga attacaagac 1140
   gaaatcggca cggcattctc cctgttcaaa accgatgaag acattacagg ccgtctgaaa 1200
   gaccgtatac agcccgaaat cttagaagcg ctgttgaaac acatcagctt cgataagttc 1260
   gtccaaattt ccttgaaagc attgcgccga attgtgcctc taatggaaca aggcaaacgt 1320
   tacgatgaag cctgcgccga aatctacgga gaccattacg gcaagaagaa tacggaagaa 1380
   aagatttatc tgccgccgat tcccgccgac gaaatccgca accccgtcgt cttgcgcgcc 1440
   ttatctcaag cacgtaaggt cattaacggc gtggtacgcc gttacggctc cccagctcgt 1500
   atccatattg aaactgcaag ggaagtaggt aaatcgttta aagaccgcaa agaaattgag 1560
   aaacgccaag aagaaaaccg caaagaccgg gaaaaagccg ccgccaaatt ccgagagtat 1620
   ttccccaatt ttgtcggaga acccaaatcc aaagatattc tgaaactgcg cctgtacgag 1680
   caacaacacg gcaaatgcct gtattcgggc aaagaaatca acttaggccg tctgaacgaa 1740
   aaaggctatg tcgaaatcga ccatgccctg ccgttctcgc gcacatggga cgacagtttc    1800
   aacaataaag tactggtatt gggcagcgaa aaccaaaaca aaggcaatca aaccccttac    1860
   gaatacttca acggcaaaga caacagccgc gaatggcagg aatttaaagc gcgtgtcgaa 1920
   accagccgtt tcccgcgcag taaaaaacaa cggattctgc tgcaaaaatt cgatgaagac 1980
   ggctttaaag aacgcaatct gaacgacacg cgctacgtca accgtttcct gtgtcaattt 2040
   gttgccgacc gtatgcggct gacaggtaaa ggcaagaaac gtgtctttgc atccaacgga 2100
   caaattacca atctgttgcg cggcttttgg ggattgcgca aagtgcgtgc ggaaaacgac     2160
   cgccatcacg ccttggacgc cgtcgtcgtt gcctgctcga ccgttgccat gcagcagaaa     2220
   attacccgtt ttgtacgcta taaagagatg aacgcgtttg acggtaaaac catagacaaa     2280
   gaaacaggag aagtgctgca tcaaaaaaca cacttcccac aaccttggga atttttcgca     2340
   caagaagtca tgattcgcgt cttcggcaaa ccggacggca aacccgaatt cgaagaagcc     2400
   gataccctag aaaaactgcg cacgttgctt gccgaaaaat tatcatctcg ccccgaagcc     2460
   gtacacgaat acgttacgcc actgtttgtt tcacgcgcgc ccaatcggaa gatgagcggg     2520
   caagggcata tggagaccgt caaatccgcc aaacgactgg acgaaggcgt cagcgtgttg     2580
   cgcgtaccgc tgacacagtt aaaactgaaa gacttggaaa aaatggtcaa tcgggagcgc     2640
   gaacctaagc tatacgaagc actgaaagca cggctggaag cacataaaga cgatcctgcc     2700
   aaagcctttg ccgagccgtt ttacaaatac gataaagcag gcaaccgcac ccaacaggta     2760
   aaagccgtac gcgtagagca agtacagaaa accggcgtat gggtgcgcaa ccataacggt     2820
   attgccgaca acgcaaccat ggtgcgcgta gatgtgtttg agaaaggcga caagtattat     2880
   ctggtaccga tttacagttg gcaggtagcg aaagggattt tgccggatag ggctgttgta     2940
   caaggaaaag atgaagaaga ttggcaactt attgatgata gtttcaactt taaattctca     3000
   ttacacccta atgatttagt cgaggttata acaaaaaaag ctagaatgtt tggttacttt     3060
   gccagctgcc atcgaggcac aggtaatatc aatatacgca ttcatgatct tgatcataaa     3120
   attggcaaaa atggaatact ggaaggtatc ggcgtcaaaa ccgccctttc attccaaaaa     3180
   taccaaattg acgaactggg caaagaaatc agaccatgcc gtctgaaaaa acgcccgcct     3240
   gtccgttaa                                                             3249
<210> 3
   <211> 3333
   <212> DNA
   <213> Artificial
<220>
   <223> Recombinant DNA encoding Cas9 protein of Neisseria meningitidis fused to nuclear localiation signal of SV40, hemagglutinin tag of human influenza virus, and synthetic nuclear localization signal
<400> 3
   atggtgccta agaagaagag aaaggtggct gccttcaaac ctaattcaat caactacatc      60
   ctcggcctcg atatcggcat cgcatccgtc ggctgggcga tggtagaaat tgacgaagaa     120
   gaaaacccca tccgcctgat tgatttgggc gtgcgcgtat ttgagcgtgc cgaagtaccg     180
   aaaacaggcg actcccttgc catggcaagg cgtttggcgc gcagtgttcg ccgcctgacc     240
   cgccgtcgcg cccaccgcct gcttcggacc cgccgcctat tgaaacgcga aggcgtatta     300
   caagccgcca attttgacga aaacggcttg attaaatcct taccgaatac accatggcaa     360
   cttcgcgcag ccgcattaga ccgcaaactg acgcctttag agtggtcggc agtcttgttg 420
   catttaatca aacatcgcgg ctatttatcg caacggaaaa acgagggcga aactgccgat 480
   aaggagcttg gcgctttgct taaaggcgta gccggcaatg cccatgcctt acagacaggc 540
   gatttccgca caccggccga attggcttta aataaatttg agaaagaaag cggccatatc 600
   cgcaatcagc gcagcgatta ttcgcatacg ttcagccgca aagatttaca ggcggagctg 660
   attttgctgt ttgaaaaaca aaaagaattt ggcaatccgc atgtttcagg cggccttaaa 720
   gaaggtattg aaaccctact gatgacgcaa cgccctgccc tgtccggcga tgccgttcaa 780
   aaaatgttgg ggcattgcac cttcgaaccg gcagagccga aagccgctaa aaacacctac 840
   acagccgaac gtttcatctg gctgaccaag ctgaacaacc tgcgtatttt agagcaaggc 900
   agcgagcggc cattgaccga taccgaacgc gccacgctta tggacgagcc atacagaaaa 960
   tccaaactga cttacgcaca agcccgtaag ctgctgggtt tagaagatac cgcctttttc 1020
   aaaggcttgc gctatggtaa agacaatgcc gaagcctcaa cattgatgga aatgaaggcc 1080
   taccatgcca tcagccgtgc actggaaaaa gaaggattga aagacaaaaa atccccatta 1140
   aacctttctc ccgaattaca agacgaaatc ggcacggcat tctccctgtt caaaaccgat 1200
   gaagacatta caggccgtct gaaagaccgt atacagcccg aaatcttaga agcgctgttg 1260
   aaacacatca gcttcgataa gttcgtccaa atttccttga aagcattgcg ccgaattgtg 1320
   cctctaatgg aacaaggcaa acgttacgat gaagcctgcg ccgaaatcta cggagaccat 1380
   tacggcaaga agaatacgga agaaaagatt tatctgccgc cgattcccgc cgacgaaatc 1440
   cgcaaccccg tcgtcttgcg cgccttatct caagcacgta aggtcattaa cggcgtggta 1500
   cgccgttacg gctccccagc tcgtatccat attgaaactg caagggaagt aggtaaatcg 1560
   tttaaagacc gcaaagaaat tgagaaacgc caagaagaaa accgcaaaga ccgggaaaaa 1620
   gccgccgcca aattccgaga gtatttcccc aattttgtcg gagaacccaa atccaaagat 1680
   attctgaaac tgcgcctgta cgagcaacaa cacggcaaat gcctgtattc gggcaaagaa 1740
   atcaacttag gccgtctgaa cgaaaaaggc tatgtcgaaa tcgaccatgc cctgccgttc 1800
   tcgcgcacat gggacgacag tttcaacaat aaagtactgg tattgggcag cgaaaaccaa 1860
   aacaaaggca atcaaacccc ttacgaatac ttcaacggca aagacaacag ccgcgaatgg 1920
   caggaattta aagcgcgtgt cgaaaccagc cgtttcccgc gcagtaaaaa acaacggatt 1980
   ctgctgcaaa aattcgatga agacggcttt aaagaacgca atctgaacga cacgcgctac 2040
   gtcaaccgtt tcctgtgtca atttgttgcc gaccgtatgc ggctgacagg taaaggcaag 2100
   aaacgtgtct ttgcatccaa cggacaaatt accaatctgt tgcgcggctt ttggggattg 2160
   cgcaaagtgc gtgcggaaaa cgaccgccat cacgccttgg acgccgtcgt cgttgcctgc 2220
   tcgaccgttg ccatgcagca gaaaattacc cgttttgtac gctataaaga gatgaacgcg 2280
   tttgacggta aaaccataga caaagaaaca ggagaagtgc tgcatcaaaa aacacacttc 2340
   ccacaacctt gggaattttt cgcacaagaa gtcatgattc gcgtcttcgg caaaccggac 2400
   ggcaaacccg aattcgaaga agccgatacc ctagaaaaac tgcgcacgtt gcttgccgaa 2460
   aaattatcat ctcgccccga agccgtacac gaatacgtta cgccactgtt tgtttcacgc 2520
   gcgcccaatc ggaagatgag cgggcaaggg catatggaga ccgtcaaatc cgccaaacga 2580
   ctggacgaag gcgtcagcgt gttgcgcgta ccgctgacac agttaaaact gaaagacttg 2640
   gaaaaaatgg tcaatcggga gcgcgaacct aagctatacg aagcactgaa agcacggctg 2700
   gaagcacata aagacgatcc tgccaaagcc tttgccgagc cgttttacaa atacgataaa 2760
   gcaggcaacc gcacccaaca ggtaaaagcc gtacgcgtag agcaagtaca gaaaaccggc 2820
   gtatgggtgc gcaaccataa cggtattgcc gacaacgcaa ccatggtgcg cgtagatgtg 2880
   tttgagaaag gcgacaagta ttatctggta ccgatttaca gttggcaggt agcgaaaggg 2940
   attttgccgg atagggctgt tgtacaagga aaagatgaag aagattggca acttattgat 3000
   gatagtttca actttaaatt ctcattacac cctaatgatt tagtcgaggt tataacaaaa 3060
   aaagctagaa tgtttggtta ctttgccagc tgccatcgag gcacaggtaa tatcaatata 3120
   cgcattcatg atcttgatca taaaattggc aaaaatggaa tactggaagg tatcggcgtc 3180
   aaaaccgccc tttcattcca aaaataccaa attgacgaac tgggcaaaga aatcagacca 3240
   tgccgtctga aaaaacgccc gcctgtccgt tacccatacg atgttccaga ttacgctgca 3300
   gctccagcag cgaagaaaaa gaagctggat taa 3333
<210> 4
   <211> 24
   <212> DNA
   <213> SV40
<400> 4
   gtgcctaaga agaagagaaa ggtg 24
<210> 5
   <211> 8
   <212> PRT
   <213> SV40
<400> 5
   Val Pro Lys Lys Lys Arg Lys Val
   1 5
<210> 6
   <211> 27
   <212> DNA
   <213> Human influenza virus
<400> 6
   tacccatacg atgttccaga ttacgct 27
<210> 7
   <211> 9
   <212> PRT
   <213> Human influenza virus
<400> 7
   Tyr Pro Tyr Asp Val Pro Asp Tyr Ala
   1               5
<210> 8
   <211> 33
   <212> DNA
   <213> Artificial
<220>
   <223> Artificial nuclear localization signal
<400> 8
   gcagctccag cagcgaagaa aaagaagctg gat 33
<210> 9
   <211> 11
   <212> PRT
   <213> Artificial
<220>
   <223> Artificial nuclear localization signal
<400> 9
   Ala Ala Pro Ala Ala Lys Lys Lys Lys Leu Asp
   1               5                   10